# EUROPEAN PATENT APPLICATION

(11) **EP 3 693 028 A1**
(43) Date of publication of application: **12.08.2020**
(21) Application number: 20151038.5
(22) Date of filing: 22.02.2017
(51) Int. Cl.: A61L 27/24, A61L 27/38, A61L 27/54, A61L 27/46, A61L 27/56

(54) **TRIZONAL MEMBRANES FOR PERIOSTEUM REGENERATION**

(30) Priority: 22.02.2016 US 201662298314 P
(62) Divisional of application: 17709529.6
(71) Applicant: The Methodist Hospital, Houston, Texas 77030 (US)
(72) Inventor: TASCOTTI, Ennio, Houston, Texas 77019 (US); TARABALLI, Francesca, Houston, Texas 77054 (US); MINARDI, Silvia, Houston, Texas 77054 (US)
(74) Representative: Walker, Ross Thomson

(57) **Abstract**

Disclosed are trilaminate collagen-based tissue scaffolds that exhibit remarkable morphological mimicry to that of the natural mammalian periosteum tissue they are useful in remodeling. In particular embodiments, periosteum-modeling trizonal membranes for reforming and regrowing human bone tissue are provided that are composed of a first zone of compact collagen, a second layer of collagen-elastin, and a third layer of biomineralized collagen.

## Description

### BACKGROUND OF THE INVENTION

### CROSS-REFERENCE TO RELATED APPLICATIONS

The present application claims priority to United States Provisional Patent Application No. 62/298,314, filed February 22, 2016 (pending; Atty. Dkt. No. 37182.188PV01); the contents of which is specifically incorporated herein in its entirety by express reference thereto.

### STATEMENT REGARDING FEDERALLY SPONSORED RESEARCH OR DEVELOPMENT

Not Applicable.

### NAMES OF THE PARTIES TO A JOINT RESEARCH AGREEMENT

Not Applicable.

### FIELD OF THE INVENTION

The present invention relates generally to the fields of medical implants, and in particular, implants used to restore or replace bone tissue. Disclosed is the development of trizonal collagen-based tissue scaffolds that exhibit remarkable morphological mimicry to that of natural tissue. In particular embodiments, periosteum-modeling trizonal membranes for reforming and regrowing human bone tissue are provided that are composed of a first zone of mineralized collagen, a second layer of collagen-elastin, and a third layer of compact collagen.

### DESCRIPTION OF RELATED ART

Bone is made up of two types of tissue: 1) compact; bone (also known as cortical bone) and cancellous bone (also known as trabecular bone). Cortical bone, which constitutes about 80% of the mass of human bone, is the denser of the two, with a porosity of typically 5-30%. Trabecular bone, conversely, is much less dense, having a porosity of typically 30-90%.

Trauma, cancer, osteoporosis, and a variety of other conditions may lead to bone loss, reduced bone growth, reduced bone volume, or a combination thereof. For these and other reasons, it is important to develop methods for improving bone growth and for regaining bone anatomy.

Extensive bone grafting is commonly used in both military and civilian orthopedic reconstruction surgeries to repair critical sized defects due to trauma or tumor resection. Clinically, more than 500,000 Americans require bone allografts annually (Hoffman and Benoit, 2013), although due to the lack of appropriate osteogenesis, angiogenesis, and remodeling of structural allografts, the 10-year post-implantation failure rate is 60% (Long *et al.,* 2014). Although most orthopedic fractures heal, the clinical management of critical (>3 mm) segmental defects continues to present major challenges for both amputation and limb-salvage approaches (Gugala *et al.,* 2007).

### PERIOSTEUM

The periosteum is a membrane that covers the outer surface of bones. Studies have indicated that periosteum can increase the rate and quantity of bone formation and improve the vascular invasion ability in large segmental defects (Zhang *et al.,* 2008). If a vascularized periosteal sleeve is present, greater vessel invasion and more rapid bone formation in an implanted bone graft can be achieved.

The periosteum plays an essential role in the healing process of both fractures and autografts. Therefore, by providing a pseudo-periosteum to revitalize allografts, a similar robust healing response may be induced. The present invention overcomes these and other inherent limitations in the prior art by providing a tissue-engineered periosteum that promotes allograft integration and healing, and that reduces allograft failure rates.

### DEFICIENCIES IN THE PRIOR ART

No examples of biomimetic membrane exist in the prior art that structurally resemble the architecture of native periosteum. Althought different examples have been recently reported in literature (Zhang *et al.,* 2008; Kang *et al.,* 2014; Zhang *et al.,* 2006; Tate *et al.,* 2011) they always introduce a polymeric component, or precellularize the scaffold prior to implantation.

### BRIEF DESCRIPTION OF THE DRAWINGS

The following drawings form part of the present specification and are included to demonstrate certain aspects of the present invention. The patent or application file contains at least one drawing executed in color. Copies of this patent or patent application publication with color drawing(s) will be provided by the Office upon request and payment of the necessary fee.

For promoting an understanding of the principles of the invention, reference will now be made to the embodiments, or examples, illustrated in the drawings and specific language will be used to describe the same. It will, nevertheless be understood that no limitation of the scope of the invention is thereby intended. Any alterations and further modifications in the described embodiments, and any further applications of the principles of the invention as described herein are contemplated as would normally occur to one of ordinary skill in the art to which the invention relates.

The invention may be better understood by reference to the following description taken in conjunction with the accompanying drawings, in which like reference numerals identify like elements, and in which:
**FIG. 1** shows an illustrative embodiment in accordance with one aspect of the present invention;
**FIG. 2** shows an illustrative embodiment in accordance with one aspect of the present invention;
**FIG. 3A, FIG.3B, FIG. 3C****, FIG. 3D,** **FIG. 3E, FIG. 3F, FIG. 3G, FIG. 3H, FIG. 3I, FIG. 3J****,** **FIG. 3K, and FIG. 3L** show an illustrative embodiment in accordance with one aspect of the present invention;
**FIG. 4A, FIG.4B, FIG. 4C, FIG. 4D, FIG. 4E, FIG. 4F, FIG. 4G, FIG. 4H, FIG. 4I, and FIG. 4J** show illustrative embodiments in accordance with one aspect of the present invention;
**FIG. 5A, FIG.5B, FIG. 5C, FIG. 5D, FIG. 5E, and FIG. 5F** show illustrative embodiments in accordance with one aspect of the present invention;
**FIG. 6A, FIG. 6B, FIG. 6C, and FIG. 6D** show illustrative embodiments in accordance with one aspect of the present invention;
**FIG. 7** shows an illustrative embodiment in accordance with one aspect of the present invention;
**FIG. 8A, FIG. 8B, FIG. 8C, FIG.8D, FIG. 8E, FIG. 8F, FIG.8G, and FIG. 8H****,** show illustrative embodiments in accordance with one aspect of the present invention;
**FIG. 9A, FIG. 9B, FIG. 9C, FIG. 9D, FIG.9E, FIG. 9F, FIG. 9G,** and **FIG. 9H** show illustrative embodiments in accordance with one aspect of the present invention;
**FIG. 10A, FIG. 10B, FIG. 10C, FIG. 10D, FIG. 10E, FIG. 10F, FIG. 10G, and FIG. 10H** show illustrative embodiments in accordance with one aspect of the present invention;
**FIG. 11A, FIG. 11B, FIG.11C, FIG.11D, FIG. 11E, FIG. 11F, FIG. 11G, FIG. 11H, FIG. 11I, and FIG. 11J** show illustrative embodiments in accordance with one aspect of the present invention;
**FIG. 12A, FIG. 12B, FIG. 12C, FIG. 12D, FIG. 12E, FIG. 12F, FIG. 12G, FIG. 12H****, and** **FIG. 12I** show illustrative embodiments in accordance with one aspect of the present invention;
**FIG. 13A, FIG. 13B, FIG. 13C, FIG. 13D, FIG. 13E, and FIG. 13F** show illustrative embodiments in accordance with one aspect of the present invention;
**FIG. 14A, FIG. 14B**, FIG. 14C-1, FIG. 14C-2, and **FIG. 14D** show morphological and chemical characterization of the osteogenic layer of the collagen shell. **FIG. 14A****:** SEM images of human bone and **FIG. 14B** of the collagen shell (layer 1). **FIG. 14C-1 and FIG. 14C-2:** X-ray diffraction and **FIG. 14D****:** thermo gravimetric analysis of bone and collagen shell;

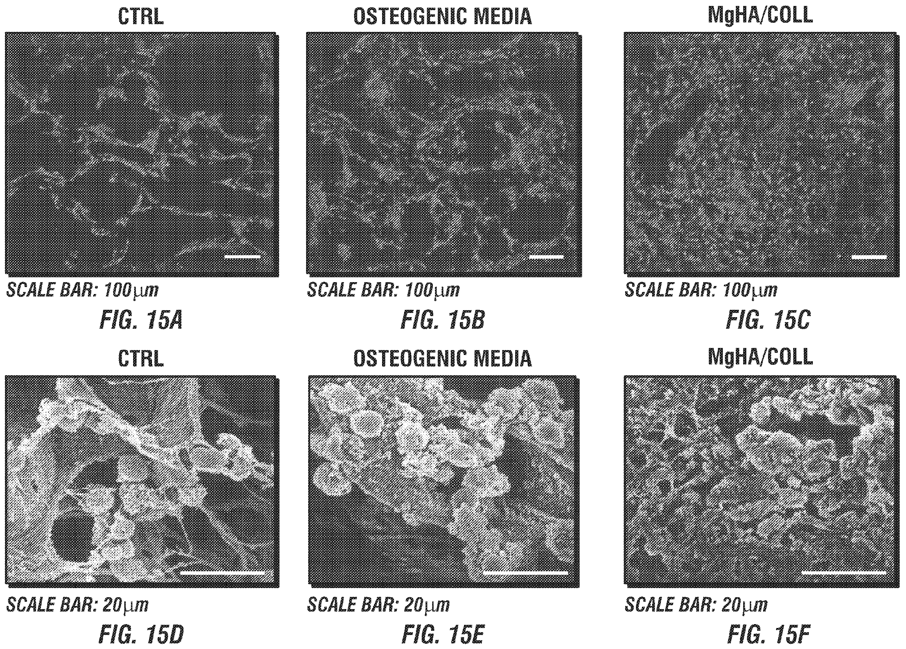

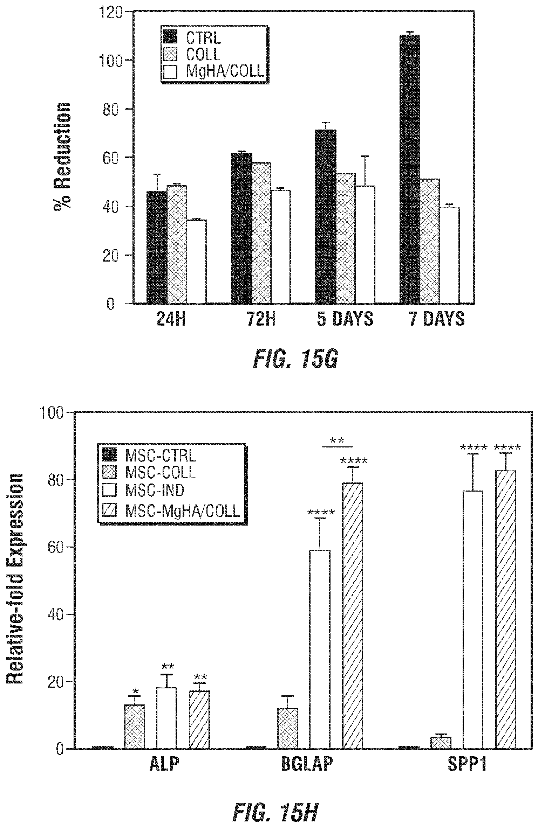

**FIG. 15A, FIG. 15B, FIG. 15C, FIG. 15D, FIG. 15E, FIG. 15F****,** **FIG. 15G, and FIG. 15H** show differentiation of mesenchymal stem cells on osteogenic shells. **FIG. 15A, FIG. 15B, and FIG. 15C****:** Confocal laser microscopy and (**FIG. 15D, FIG. 15E, and FIG. 15F****)** SEM images of MSC seeded on collagen scaffolds in complete media (**FIG. 15A and FIG. 15D**), inducing media (**FIG. 15B** and **FIG. 15E**) and on collagen shell without addition of any other osteodifferentiating stimuli **(****FIG. 15C and FIG. 15F**). Alamar assay for (**FIG. 15G**) cell growth and (**FIG. 15H**) gene expression analysis;
**FIG. 16A and FIG. 16B** show quantification of bone formation in a rabbit spinal fusion model. **FIG. 16A****:** DynaCT micrographs showing the 3D reconstruction of newly formed bone generated by the collagen shell. **FIG. 16B****:** Quantification of the trabecular bone (200 HU) and compact bone (500 HU);
**FIG. 17** shows the physico-chemical characterization of Zone I;

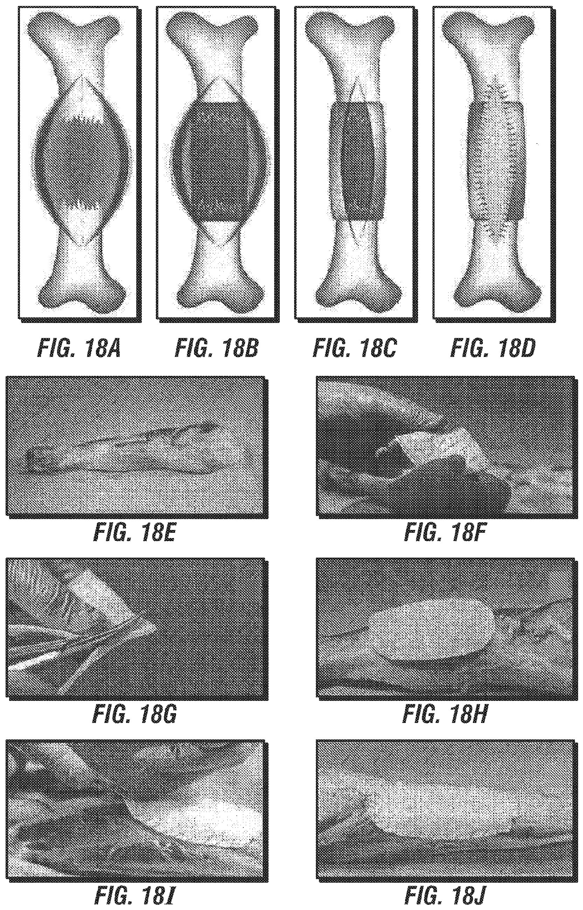

**FIG. 18, FIG. 18B, FIG. 18C, FIG. 18D, FIG. 18E, FIG. 18F, FIG. 18G, FIG. 18H, FIG. 18I, and FIG. 18J** show schematic representations of repair of segmental bone defect. **FIG. 18A****:** Critical sized bone defect created with incised periosteum; **FIG. 18B****:** Placement of PEU shell implant; **FIG. 18C****:** Native periosteum reapproximated with small residual bare area; **FIG. 18D****:** Coverage of bare area with collagen shell; **FIG. 18E** Sheep bone following post-periosteum reapproximation; **FIG. 18F****:** Rehydrated collagen shell; **FIG. 18G and FIG. 18H****:** Customization of collagen shell prior to implantation; **FIG. 18I and FIG. 18J****:** Suturing to periosteum and covering of the bare area;

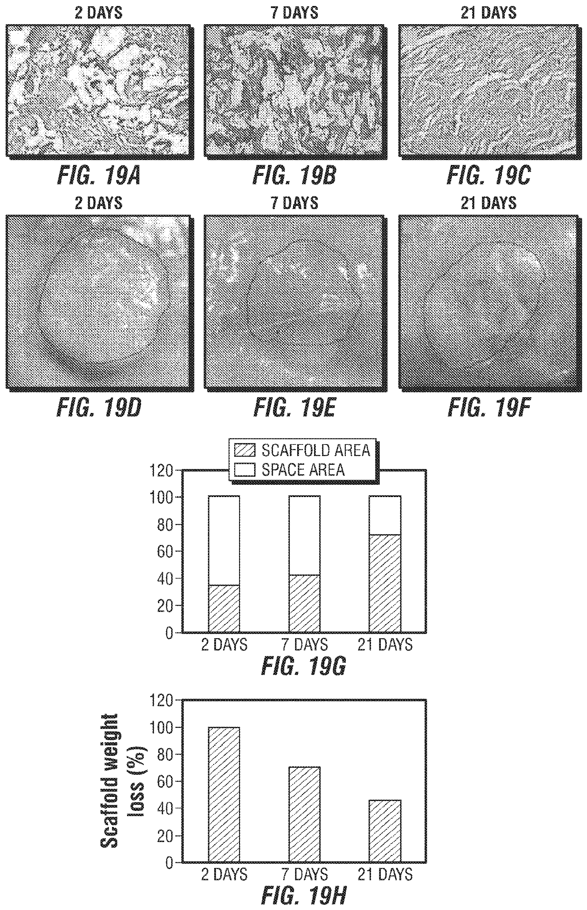

**FIG. 19A, FIG. 19B, FIG. 19C, FIG. 19D, FIG. 19E, FIG. 19F, FIG. 19G, and FIG. 19H** show degradation of implanted collagen shell. **FIG. 19A, FIG. 19B, and FIG. 19C** show histological sections of collagen shell in subcutaneous implants at two, seven, and 21 days, respectively. **FIG. 19D, FIG. 19E, and FIG. 19F** are photographs of collagen shell implanted in the rat skin. **FIG. 19G** shows quantification of the area of the histology slides occupied by the scaffold (pink) or by the pores (grey). **FIG. 19H** shows the evaluation of scaffold degradation by weight loss over time. Red arrows points to blood vessels connecting the scaffold to the central circulation;

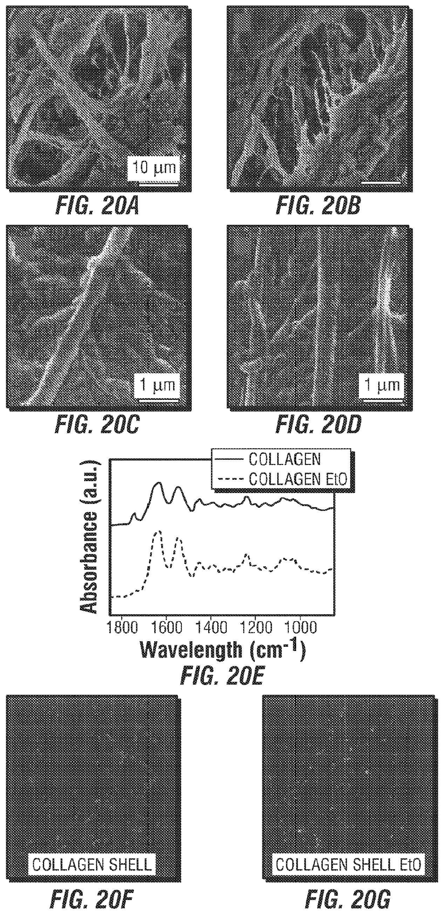

**FIG. 20A, FIG.20B, FIG. 20C, FIG. 20D, FIG. 20E, FIG. 20F, and FIG. 20G** show the porosity and architecture of the Zone II membrane collagen and the shell characterization after ethanol sterilization. **FIG. 20A, FIG. 20B, FIG. 20C, and FIG. 20D** show SEM images of collagen shell. Porosity and mineralization of Zone I is shown before (**FIG. 20A**) and after (**FIG. 20B**) sterilization. The collagen ultrastructure of Zone III is shown before (**FIG. 20C**) and after (**FIG. 20D**) ethanol treatment. FTIR spectra of collagen shell before (black line) and after (red line) ethanol. Confocal microscope images of MSC growth on collagen shell are shown before (**FIG. 20F**) and after (G) ethanol treatment;
**FIG. 21A, FIG. 21B, FIG. 21C, FIG. 21D, FIG. 21E, and FIG. 21F** show illustrative embodiments in accordance with one aspect of the invention;

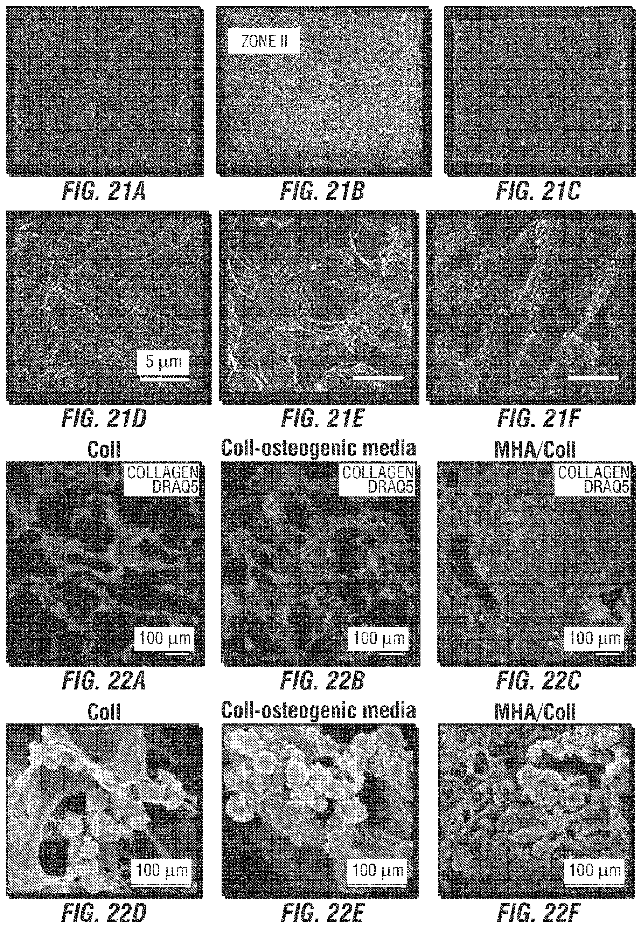

**FIG. 22A, FIG. 22B, FIG. 22C, FIG. 22D, FIG. 22E, and FIG. 22F** show the culture of h-BM-MSC on MHA/Coll. The h-BM-MSC used in the present study were isolated from the bone marrow of two women (age 28 and 35 respectively), at the Texas A&M Institute for Regenerative Medicine (Temple, TX, USA). The medium utilized was composed of α-MEM, 10% FBS, 2% glutamine, 1% β-FGF and 1% streptomycin/amphotericin B (Gibco). Cells were serially passaged using TrypLE Express (Invitrogen) when at 80% confluency. At passage 4, 3 × 10⁵ h-BM-MSC were seeded on MHA/Coll. Cells were also cultured on Coll scaffolds with regular media (negative controls) or osteogenic media (positive control for osteogenic differentiation). The osteogenic media was purchased from Gibco, and used according to the protocols of the manufacturers. Media changes were performed every three days. At 3 weeks, the 3D cultures (n=3) were imaged by a A1 confocal laser microscope (Nikon) and 10⁴ cells/cm² were seeded and incubated at 37°C in humidified atmosphere (90%) with 5% CO₂ and 5% O₂. The medium utilized was composed of α-MEM, 10% FBS, 2% glutamine, 1% β-FGF and 1% streptomycin/amphotericin B (Gibco). Cells were serially passaged using TrypLE Express (Invitrogen) when at 80% confluency. At passage 4, 3 × 10⁵ h-BM-MSC cells were seeded on MHA/Coll. Cells were also cultured on Coll scaffolds with regular media (negative controls) or osteogenic media (positive control for osteogenic differentiation). The osteogenic media was purchased from Gibco, and used according to the protocols of the manufacturers. Media change was performed every three days. At 3 weeks, the 3D cultures (n=3) were imaged by an A1 confocal laser microscope (Nikon) and SEM. Cell distribution was assessed by confocal laser microscopy; the cells were fixed with 4% paraformaldehyde (R&D Systems), and then stained with DRAQ5™ (Thermo Fisher), according to manifacturer's protocol. The scaffolds were visualized in the DAPI field (350 nm), where they are auto-fluorescent. Images were analyzed with the software NIS Elements (Nikon). The morphology of the cells was evaluated by SEM. For SEM imaging, cells were fixed with 2.5% glutaraldehyde (Electron Microscopy Science), and then dehydrated in increasing concentrations of ethanol, up to 100%, and then vacuum dried. Prior to imaging samples were sputter coated with 9 nm of Pt/Pb, and imaged at 10 kV. The amount of a newly deposited calcium phosphate phase was quantified by SEM-EDS. For h-BM-MSC-MHA/Coll constructs, MHA/Coll was also analyzed, as a baseline;

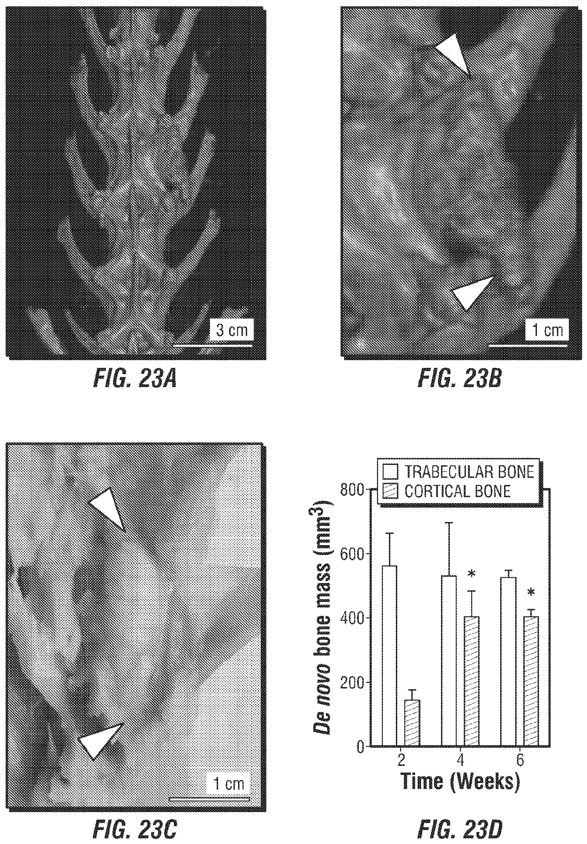

**FIG. 23A, FIG. 23B, FIG. 23C, and FIG. 23D** show an illustrative rabbit bone orthotopic model. All animal work was approved and supervised by the Houston Methodist Research Institute (HMRI) Institutional Animal Care and Use Committee (IACUC, AUP-1111-0058) and guidelines from the American Association for Laboratory Animal Science (AALAS) as well as animal care procedures outlined by the NIH Guide for the Care and Use of Laboratory Animals, and were strictly enforced. Scaffolds (4 ×1 cm) were sterilized in an AN74ix ethylene oxide chamber (Andersen, Haw River, NC, USA). New Zealand White rabbits (n=12, Charles River Labs, Houston, TX, USA) weighing 4.0-5.0 kg each underwent a 72-hr acclimation period upon arrival before being housed individually in cages, and allowed weight-bearing ambulation, food/water *ad libitum,* and a tasteless aqueous antibiotic solution (penicillin) for infection prophylaxis. Under the effects of ketamine, midazolam and inhaled isoflurane anesthesia, a dorsal midline incision approximately 10 cm in length was made directly over palpable spinous processes of the lumbar spine through the skin and subcutaneous tissues. Next, bilateral 6-cm incisions were made just lateral to the palpable mammary bodies from the level of the L4 vertebra to the L7 vertebra. Using blunt dissection between the longissimus, multifidus and ileocostalis muscles, the transverse processes (TP) of L5-L6 were exposed and cleaned of thin muscular attachments using a molt style periosteal elevator. Extra care was taken during dissection near the superior edge of the TP-vertebral body (VB) junction, where the neurovascular bundle exits the spinal canal. Each TP and adjoining intertransverse VB portion was decorticated using a high-speed cone burr until punctate bleeding from the marrow space was encountered. A 3-cm long (1-cm diameter) MHA/Coll scaffold was placed between the decorticated TPs on the experimental anatomical right side, functioning in effect to bridge the defect; meanwhile the anatomical left side was used as an internal control, receiving decortication alone. Incisions were approximated with suture and after 6 weeks *in vivo,* animal subjects were humanely euthanized for harvest of the biomaterial samples. No animals suffered any unforeseen morbidity or mortality requiring removal from the study. Under the effect of sedation, lumbosacral computed tomography (DynaCT) scans were acquired for all animals (n= 12) at 24 hrs, 2-, 4- and 6-weeks' post-implantation, with an Axiom Artis C-arm (d)FC (Siemens Healthcare). Scanning was performed with a 48 × 36 cm flat-panel integrated detector. Acquisition parameters for DynaCT were as follows: 70 kV tube voltage, automatic tube current of 107 mA, 20-sec scan. Each scan entailed 222 degrees of rotation, with 1 image taken every 0.5° for a total of 444 images (each digital acquisition had a matrix of 514 × 514 pixels) per acquisition. Three-dimensional rendering was obtained through the Inveon Research Workplace 4.2 Software (Siemen Medical Solution Inc.). 6 weeks postoperatively, the implants were isolated from the proximal and distal ends of the spine using two axial cuts, and were submitted to Ratliff Histology (Franklin, TN, USA) for processing to slides in methyl metacrylate resin. The undecalcified samples were then cut sagittally using the spinal canal as the center point. Afterwards, each half was dehydrated and infiltrated and embedded with acrylic resin. Sections were created using the cut and grind technique with the EXAKT Cutting and Grinding System (5-10 micron sections at 500 microns between each level). Sections were stained with Von Kossa/MacNeal's Tetrachrome, Goldner's Trichrome and hematoxylin and eosin staining, according to standard protocols;

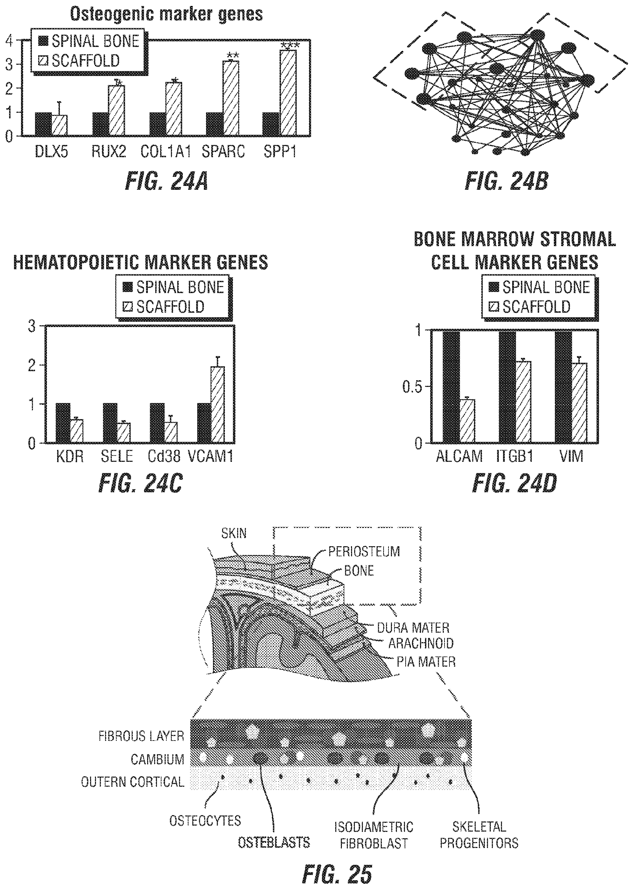

**FIG. 24A, FIG. 24B, FIG. 24C, and FIG. 24D** show *in vivo* osteogenesis and hematopoiesis. When the MHA/Coll scaffold was implanted in an ectopic rabbit model, it exerted an impressive osteinductive ability to form an extended bone mass at 6 wks (refer to previous publication). First, on molecular level, the functional potentials were explored for bone mass which newly generated on implanted scaffold and compared it with native spinal bone. Strikingly, bone mass grown on scaffold, instead of the native bone, demonstrated an augmented osteogenesis with a gradient heightened expression of the marker genes (RUNX2 2.21±0.24 (p<0.05), COL1A1 2.28±0.16 (p < 0.05), SPARC 3.19±0.07 (p < 0.01), and SPP1 3.67±0.09 (p < 0.001). The presence of SPARC and SPP1, which were markers for late osteoblasts and osteocytes, indicated that bone maturation on the scaffold initiated at only 6 wks. Moreover, the development of hematopoietic cells and bone marrow stromal cells in the bone mass on scaffold was proven. KDR, CD38, and SELE, which are key receptors or surface markers defining hematopoietic stem cells (HSCs), were determined in bone mass on scaffold with a level of more than 50% compared with it in native bone. VCAM1, an adhesion molecule whose expression strongly reduced during hematopoietic progenitor cell mobilization, also presented in bone mass with a 1.95±0.25-fold increase in comparison with native bone, suggesting newly developed HSCs had a weaker migration activity. Marker genes of bone marrow stromal cells, in consistent with marker genes of HSCs, were also identified in scaffold bone mass (ALCAM, ITGB1, and VIM with a level of 39%, 73%, and 72% when normalized to native bone);
**FIG. 25** depicts a schematic cross-section of the mammalian skull;
**FIG. 26A, FIG. 26B, FIG. 26C, FIG. 26D, FIG. 26E, FIG. 26F, FIG. 26G, FIG. 26H****, and** **FIG. 26I** showed increased compact and trabecular bone that did not occur in animals without the implant. The newly-formed bone was observed along the defect's edges (adjacent to old bone). Tissue samples showed bone bridging across the defect, especially along the periosteum. Blood vessels were observed throughout the newly-formed tissue, mainly in the superior zone, due to apposition of the periosteum layer on the PMM Layer I;

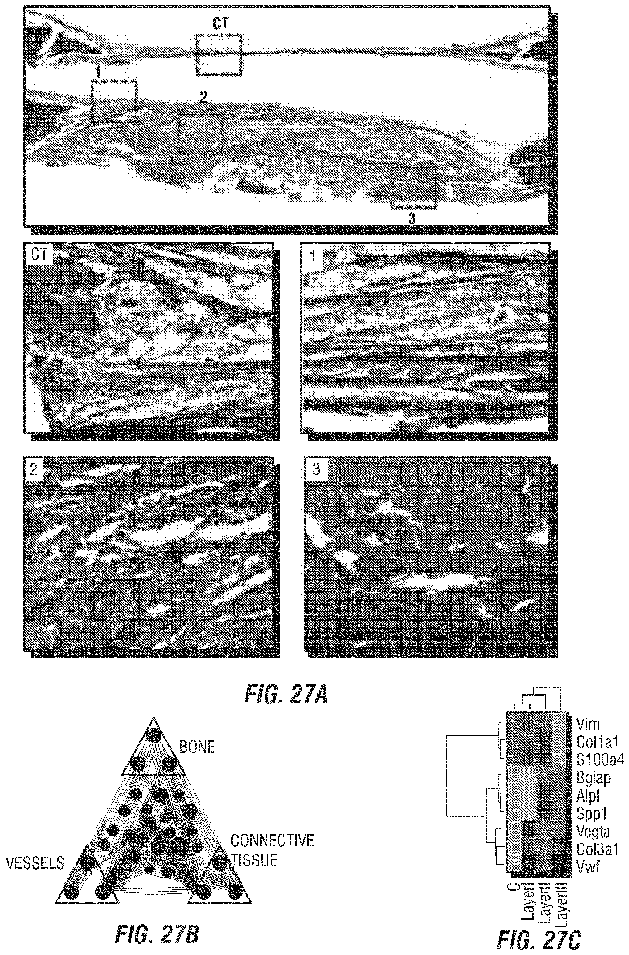

**FIG. 27A, FIG. 27B, and FIG. 27C** show histological sections cut coronally through the defect after 4 weeks of implantation. (top) Control and (botton) PMM implanted (**FIG. 27A**)**.** The sections are stained with Masson's trichrome. Numbers indicate the different area reported in the magnification at the bottom. The control showed only a thin connective tissue that connect the two bone ends. PMM in 4 week bridge completely the critical size defect maintaining the organization of the trilayers. The periosteum completely vascularized. The Layer III (2) showed area of mineralized tissue (4). **FIG. 27B** illustrates interactome of the genes analyzed by Laser microdissection (LM). RNA was extracted from microdissected tissues and the expression of the genes relative to vessels, bone and connective tissue was evaluated; **FIG. 27C** shows a heat map of the three layers;

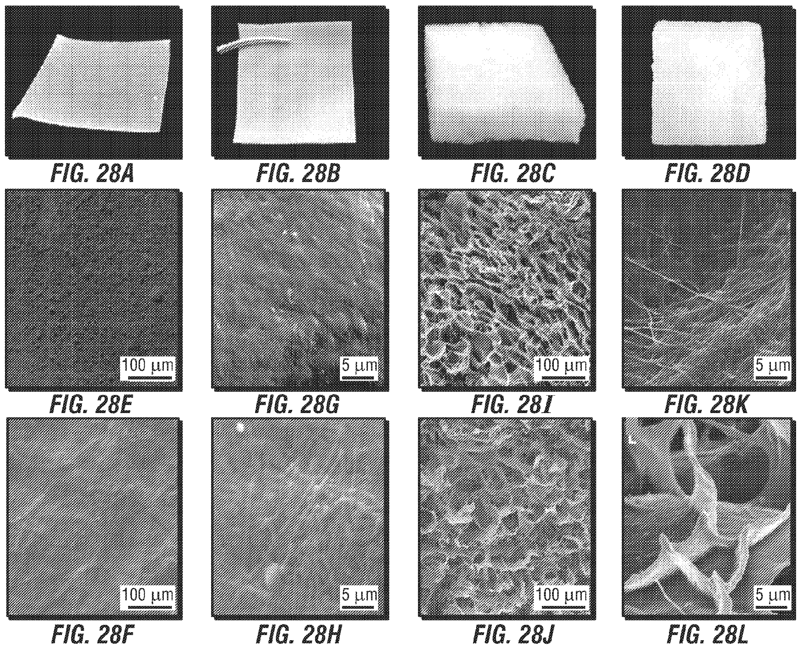

FIG. 28A, FIG. 28B, FIG. 28C, FIG. 28D, FIG. 28E, FIG. 28F, FIG. 28G, FIG. 28H, FIG. 28I, FIG. 28J, FIG. 28K, and FIG. 28L show the characterization of CollE Sheets and Scaffolds. Images of the CollE Sheets (**FIG. 28A**, **FIG. 28B**) and CollE Scaffolds (**FIG. 28C, FIG. 28D**) when dry or re-hydrated, respectively. SEM images of Coll and CollE Sheets at 1000X (**FIG. 28E and FIG. 28G**, respectively) and 15000x (**FIG. 28F** and FIG. 28H, respectively). SEM micrographs of Coll and CollE Scaffolds at 1000X (**FIG. 28I and FIG. 28K**, respectively) and 15000X (**FIG. 28J and FIG. 28L**, respectively);
**FIG. 29A, FIG. 29B, and FIG. 29C** show the absorbance (**FIG. 29A**), water contact angle (**FIG. 29B**) and swelling properties (**FIG. 29C**) of Coll Sheet, CollE Sheet, Coll Scaffold and CollE Scaffold;
**FIG. 30A, FIG. 30B, FIG. 30C, FIG. 30D, FIG. 30E, and FIG. 30F** show the *in vitro* degradation study and mechanical properties of the meshes. *In vitro* degradation of the Coll and CollE Sheets and Scaffolds (FIG. 29A, FIG. 29B, FIG. 29C, and FIG. 29D). DSC analysis (FIG. 29E) and mechanical characterization of Coll Sheet, CollE Sheet, Coll Scaffold and CollE Scaffold. (FIG. 29F). Values represent mean ±standard deviation. A value of p < 0.05 was considered statistically significant, compared to the rat abdominal wall: **p < 0.01, ****p < 0.0001;

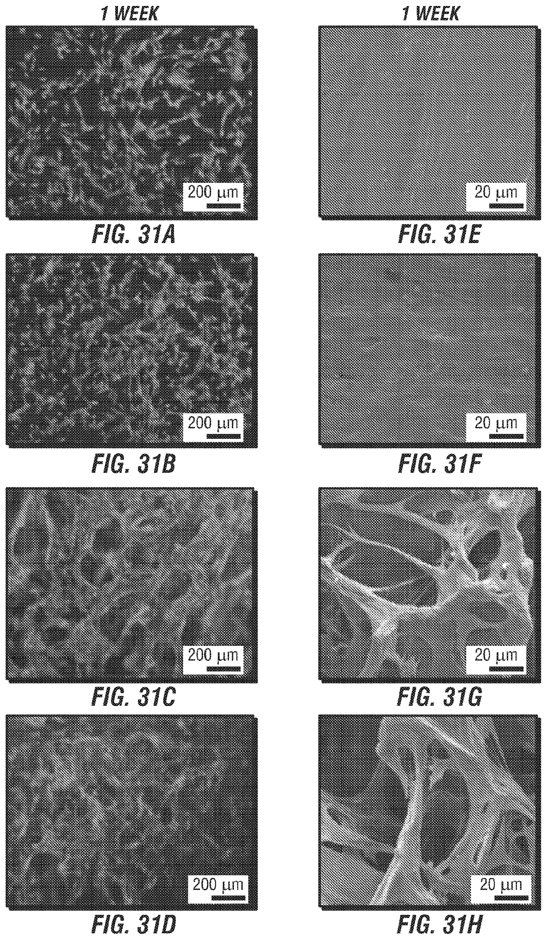

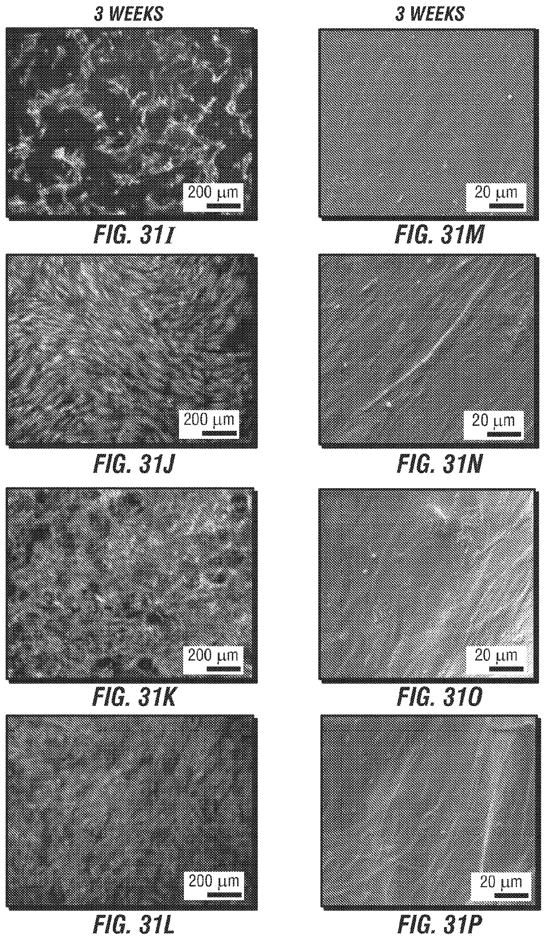

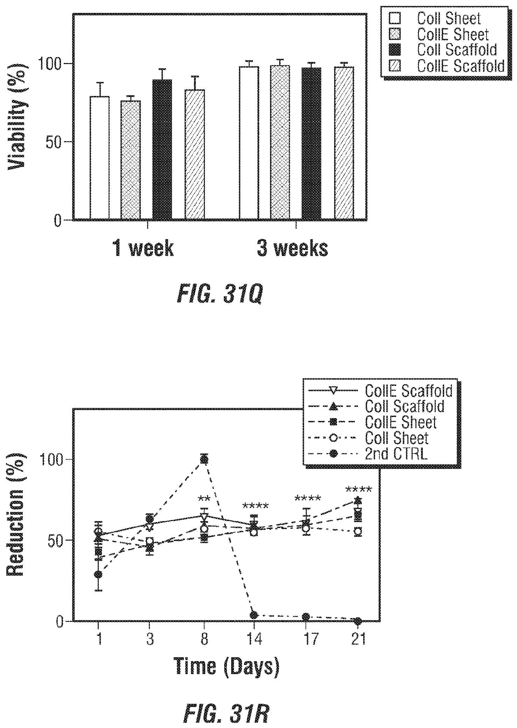

**FIG. 31A, FIG. 31B, FIG. 31C, FIG. 31D, FIG. 31E, FIG. 31F, FIG. 31G, FIG. 31H****,** **FIG. 31I, FIG.31J, FIG. 31K, FIG.31L, FIG.31M, FIG. 31N, FIG. 31O, FIG. 31P****,** **FIG. 31Q, and FIG. 31R** show the *in vitro* 3D culture of h-BM-MSC on Coll and CollE Sheets, and Coll and CollE Scaffolds. Cells were cultured on the meshes up to 3 weeks. Coll Sheet, CollE Sheet, Coll Scaffold and CollE Scaffold (**FIG. 31A, FIG.31B, FIG. 31C and FIG. 31D**, respectively) visualized by LIVE/DEAD staining, or imaged by SEM (**FIG. 31E, FIG. 31F, FIG.31G and FIG.31H**) at I week, and 3 weeks (**FIG. 31I, FIG.31J, FIG. 31K, FIG. 31L, FIG. 31M, FIG. 31N, FIG. 31O, FIG. 31P**). Cell viability (**FIG. 31Q**) was assessed by LIVE/DEAD assay, and cell growth by Alamar Blue assay (**FIG. 31R**)**.** Values represent mean ±standard deviation. A value of p < 0.05 was considered statistically significant: **p < 0.01, ****p < 0.0001;
**FIG. 32A, FIG. 32B, FIG. 32C, FIG. 32D, FIG. 32E, FIG. 32F, FIG. 32G, FIG. 32H, FIG. 32I, and FIG. 32J** show rat ventral hernia repair. Surgery for the implantation of CollE Sheets (**FIG. 32A, FIG. 32B, FIG. 32C**) and of CollE Scaffolds (**FIG. 32D, FIG. 32E, FIG. 32F**)**.** The scaffolds were 3 cm long (indicated by black arrows). The defect repaired with CollE Sheets (**FIG. 32G, FIG. 32H**) and with CollE Scaffold (**FIG. 32I, FIG. 32J**)**,** 6-weeks' post-surgery. The white arrows indicate newly formed vessels, within the implants;

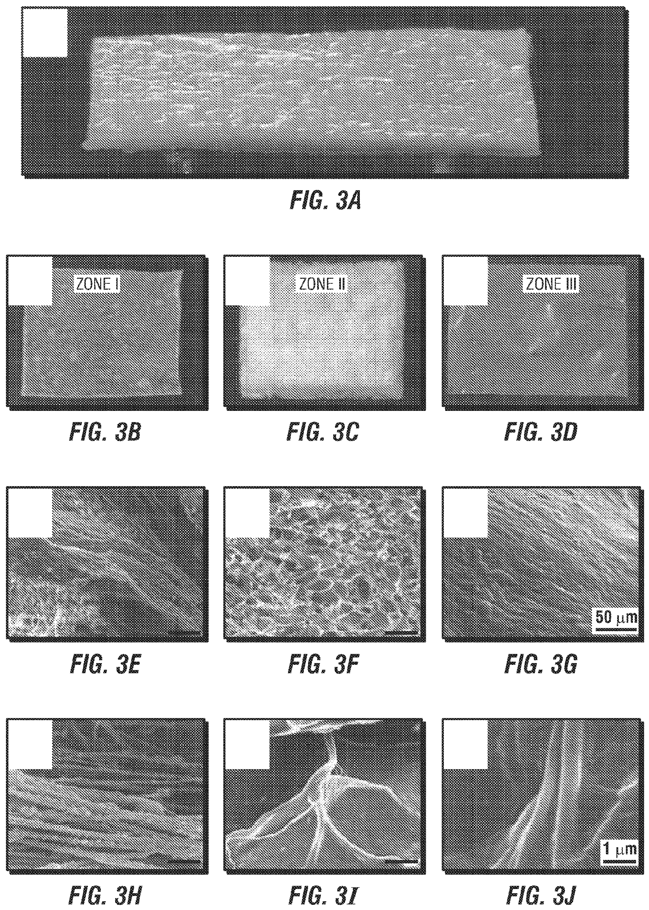

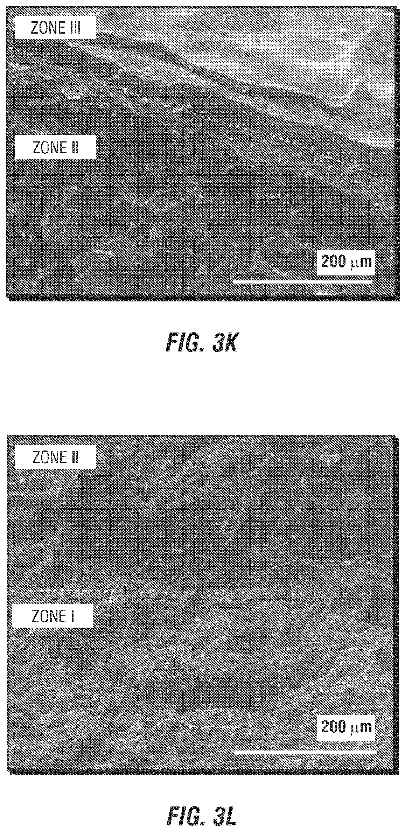

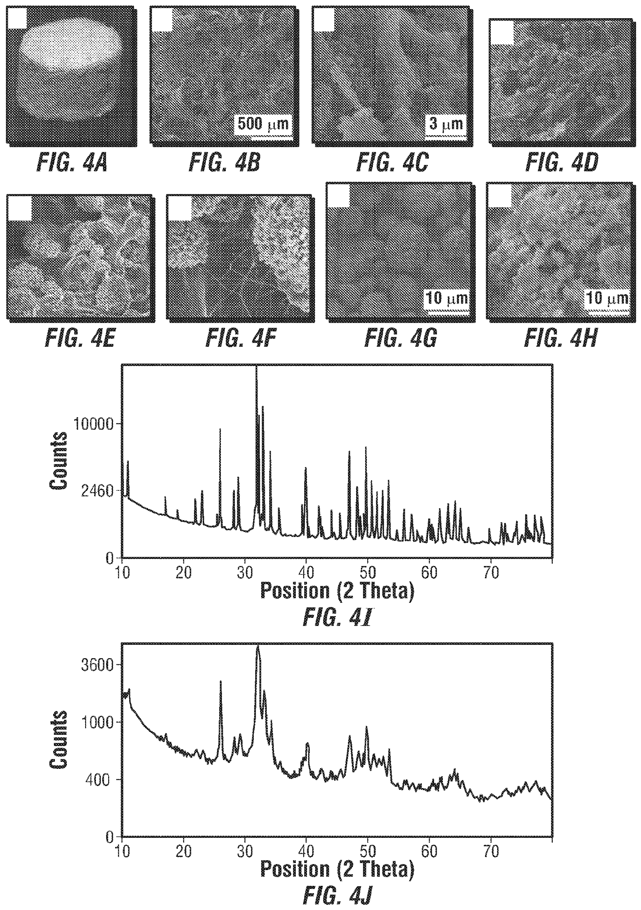

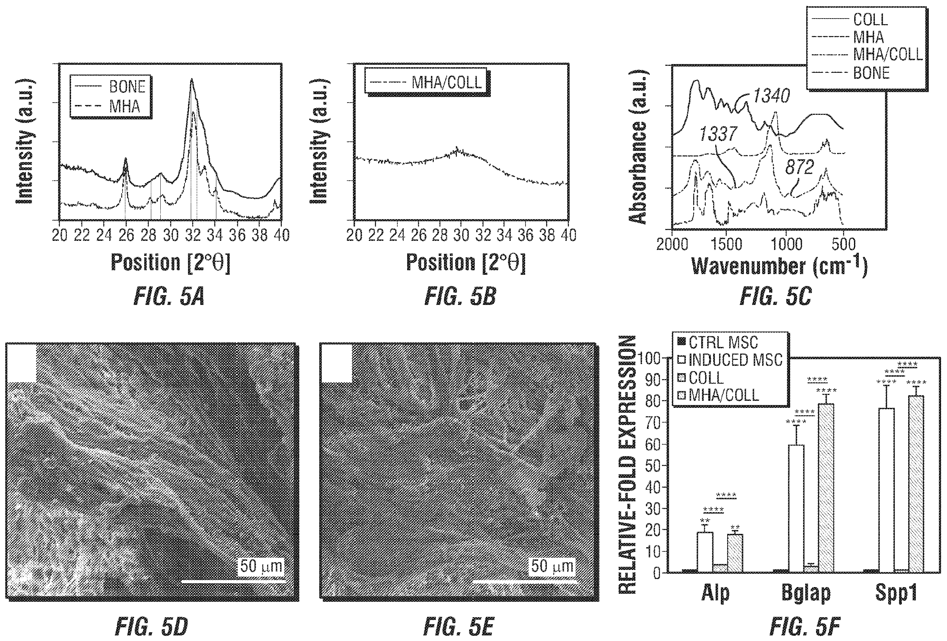

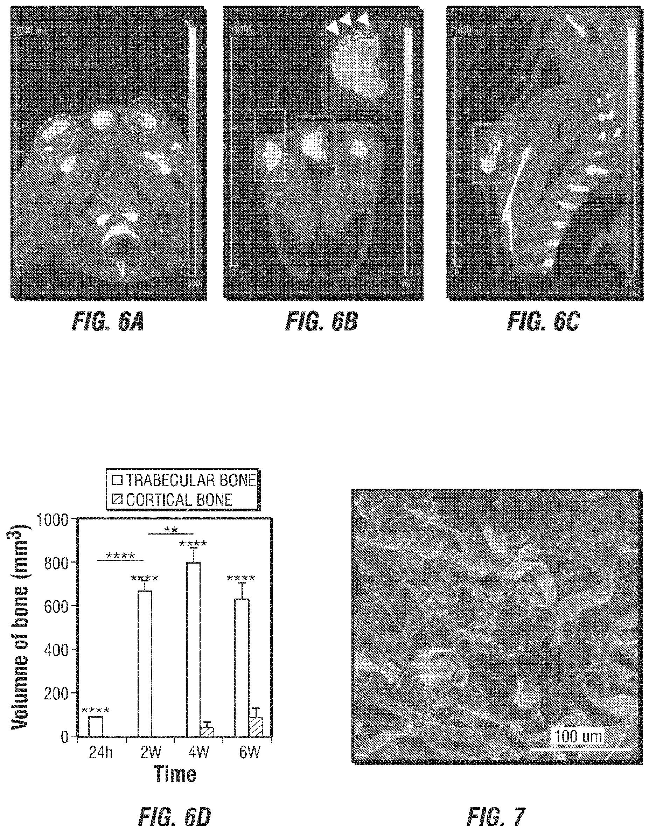

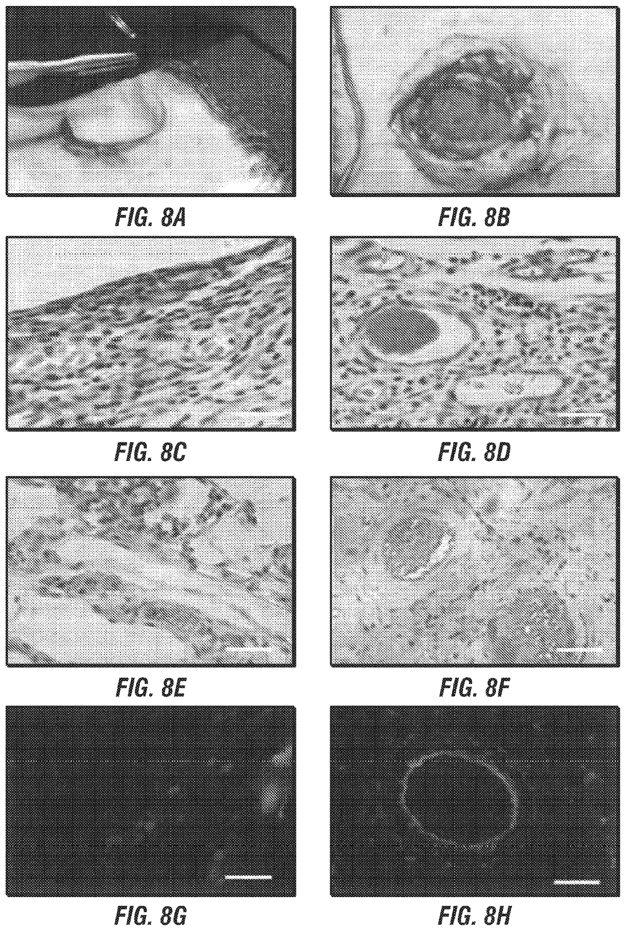

**FIG. 33A-1, FIG. 33A-2, FIG. 33A-3, FIG. 33A-4, FIG. 33A-5, FIG. 33A-6, FIG. 33A-7, FIG. 33A-8, FIG. 33B-1, FIG. 33B-2, FIG. 33B-3, FIG. 33B-4, FIG. 33B-5, FIG. 33B-6, FIG. 33B-7, and FIG. 33B-8** show the histology of explants at 6 weeks. Masson's trichrome staining of the tissue formed within Coll Sheet, CollE Sheet, Coll Scaffold and CollE Scaffold, in 6 weeks after implantation in a rat ventral hernia model. Collagen is stained in blue, muscles in bright red and cells in pink;

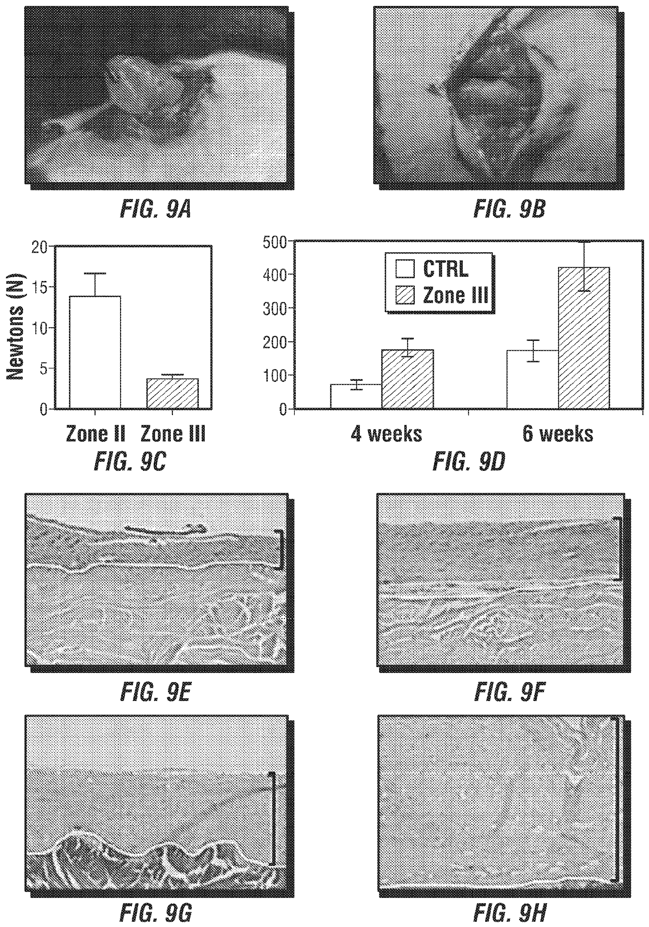

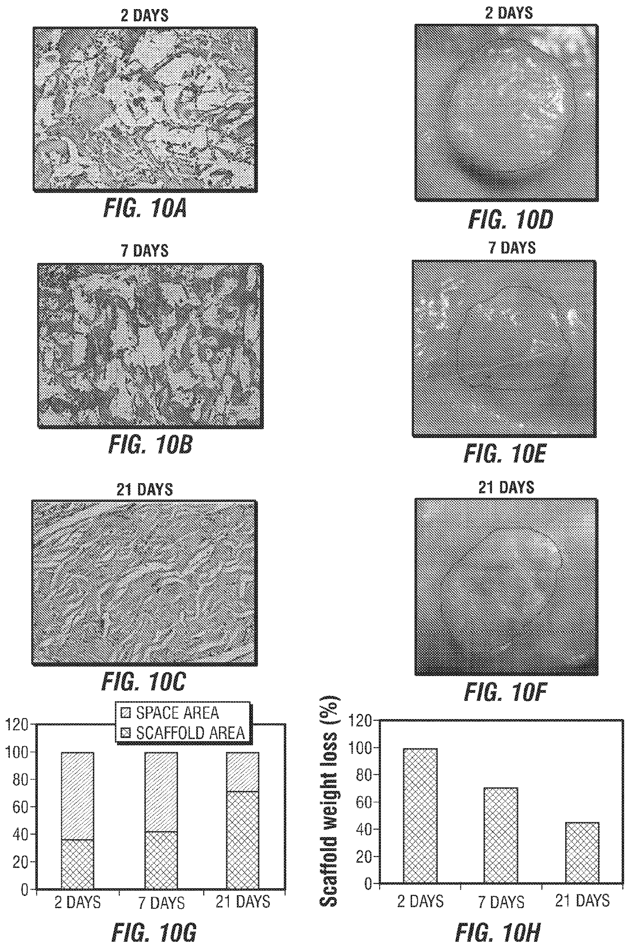

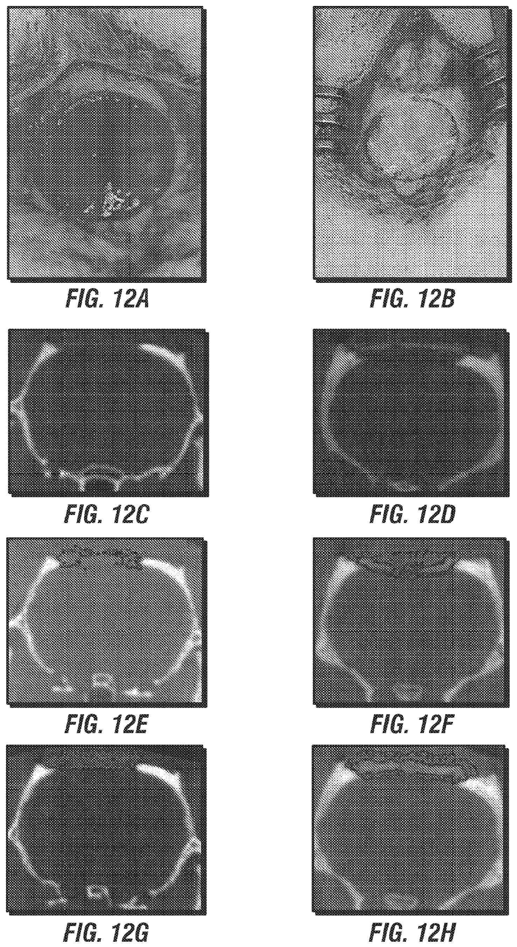

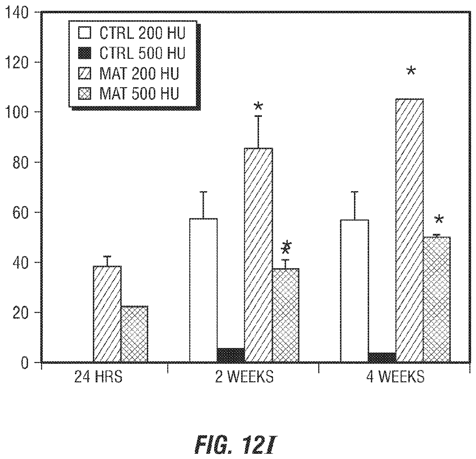

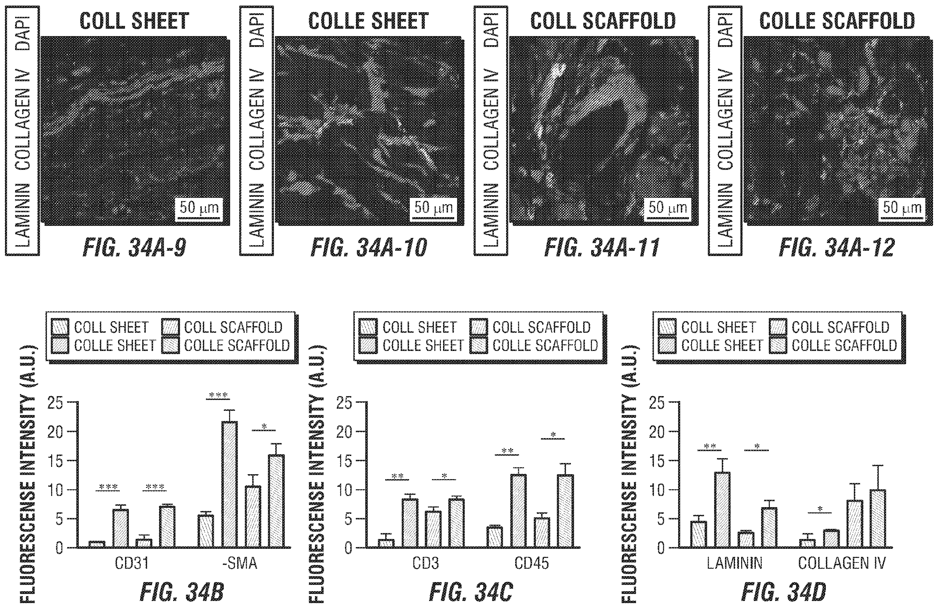

**FIG. 34A-1, FIG. 34A-2, FIG. 34A-3, FIG. 34A-4, FIG. 34A-5, FIG. 34A-6, FIG. 34A-7, FIG. 34A-8, FIG. 34A-9, FIG. 34A-10, FIG. 34A-11, FIG. 34A-12,** **FIG. 34B, FIG. 34C, and FIG. 34D** show the immunofluoscence evaluation of neovascularization. Newly formed vessels within the grafts (Coll Sheet, CollE Sheet, Coll Scaffold, CollE Sheet) were stained for smooth muscle cells (a-SMA, green) and endothelial cells (CD31 in red); staining of lymphocytes and leukocytes (CD3, in white and CD45 in yellow, respectively), and basal membrane proteins (Laminin, pink and Collagen IV in orange) (**FIG. 34A-1, FIG. 34A-2, FIG. 34A-3, FIG. 34A-4, FIG. 34A-5, FIG. 34A-6, FIG. 34A-7, FIG. 34A-8, FIG. 34A-9, FIG. 34A-10, FIG. 34A-11, FIG. 34A-12**)**.** All tissue slices were stained with DAPI, shown in blue. **FIG. 34B, FIG. 34C, and FIG. 34D****:** Quantification of the fluorescence intensity of each marker, per area fraction. Values represent mean ±standard deviation. A value of p<0.05 was considered statistically significant: *p < 0.05, **p < 0.01, ***p < 0.001; and

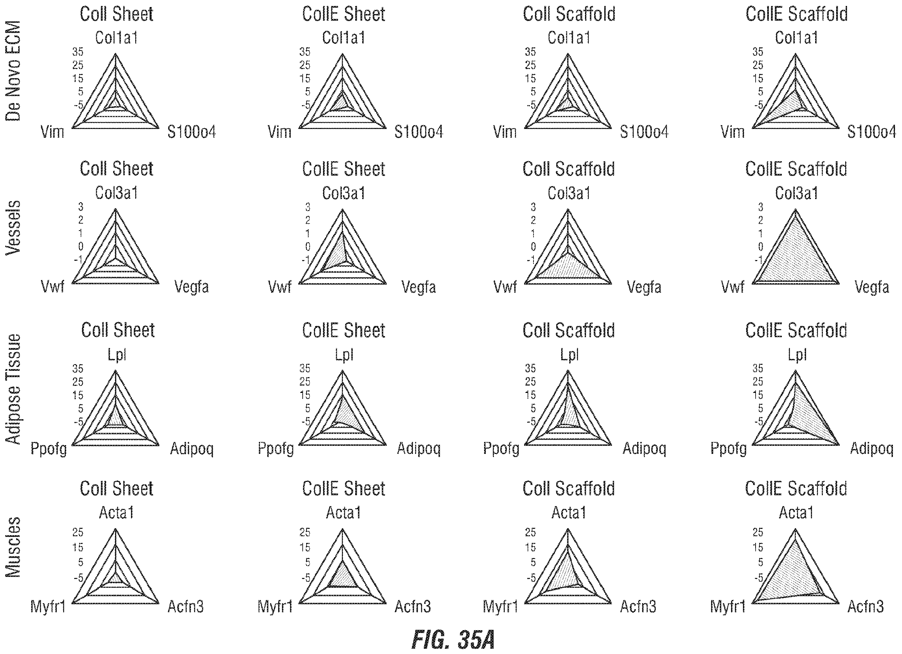

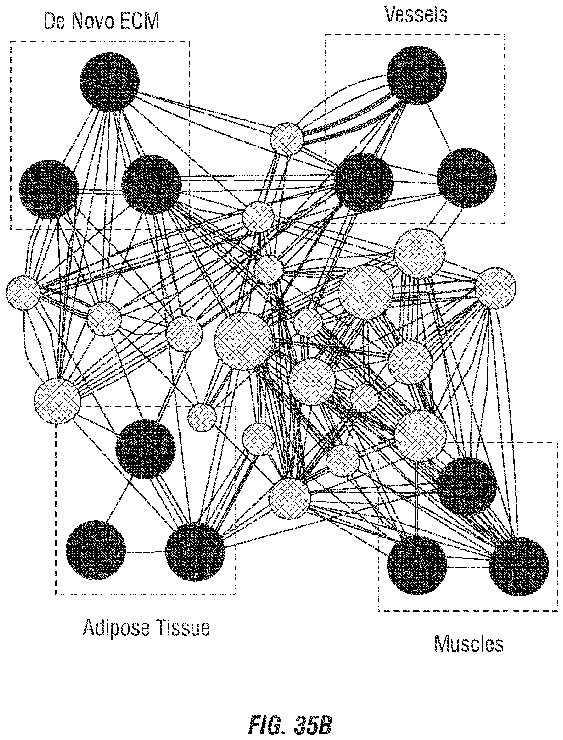

**FIG. 35A** **and** **FIG. 35B** show the gene expression analysis. **FIG. 35A****:** Radar charts illustrating the overall level of expression of clusters of marker genes associated with de novo matrix deposition, angiogenesis, adipogenesis and muscle tissue, per each group (**FIG. 35B**) (Coll Sheet, CollE Sheet, Coll Scaffold, CollE Scaffold), 6 weeks post-implantation in a rat ventral hernia repair model.

### BRIEF SUMMARY OF THE INVENTION

The present invention overcomes these and other limitations inherent in the prior art by providing methods for the development of a trizonal tissue scaffold that more accurately models native periosteum than conventional existing technologies. The system developed herein provides the closest recreation of a tissue scaffold to permit normal bone reformation and growth. In sharp contrast to the prior art, the membranes disclosed herein, are able to induce *per se,* without the adition of any extra drug or biologicals.

The dislosed tri-zonal membranes are able to recapitulate the architecture and the composition of the extracellular matrix of the periosteum. In an overall and general sense, the membranes of the invention are preferably composed of a first zone (Zone I; "ZI") that includes a layer of compact collagen (*i.e.,* a fibroblastic zone), a second zone (Zone II; "ZII") that includes a layer of collagen-elastin (which permits vascularization and recapitulates the elastic features of periosteum), and a third zone (Zone III; "ZIII") that includes a layer of mineralized collagen (which preferably exploits the presence of hydroxyapatite to induce bone formation).

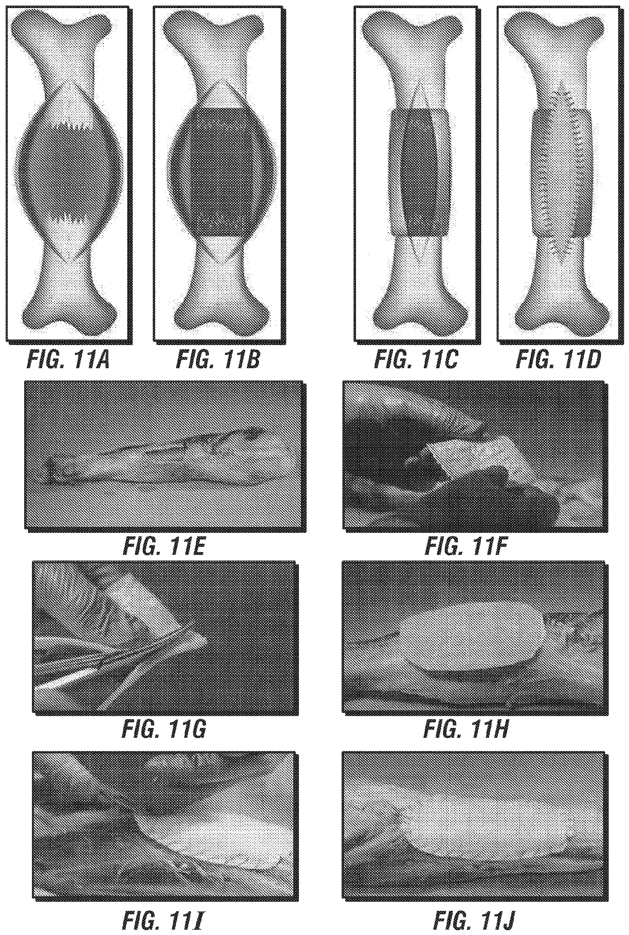

This periosteum-like material trilaminate material has the ability to serve as a scaffold for a tissue-engineered periosteum that maintains and mimics its natural structure. In illustrative embodiments, a natural-derived biomaterial has been developed which mimics both the composition and the architecture of the periosteum, and that reduces complications in cases of severe open fracture, as seen in **FIG. 11A, FIG. 11B, FIG. 11C****, and** F**IG. 11D**.

Unlike previous multi-layer membranes that mimic the osteochondral region (*i.e.,* bone-interface-cartilage), the tri-laminate material disclosed herein mimics the periosteum region-that is, a mineralized layer closest to bone, a porous, vascularized collagen+elastin layer in the middle, and a non-porous, compact collagen layer that is closest to tissue.

### DESCRIPTION OF ILLUSTRATIVE EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would be a routine undertaking for those of ordinary skill in the art having the benefit of this disclosure.

The role of periosteum as an important support tissue for underlying cortical bone with progenitor cell-donating potential has been well established. Clinical and experimental data support the pivotal role that the periosteum plays in bone autograft healing and remodeling.

The periosteum contains mesenchymal stem cells (MSC) that are capable of differentiating into bone and cartilage cells.

The inventors have extensive experience with complex, fine-tuned collagen-based scaffolds that are applied to a variety of tissue engineering applications, including but not limited to: osteochondral regeneration, spinal fusion, and soft tissues' critical-sized defect for general surgery (the ventral abdominal hernia) (Murphy *et al.,* 2010; Murphy *et al.,* 2011; Murphy *et al.,* 2012; Minardi *et al.,* 2015). Data indicate that these engineered scaffolds provide the perfect environment to promote cell population, transmigration, and ultimately, tissue growth across a defect that far supersedes the weak scar that would otherwise form in its absence (Minardi *et al.,* 2015).

Different biomaterials have been proposed as bone substitutes with conflicting results. Among these, hydroxyapatite (HA) and other calcium phosphate ceramics have shown the most promising results due to their osteoconductive properties and absence of immune response. Tampieri and co-workers developed biomimetic multisubstituted apatites (*e.g.,* magnesium and carbonate substituted hydroxyapatite) with enhanced osteoinductivity in a sheep model and led to successful pilot clinical studies in Europe for repairing long-bone loss (O'Brien, 2011). The introduction of specific doping ions in the apatite lattice allowed for the enhancement of the bioactivity of the ceramic phase of these hybrid materials, such that the addition of biologics was not required. The efficacy of these materials has also been proven in other models for bone repair, such as for the regeneration of subchondral bone in a sheep model and in humans (Filardo *et al.,* 2013).

A new formulation has been developed, and a collagen shell designed that confers a higher degree of mimicry for spongy bone, both at the morphological and compositional level. Data indicated that the collagen shell could induce a faster and more efficient differentiation of MSC without the use of any biologics. With the ability to closely mimic the three zones of native periosteum using the collagen shells described herein, the problem of delayed or absent bone regrowth over exposed areas of the PEU implant can finally be addressed.

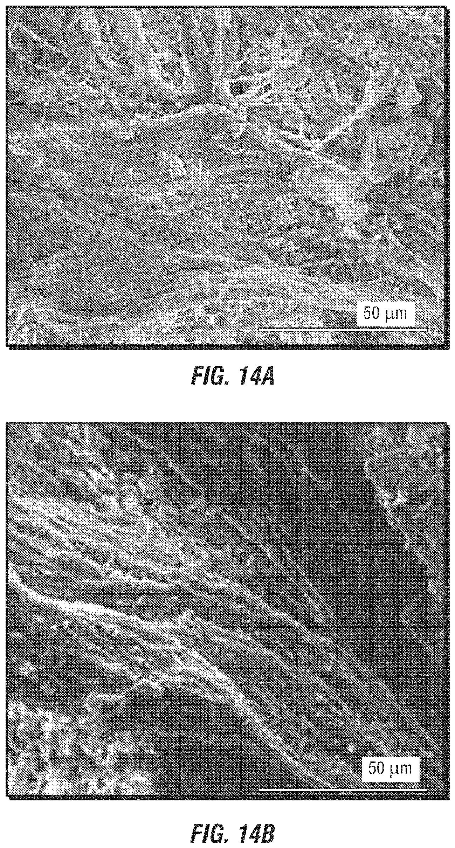

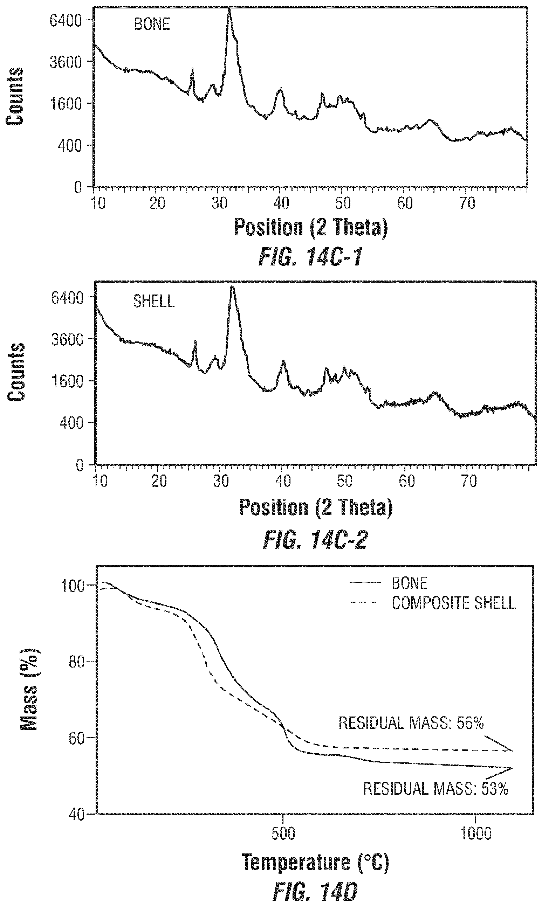

Shown in FIG. 14A and FIG. 14B are close-up views of human spongy bone and the collagen shell, respectively, revealing extensive morphological mimicry, as demonstrated by the qualitative X-ray diffraction analysis (FIG. 14C), showing that the mineral phase of the sponge was identical to that of bone. In addition, the apatite/collagen ratio resembled that of the natural tissue as found by thermogravimetric analysis (see FIG. 14D).

The collagen shell has significant regenerative potential. Bone marrow mesenchymal stem cells seeded on collagen scaffolds are able to attach, proliferate, spread (FIG. 15A), and undergo osteo-differentiation if grown in osteogenic media (FIG. 15B). However, if the cells are seeded on the collagen shell, they efficiently differentiate without the addition of inducing media, creating a denser cell-collagen construct closely resembling bone tissue (FIG. 15C). These findings were further confirmed by scanning electron microscopy (FIG. 15D, FIG. 15E, and FIG. 15F). Cell growth appeared to be reduced on our collagen shell (FIG. 15G), correlating with the higher degree of osteo-differentiation of cells (FIG. 15H). In fact, the collagen shell alone (MSC-MgHA/Coll) induced a higher level of expression of both osteocalcin and osteopontin at 3 weeks, compared to cells grown in 2D conditions, on collagen scaffolds, or on collagen scaffolds with inducing media.

### PHARMACEUTICAL FORMULATIONS

In certain embodiments, the present invention concerns compositions prepared in pharmaceutically-acceptable formulations for delivery to one or more cells or tissues of an animal, either alone, or in combination with one or more other modalities of diagnosis, prophylaxis and/or therapy. The formulation of pharmaceutically acceptable excipients and carrier solutions is well known to those of ordinary skill in the art, as is the development of suitable surgical implantation methods for using the particular membrane compositions described herein in a variety of treatment regimens, and particularly those involving bone regrowth.

Sterile injectable compositions may be prepared by incorporating the disclosed tissue scaffolds in the required amount in the appropriate solvent with several of the other ingredients enumerated above, as required, followed by filtered sterilization. Generally, dispersions can be prepared by incorporating the selected sterilized active ingredient(s) into a sterile vehicle that contains the basic dispersion medium and the required other ingredients from those enumerated above. The tissue scaffolds disclosed herein may also be formulated in solutions comprising a neutral or salt form to maintain the integrity of the membranes prior to implantation.

Pharmaceutically-acceptable salts include the acid addition salts (formed with the free amino groups of the protein), and which are formed with inorganic acids such as, without limitation, hydrochloric or phosphoric acids, or organic acids such as, without limitation, acetic, oxalic, tartaric, mandelic, and the like. Salts formed with the free carboxyl groups can also be derived from inorganic bases such as, without limitation, sodium, potassium, ammonium, calcium, or ferric hydroxides, and such organic bases as isopropylamine, trimethylamine, histidine, procaine, and the like. Upon formulation, solutions will be administered in a manner compatible with the dosage formulation, and in such amount as is effective for the intended application. The formulations are readily administered in a variety of dosage forms such as injectable solutions, topical preparations, oral formulations, including sustain-release capsules, hydrogels, colloids, viscous gels, transdermal reagents, intranasal and inhalation formulations, and the like.

The amount, implantation regimen, formulation, and prepartation of the tissue scaffolds disclosed herein will be within the purview of the ordinary-skilled artisan having benefit of the present teaching. It is likely, however, that the administration of a particular tissue scaffolds may be achieved by a single surgical implantation, such as, without limitation, a single implantation of a sufficient quantity of the engineered tissue matrix agent to provide the desired benefit to the patient undergoing such a procedure. Alternatively, in some circumstances, it may be desirable to provide multiple, or successive administrations of the tissue scaffolds, either over a relatively short, or even a relatively prolonged period, as may be determined by the medical practitioner overseeing the surgical implantation of the tissue scaffolds to the individual undergoing treatment for bone repair and reformation.

The compositions of the present invention may further comprise one or more excipients, buffers, or diluents that are particularly formulated for contact with mammalian cells, and in particular human cells, and/or for administration to a mammalian subject, such as a human patient. Compositions may further optionally comprise one or more diagnostic or prognostic agents, and/or may be formulated within a population of microspheres, microparticles, nanospheres, or nanoparticles, and may be formulated for administration to one or more cells, tissues, organs, or body of a human in particular.

Formulation of pharmaceutically-acceptable excipients and carrier solutions is well-known to those of skill in the art, as is the development of suitable dosing, diagnostic, and/or treatment regimens for using the particular compositions described herein in a variety of modalities, including *e.g.,* without limitation, oral, parenteral, intravenous, intranasal, intratumoral, and intramuscular routes of administration.

The particular amount of tissue scaffold material employed, and the particular time of implantation, or the treatment regimen selected will be within the purview of a person of ordinary skill in the art having benefit of the present teaching. It is likely, however, that the administration of the disclosed tissue scaffolds may be achieved by administration of one or more doses of the formulation, during a time effective to provide the desired benefit to the patient undergoing such treatment. Such dosing regimens may be determined by the medical practitioner overseeing the administration of the compounds, depending upon the particular condition or the patient, the extent or duration of the therapy being administered, *etc.*

The tissue scaffolds disclosed herein are not in any way limited to use only in humans, or even to primates, or mammals. In certain embodiments, the methods and implantable matrices disclosed herein may be employed in the surgical intervention of avian, amphibian, reptilian, and/or other animal species, and may be formulated for veterinary surgical use, including, without limitation, for implantation into selected livestock, exotic or domesticated animals, companion animals (including pets and such like), non-human primates, as well as zoological or otherwise captive specimens, and such like.

### COMPOSITIONS FOR THE PREPARATION OF MEDICAMENTS

Another important aspect of the present invention concerns methods for using the disclosed tissue scaffold compositions (as well as formulations including them) in the preparation of medicaments for treating and/or ameliorating one or more symptoms of one or more diseases, dysfunctions, abnormal conditions, or disorders in an animal, including, for example, vertebrate mammals. Use of the disclosed compositions is particularly contemplated in the treatment of one or more defects in human bone tissue.

Such use generally involves administration to the mammal in need thereof one or more of the disclosed bone tissue scaffolding compositions, in an amount and for a time sufficient to treat or ameliorate one or more symptoms of a bone injury, defect, or disease in an affected mammal.

Pharmaceutical formulations including one or more of the disclosed trizonal compositions also form part of the present invention, and particularly those compositions that further include at least a first pharmaceutically-acceptable excipient for use in the therapy and/or amelioration of one or more symptoms of bone disease or bone defect in an affected mammal.

### EXEMPLARY DEFINITIONS

In accordance with the present invention, polynucleotides, nucleic acid segments, nucleic acid sequences, and the like, include, but are not limited to, DNAs (including and not limited to genomic or extragenomic DNAs), genes, peptide nucleic acids (PNAs) RNAs (including, but not limited to, rRNAs, mRNAs and tRNAs), nucleosides, and suitable nucleic acid segments either obtained from natural sources, chemically synthesized, modified, or otherwise prepared or synthesized in whole or in part by the hand of man.

Unless defined otherwise, all technical and scientific terms used herein have the same meaning as commonly understood by one of ordinary skill in the art to which this invention belongs. The following references provide one of skill with a general definition of many of the terms used in this invention: Dictionary of Biochemistry and Molecular Biology, (2nd Ed.) J. Stenesh (Ed.), Wiley-Interscience (1989); Dictionary of Microbiology and Molecular Biology (3rd Ed.), P. Singleton and D. Sainsbury (Eds.), Wiley-Interscience (2007); Chambers Dictionary of Science and Technology (2nd Ed.), P. Walker (Ed.), Chambers (2007); Glossary of Genetics (5th Ed.), R. Rieger et al. (Eds.), Springer-Verlag (1991); and The HarperCollins Dictionary of Biology, W.G. Hale and J.P. Margham, (Eds.), HarperCollins (1991).

**Although** any methods and compositions similar or equivalent to those described herein can be used in the practice or testing of the present invention, the preferred methods, and compositions are described herein. For purposes of the present invention, the following terms are defined below for sake of clarity and ease of reference:

In accordance with long standing patent law convention, the words "a" and "an," when used in this application, including the claims, denote "one or more."

The terms "about" and "approximately" as used herein, are interchangeable, and should generally be understood to refer to a range of numbers around a given number, as well as to all numbers in a recited range of numbers (*e.g.,* "about 5 to 15" means "about 5 to about 15" unless otherwise stated). Moreover, all numerical ranges herein should be understood to include each whole integer within the range.

As used herein, "bioactive" shall include a quality of a material such that the material has an osteointegrative potential, or in other words the ability to bond with bone. Generally, materials that are bioactive develop an adherent interface with tissues that resist substantial mechanical forces.

As used herein, a "biocompatible" material is a synthetic or natural material used to replace part of a living system or to function in intimate contact with living tissue. Biocompatible materials are intended to interface with biological systems to evaluate, treat, augment, or replace any tissue, organ, or function of the body. The biocompatible material has the ability to perform with an appropriate host response in a specific application and does not have toxic or injurious effects on biological systems. One example of a biocompatible material can be a biocompatible ceramic.

The term "biologically-functional equivalent" is well understood in the art, and is further defined in detail herein. Accordingly, sequences that have about 85% to about 90%; or more preferably, about 91% to about 95%; or even more preferably, about 96% to about 99%; of nucleotides that are identical or functionally-equivalent to one or more of the nucleotide sequences provided herein are particularly contemplated to be useful in the practice of the methods and compositions set forth in the instant application.

As used herein, "biomimetic" shall mean a resemblance of a synthesized material to a substance that occurs naturally in a human body and which is not rejected by (*e.g.,* does not cause an adverse reaction in) the human body.

As used herein, the term "buffer" includes one or more compositions, or aqueous solutions thereof, that resist fluctuation in the pH when an acid or an alkali is added to the solution or composition that includes the buffer. This resistance to pH change is due to the buffering properties of such solutions, and may be a function of one or more specific compounds included in the composition. Thus, solutions or other compositions exhibiting buffering activity are referred to as buffers or buffer solutions. Buffers generally do not have an unlimited ability to maintain the pH of a solution or composition; rather, they are typically able to maintain the pH within certain ranges, for example from a pH of about 5 to 7.

As used herein, the term "carrier" is intended to include any solvent(s), dispersion medium, coating(s), diluent(s), buffer(s), isotonic agent(s), solution(s), suspension(s), colloid(s), inert (s), or such like, or a combination thereof that is pharmaceutically acceptable for administration to the relevant animal or acceptable for a therapeutic or diagnostic purpose, as applicable.

As used herein, "chondrocyte" shall mean a differentiated cell responsible for secretion of extracellular matrix of cartilage. Preferably, the cells are from a compatible human donor. More preferably, the cells are from the patient (*i.e.,* autologous cells).

As used herein, the term "DNA segment" refers to a DNA molecule that has been isolated free of total genomic DNA of a particular species. Therefore, a DNA segment obtained from a biological sample using one of the compositions disclosed herein refers to one or more DNA segments that have been isolated away from, or purified free from, total genomic DNA of the particular species from which they are obtained. Included within the term "DNA segment," are DNA segments and smaller fragments of such segments, as well as recombinant vectors, including, for example, plasmids, cosmids, phage, viruses, and the like.

The term "effective amount," as used herein, refers to an amount that is capable of treating or ameliorating a disease or condition or otherwise capable of producing an intended therapeutic effect.

As used herein, "fibroblast" shall mean a cell of connective tissue that secretes proteins and molecular collagen including fibrillar procollagen, fibronectin and collagenase, from which an extracellular fibrillar matrix of connective tissue may be formed. Fibroblasts synthesize and maintain the extracellular matrix of many tissues, including but not limited to connective tissue. The fibroblast cell may be mesodermally derived, and secrete proteins and molecular collagen including fibrillar procollagen, fibronectin and collagenase, from which an extracellular fibrillar matrix of connective tissue may be formed. A "fibroblast-like cell" means a cell that shares certain characteristics with a fibroblast (such as expression of certain proteins).

The terms "for example" or *"e.g.,"* as used herein, are used merely by way of example, without limitation intended, and should not be construed as referring only those items explicitly enumerated in the specification.

As used herein, "hard tissue" is intended to include mineralized tissues, such as bone, teeth, and cartilage. Mineralized tissues are biological tissues that incorporate minerals into soft matrices.

As used herein, a "heterologous" sequence is defined in relation to a predetermined, reference sequence, such as, a polynucleotide or a polypeptide sequence. For example, with respect to a structural gene sequence, a heterologous promoter is defined as a promoter which does not naturally occur adjacent to the referenced structural gene, but which is positioned by laboratory manipulation. Likewise, a heterologous gene or nucleic acid segment is defined as a gene or segment that does not naturally occur adjacent to the referenced promoter and/or enhancer elements.

As used herein, "homologous" means, when referring to polynucleotides, sequences that have the same essential nucleotide sequence, despite arising from different origins. Typically, homologous nucleic acid sequences are derived from closely related genes or organisms possessing one or more substantially similar genomic sequences. By contrast, an "analogous" polynucleotide is one that shares the same function with a polynucleotide from a different species or organism, but may have a significantly different primary nucleotide sequence that encodes one or more proteins or polypeptides that accomplish similar functions or possess similar biological activity. Analogous polynucleotides may often be derived from two or more organisms that are not closely related (*e.g.,* either genetically or phylogenetically).

As used herein, the term "homology" refers to a degree of complementarity between two or more polynucleotide or polypeptide sequences. The word "identity" may substitute for the word "homology" when a first nucleic acid or amino acid sequence has the exact same primary sequence as a second nucleic acid or amino acid sequence. Sequence homology and sequence identity can be determined by analyzing two or more sequences using algorithms and computer programs known in the art. Such methods may be used to assess whether a given sequence is identical or homologous to another selected sequence.

The terms "identical" or percent "identity," in the context of two or more nucleic acid or polypeptide sequences, refer to two or more sequences or subsequences that are the same or have a specified percentage of amino acid residues or nucleotides that are the same, when compared and aligned for maximum correspondence, as measured using one of the sequence comparison algorithms described below (or other algorithms available to persons of ordinary skill) or by visual inspection.

As used herein, "implantable" or "suitable for implantation" means surgically appropriate for insertion into the body of a host, e.g., biocompatible, or having the desired design and physical properties.

As used herein, the phrase "in need of treatment" refers to a judgment made by a caregiver such as a physician or veterinarian that a patient requires (or will benefit in one or more ways) from treatment. Such judgment may made based on a variety of factors that are in the realm of a caregiver's expertise, and may include the knowledge that the patient is ill as the result of a disease state that is treatable by one or more compound or pharmaceutical compositions such as those set forth herein.

The phrases "isolated" or "biologically pure" refer to material that is substantially, or essentially, free from components that normally accompany the material as it is found in its native state.

As used herein, the term "kit" may be used to describe variations of the portable, self-contained enclosure that includes at least one set of reagents, components, or pharmaceutically-formulated compositions to conduct one or more of the assay methods of the present invention. Optionally, such kit may include one or more sets of instructions for use of the enclosed reagents, such as, for example, in a laboratory or clinical application.

"Link" or "join" refers to any method known in the art for functionally connecting one or more proteins, peptides, nucleic acids, or polynucleotides, including, without limitation, recombinant fusion, covalent bonding, disulfide bonding, ionic bonding, hydrogen bonding, electrostatic bonding, and the like.

As used herein, "matrix" shall mean a three-dimensional structure fabricated with biomaterials. The biomaterials can be biologically-derived or synthetic.

As used herein, a "medical prosthetic device," "medical implant," "implant," and such like, relate to a device intended to be implanted into the body of a vertebrate animal, such as a mammal, and in particular a human. Implants in the present context may be used to replace anatomy and/or restore any function of the body. Examples of such devices include, but are not limited to, dental implants and orthopedic implants. In the present context, orthopedic implants includes within its scope any device intended to be implanted into the body of a vertebrate animal, in particular a mammal such as a human, for preservation and restoration of the function of the musculoskeletal system, particularly joints and bones, including the alleviation of pain in these structures.

In the present context, dental implants include any device intended to be implanted into the oral cavity of a vertebrate animal, in particular a mammal such as a human, in tooth restoration procedures. Generally, a dental implant is composed of one or several implant parts. For instance, a dental implant usually comprises a dental fixture coupled to secondary implant parts, such as an abutment and/or a dental restoration such as a crown, bridge, or denture. However, any device, such as a dental fixture, intended for implantation may alone be referred to as an implant even if other parts are to be connected thereto. Orthopedic and dental implants may also be denoted as orthopedic and dental prosthetic devices as is clear from the above.The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by the hand of man in a laboratory is naturally-occurring. As used herein, laboratory strains of rodents that may have been selectively bred according to classical genetics are considered naturally-occurring animals.

As used herein, "mesh" means a network of material. The mesh may be woven synthetic fibers, non-woven synthetic fibers, nanofibers, or any combination thereof, or any material suitable for implantation into a mammal, and in particular, for implantation into a human.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by the hand of man in a laboratory is naturally-occurring. As used herein, laboratory strains of rodents that may have been selectively bred according to classical genetics are considered naturally-occurring animals.

As used herein, the term "nucleic acid" includes one or more types of: polydeoxyribonucleotides (containing 2-deoxy-D-ribose), polyribonucleotides (containing D-ribose), and any other type of polynucleotide that is an N-glycoside of a purine or pyrimidine base, or modified purine or pyrimidine bases (including abasic sites). The term "nucleic acid," as used herein, also includes polymers of ribonucleosides or deoxyribonucleosides that are covalently bonded, typically by phosphodiester linkages between subunits, but in some cases by phosphorothioates, methylphosphonates, and the like. "Nucleic acids" include single- and double-stranded DNA, as well as single- and double-stranded RNA. Exemplary nucleic acids include, without limitation, gDNA; hnRNA; mRNA; rRNA, tRNA, micro RNA (miRNA), small interfering RNA (siRNA), small nucleolar RNA (snORNA), small nuclear RNA (snRNA), and small temporal RNA (stRNA), and the like, and any combination thereof.

The term "operably linked," as used herein, refers to that the nucleic acid sequences being linked are typically contiguous, or substantially contiguous, and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

As used herein, "osteoblast" shall mean a bone-forming cell which forms an osseous matrix in which it becomes enclosed as an osteocyte. It may be derived from mesenchymal osteoprogenitor cells. The term may also be used broadly to encompass osteoblast-like, and related, cells, such as osteocytes and osteoclasts. An "osteoblast-like cell" means a cell that shares certain characteristics with an osteoblast (such as expression of certain proteins unique to bones), but is not an osteoblast. "Osteoblast-like cells" include preosteoblasts and osteoprogenitor cells. Preferably the cells are from a compatible human donor. More preferably, the cells are from the patient (*i.e.,* autologous cells).

As used herein, "osteointegrative" means having the ability to chemically bond to bone.

As used herein, the term "patient" (also interchangeably referred to as "host" or "subject"), refers to any host that can serve as a recipient of one or more of the therapeutic or diagnostic formulations as discussed herein. In certain aspects, the patient is a vertebrate animal, which is intended to denote any animal species (and preferably, a mammalian species such as a human being). In certain embodiments, a patient may be any animal host, including but not limited to, human and non-human primates, avians, reptiles, amphibians, bovines, canines, caprines, cavines, corvines, epines, equines, felines, hircines, lapines, leporines, lupines, murines, ovines, porcines, racines, vulpines, and the like, including, without limitation, domesticated livestock, herding or migratory animals or birds, exotics or zoological specimens, as well as companion animals, pets, or any animal under the care of a veterinary or animal medical care practitioner.

The phrase "pharmaceutically-acceptable" refers to molecular entities and compositions that preferably do not produce an allergic or similar untoward reaction when administered to a mammal, and in particular, when administered to a human. As used herein, "pharmaceutically acceptable salt" refers to a salt that preferably retains the desired biological activity of the parent compound and does not impart any undesired toxicological effects. Examples of such salts include, without limitation, acid addition salts formed with inorganic acids (*e.g.,* hydrochloric acid, hydrobromic acid, sulfuric acid, phosphoric acid, nitric acid, and the like); and salts formed with organic acids including, without limitation, acetic acid. oxalic acid, tartaric acid, succinic acid, maleic acid, fumaric acid, gluconic acid, citric acid, malic acid, ascorbic acid, benzoic acid, tannic acid, pamoic (embonic) acid, alginic acid, naphthoic acid, polyglutamic acid, naphthalenesulfonic acids, naphthalenedisulfonic acids, polygalacturonic acid; salts with polyvalent metal cations such as zinc, calcium, bismuth, barium, magnesium, aluminum, copper, cobalt, nickel, cadmium, and the like; salts formed with an organic cation formed from *N,N'*-dibenzylethylenediamine or ethylenediamine; and combinations thereof.

The term "pharmaceutically-acceptable salt" as used herein refers to a compound of the present disclosure derived from pharmaceutically acceptable bases, inorganic or organic acids. Examples of suitable acids include, but are not limited to, hydrochloric, hydrobromic, sulfuric, nitric, perchloric, fumaric, maleic, phosphoric, glycollic, lactic, salicyclic, succinic, toluene-*p*-sulfonic, tartaric, acetic, citric, methanesulfonic, formic, benzoic, malonic, naphthalene-2-sulfonic, trifluoroacetic and benzenesulfonic acids. Salts derived from appropriate bases include, but are not limited to, alkali such as sodium and ammonia.

As used herein, the term "plasmid" or "vector" refers to a genetic construct that is composed of genetic material (*i.e.,* nucleic acids). Typically, a plasmid or a vector contains an origin of replication that is functional in bacterial host cells, *e.g., Escherichia coli,* and selectable markers for detecting bacterial host cells including the plasmid. Plasmids and vectors of the present invention may include one or more genetic elements as described herein arranged such that an inserted coding sequence can be transcribed and translated in a suitable expression cells. In addition, the plasmid or vector may include one or more nucleic acid segments, genes, promoters, enhancers, activators, multiple cloning regions, or any combination thereof, including segments that are obtained from or derived from one or more natural and/or artificial sources.

As used herein, "polymer" means a chemical compound or mixture of compounds formed by polymerization and including repeating structural units. Polymers may be constructed in multiple forms and compositions or combinations of compositions.

As used herein, the term "polypeptide" is intended to encompass a singular "polypeptide" as well as plural "polypeptides," and includes any chain or chains of two or more amino acids. Thus, as used herein, terms including, but not limited to "peptide," "dipeptide," "tripeptide," "protein," "enzyme," "amino acid chain," and "contiguous amino acid sequence" are all encompassed within the definition of a "polypeptide," and the term "polypeptide" can be used instead of, or interchangeably with, any of these terms. The term further includes polypeptides that have undergone one or more post-translational modification(s), including for example, but not limited to, glycosylation, acetylation, phosphorylation, amidation, derivatization, proteolytic cleavage, post-translation processing, or modification by inclusion of one or more non-naturally occurring amino acids. Conventional nomenclature exists in the art for polynucleotide and polypeptide structures.

For example, one-letter and three-letter abbreviations are widely employed to describe amino acids: Alanine (A; Ala), Arginine (R; Arg), Asparagine (N; Asn), Aspartic Acid (D; Asp), Cysteine (C; Cys), Glutamine (Q; Gln), Glutamic Acid (E; Glu), Glycine (G; Gly), Histidine (H; His), Isoleucine (I; Ile), Leucine (L; Leu), Methionine (M; Met), Phenylalanine (F; Phe), Proline (P; Pro), Serine (S; Ser), Threonine (T; Thr), Tryptophan (W; Trp), Tyrosine (Y; Tyr), Valine (V; Val), and Lysine (K; Lys). Amino acid residues described herein are preferred to be in the "L" isomeric form. However, residues in the "D" isomeric form may be substituted for any L-amino acid residue provided the desired properties of the polypeptide are retained.

As used herein, the terms "prevent," "preventing," "prevention," "suppress," "suppressing," and "suppression" as used herein refer to administering a compound either alone or as contained in a pharmaceutical composition prior to the onset of clinical symptoms of a disease state so as to prevent any symptom, aspect or characteristic of the disease state. Such preventing and suppressing need not be absolute to be deemed medically useful.

As used herein, "porosity" means the ratio of the volume of interstices of a material to a volume of a mass of the material.

"Protein" is used herein interchangeably with "peptide" and "polypeptide," and includes both peptides and polypeptides produced synthetically, recombinantly, or in vitro and peptides and polypeptides expressed *in vivo* after nucleic acid sequences are administered into a host animal or human subject. The term "polypeptide" is preferably intended to refer to any amino acid chain length, including those of short peptides from about two to about 20 amino acid residues in length, oligopeptides from about 10 to about 100 amino acid residues in length, and longer polypeptides including from about 100 amino acid residues or more in length. Furthermore, the term is also intended to include enzymes, *i.e.,* functional biomolecules including at least one amino acid polymer. Polypeptides and proteins of the present invention also include polypeptides and proteins that are or have been post-translationally modified, and include any sugar or other derivative(s) or conjugate(s) added to the backbone amino acid chain.

"Purified," as used herein, means separated from many other compounds or entities. A compound or entity may be partially purified, substantially purified, or pure. A compound or entity is considered pure when it is removed from substantially all other compounds or entities, *i.e.,* is preferably at least about 90%, more preferably at least about 91%, 92%, 93%, 94%, 95%, 96%, 97%, 98%, 99%, or greater than 99% pure. A partially or substantially purified compound or entity may be removed from at least 50%, at least 60%, at least 70%, or at least 80% of the material with which it is naturally found, *e.g.,* cellular material such as cellular proteins and/or nucleic acids.

The term "recombinant" indicates that the material (*e.g.,* a polynucleotide or a polypeptide) has been artificially or synthetically (non-naturally) altered by human intervention. The alteration can be performed on the material within or removed from, its natural environment, or native state. Specifically, *e.g.,* a promoter sequence is "recombinant" when it is produced by the expression of a nucleic acid segment engineered by the hand of man. For example, a "recombinant nucleic acid" is one that is made by recombining nucleic acids, *e.g.,* during cloning, DNA shuffling or other procedures, or by chemical or other mutagenesis; a "recombinant polypeptide" or "recombinant protein" is a polypeptide or protein which is produced by expression of a recombinant nucleic acid; and a "recombinant virus," *e.g.,* a recombinant AAV virus, is produced by the expression of a recombinant nucleic acid.

The term "regulatory element," as used herein, refers to a region or regions of a nucleic acid sequence that regulates transcription. Exemplary regulatory elements include, but are not limited to, enhancers, post-transcriptional elements, transcriptional control sequences, and such like.

The term "RNA segment" refers to an RNA molecule that has been isolated free of total cellular RNA of a particular species. Therefore, RNA segments can refer to one or more RNA segments (either of native or synthetic origin) that have been isolated away from, or purified free from, other RNAs. Included within the term "RNA segment," are RNA segments and smaller fragments of such segments.

The term "a sequence essentially as set forth in SEQ ID NO:X" means that the sequence substantially corresponds to a portion of SEQ ID NO:X and has relatively few nucleotides (or amino acids in the case of polypeptide sequences) that are not identical to, or a biologically functional equivalent of, the nucleotides (or amino acids) of SEQ ID NO:X. The term "biologically functional equivalent" is well understood in the art, and is further defined in detail herein. Accordingly, sequences that have about 85% to about 90%; or more preferably, about 91 % to about 95%; or even more preferably, about 96% to about 99%; of nucleotides that are identical or functionally equivalent to one or more of the nucleotide sequences provided herein are particularly contemplated to be useful in the practice of the invention.

Suitable standard hybridization conditions for nucleic acids for use in the present invention include, for example, hybridization in 50% formamide, 5× Denhardt's solution, 5×SSC, 25 mM sodium phosphate, 0.1% SDS and 100 µg/mL of denatured salmon sperm DNA at 42°C for 16 hr followed by 1 hr sequential washes with 0.1× SSC, 0.1% SDS solution at 60°C to remove the desired amount of background signal. Lower stringency hybridization conditions for the present invention include, for example, hybridization in 35% formamide, 5×Denhardt's solution, 5× SSC, 25 mM sodium phosphate, 0.1% SDS and 100 µg_{/}mL denatured salmon sperm DNA or *E*. *coli* DNA at 42°C for 16 hr followed by sequential washes with 0.8× SSC, 0.1% SDS at 55°C. Those of ordinary skill in the art will recognize that such hybridization conditions can be readily adjusted to obtain the desired level of stringency for a particular application.

As used herein, "scaffold," relates to an open porous structure. A scaffold may comprise one or more building materials to create the structure of the scaffold. Additionally, the scaffold may further comprise other substances, such as one or more biologically active molecules or such like.

As used herein, "soft tissue" is intended to include tissues that connect, support, or surround other structures and organs of the body, not being bone. Soft tissue includes ligaments, tendons, fascia, skin, fibrous tissues, fat, synovial membranes, epithelium, muscles, nerves and blood vessels.

As used herein, "stem cell" means an unspecialized cell that has the potential to develop into many different cell types in the body, such as mesenchymal osteoprogenitor cells, osteoblasts, osteocytes, osteoclasts, chondrocytes, and chondrocyte progenitor cells. Preferably, the cells are from a compatible human donor. More preferably, the cells are from the patient (*i.e.,* autologous cells).

As used herein, the term "structural gene" is intended to generally describe a polynucleotide, such as a gene, that is expressed to produce an encoded peptide, polypeptide, protein, ribozyme, catalytic RNA molecule, or antisense molecule.

The term "subject," as used herein, describes an organism, including mammals such as primates, to which treatment with the compositions according to the present invention can be provided. Mammalian species that can benefit from the disclosed methods of treatment include, but are not limited to, apes; chimpanzees; orangutans; humans; monkeys; domesticated animals such as dogs and cats; livestock such as horses, cattle, pigs, sheep, goats, and chickens; and other animals such as mice, rats, guinea pigs, and hamsters.

The term "substantially complementary," when used to define either amino acid or nucleic acid sequences, means that a particular subject sequence, for example, an oligonucleotide sequence, is substantially complementary to all or a portion of the selected sequence, and thus will specifically bind to a portion of an mRNA encoding the selected sequence. As such, typically the sequences will be highly complementary to the mRNA "target" sequence, and will have no more than about 1, about 2, about 3, about 4, about 5, about 6, about 7, about 8, about 9, or about 10 or so base mismatches throughout the complementary portion of the sequence. In many instances, it may be desirable for the sequences to be exact matches, *i.e.,* be completely complementary to the sequence to which the oligonucleotide specifically binds, and therefore have zero mismatches along the complementary stretch. As such, highly complementary sequences will typically bind quite specifically to the target sequence region of the mRNA and will therefore be highly efficient in reducing, and/or even inhibiting the translation of the target mRNA sequence into polypeptide product.

Substantially complementary nucleic acid sequences will be greater than about 80 percent complementary (or "% exact-match") to a corresponding nucleic acid target sequence to which the nucleic acid specifically binds, and will, more preferably be greater than about 85 percent complementary to the corresponding target sequence to which the nucleic acid specifically binds. In certain aspects, as described above, it will be desirable to have even more substantially complementary nucleic acid sequences for use in the practice of the invention, and in such instances, the nucleic acid sequences will be greater than about 90 percent complementary to the corresponding target sequence to which the nucleic acid specifically binds, and may in certain embodiments be greater than about 95 percent complementary to the corresponding target sequence to which the nucleic acid specifically binds, and even up to and including about 96%, about 97%, about 98%, about 99%, and even about 100% exact match complementary to all or a portion of the target sequence to which the designed nucleic acid specifically binds.

Percent similarity or percent complementary of any of the disclosed nucleic acid sequences may be determined, for example, by comparing sequence information using the GAP computer program, version 6.0, available from the University of Wisconsin Genetics Computer Group (UWGCG). The GAP program utilizes the alignment method of Needleman and Wunsch (1970). Briefly, the GAP program defines similarity as the number of aligned symbols (*i.e.,* nucleotides or amino acids) that are similar, divided by the total number of symbols in the shorter of the two sequences. The preferred default parameters for the GAP program include: (1) a unary comparison matrix (containing a value of 1 for identities and 0 for non-identities) for nucleotides, and the weighted comparison matrix of Gribskov and Burgess (1986), (2) a penalty of 3.0 for each gap and an additional 0.10 penalty for each symbol in each gap; and (3) no penalty for end gaps.

As used herein, the term "substantially free" or "essentially free" in connection with the amount of a component preferably refers to a composition that contains less than about 10 weight percent, preferably less than about 5 weight percent, and more preferably less than about 1 weight percent of a compound. In preferred embodiments, these terms refer to less than about 0.5 weight percent, less than about 0.1 weight percent, or less than about 0.01 weight percent.

As used herein, the term "substantially free" or "essentially free" in connection with the amount of a component preferably refers to a composition that contains less than about 10 weight percent, preferably less than about 5 weight percent, and more preferably less than about 1 weight percent of a compound. In preferred embodiments, these terms refer to less than about 0.5 weight percent, less than about 0.1 weight percent, or less than about 0.01 weight percent.

The terms "substantially corresponds to," "substantially homologous," or "substantial identity," as used herein, denote characteristics of a nucleic acid or an amino acid sequence, wherein a selected nucleic acid sequence or a selected amino acid sequence has at least about 70 or about 75 percent sequence identity as compared to a selected reference nucleic acid or amino acid sequence. More typically, the selected sequence and the reference sequence will have at least about 76, 77, 78, 79, 80, 81, 82, 83, 84 or even 85 percent sequence identity, and more preferably, at least about 86, 87, 88, 89, 90, 91, 92, 93, 94, or 95 percent sequence identity. More preferably still, highly homologous sequences often share greater than at least about 96, 97, 98, or 99 percent sequence identity between the selected sequence and the reference sequence to which it was compared.

As used herein, "synthetic" shall mean that the material is not of a human or animal origin.

The term "therapeutically-practical period" means the period of time that is necessary for one or more active agents to be therapeutically effective. The term "therapeutically-effective" refers to reduction in severity and/or frequency of one or more symptoms, elimination of one or more symptoms and/or underlying cause, prevention of the occurrence of symptoms and/or their underlying cause, and the improvement or a remediation of damage.

A "therapeutic agent" may be any physiologically or pharmacologically active substance that may produce a desired biological effect in a targeted site in a subject. The therapeutic agent may be a chemotherapeutic agent, an immunosuppressive agent, a cytokine, a cytotoxic agent, a nucleolytic compound, a radioactive isotope, a receptor, and a pro-drug activating enzyme, which may be naturally occurring, produced by synthetic or recombinant methods, or a combination thereof. Drugs that are affected by classical multidrug resistance, such as vinca alkaloids (*e.g.,* vinblastine and vincristine), the anthracyclines (*e.g.,* doxorubicin and daunorubicin), RNA transcription inhibitors (*e.g.,* actinomycin-D) and microtubule stabilizing drugs (*e.g.,* paclitaxel) may have particular utility as the therapeutic agent. Cytokines may be also used as the therapeutic agent. Examples of such cytokines are lymphokines, monokines, and traditional polypeptide hormones. A cancer chemotherapy agent may be a preferred therapeutic agent. For a more detailed description of anticancer agents and other therapeutic agents, those skilled in the art are referred to any number of instructive manuals including, but not limited to, the Physician's Desk Reference and Hardman and Limbird (2001).

As used herein, a "transcription factor recognition site" and a "transcription factor binding site" refer to a polynucleotide sequence(s) or sequence motif(s), which are identified as being sites for the sequence-specific interaction of one or more transcription factors, frequently taking the form of direct protein-DNA binding. Typically, transcription factor binding sites can be identified by DNA footprinting, gel mobility shift assays, and the like, and/or can be predicted based on known consensus sequence motifs, or by other methods known to those of ordinary skill in the art.

"Transcriptional regulatory element" refers to a polynucleotide sequence that activates transcription alone or in combination with one or more other nucleic acid sequences. A transcriptional regulatory element can, for example, comprise one or more promoters, one or more response elements, one or more negative regulatory elements, and/or one or more enhancers.

"Transcriptional unit" refers to a polynucleotide sequence that comprises at least a first structural gene operably linked to at least a first *cis-*acting promoter sequence and optionally linked operably to one or more other *cis*-acting nucleic acid sequences necessary for efficient transcription of the structural gene sequences, and at least a first distal regulatory element as may be required for the appropriate tissue-specific and developmental transcription of the structural gene sequence operably positioned under the control of the promoter and/or enhancer elements, as well as any additional *cis-*sequences that are necessary for efficient transcription and translation (*e.g.,* polyadenylation site(s), mRNA stability controlling sequence(s), *etc.*

As used herein, the term "transformation" is intended to generally describe a process of introducing an exogenous polynucleotide sequence (*e.g.,* a viral vector, a plasmid, or a recombinant DNA or RNA molecule) into a host cell or protoplast in which the exogenous polynucleotide is incorporated into at least a first chromosome or is capable of autonomous replication within the transformed host cell. Transfection, electroporation, and "naked" nucleic acid uptake all represent examples of techniques used to transform a host cell with one or more polynucleotides.

As used herein, the term "transformed cell" is intended to mean a host cell whose nucleic acid complement has been altered by the introduction of one or more exogenous polynucleotides into that cell.

"Treating" or "treatment of' as used herein, refers to providing any type of medical or surgical management to a subject. Treating can include, but is not limited to, administering a composition comprising a therapeutic agent to a subject. "Treating" includes any administration or application of a compound or composition of the invention to a subject for purposes such as curing, reversing, alleviating, reducing the severity of, inhibiting the progression of, or reducing the likelihood of a disease, disorder, or condition or one or more symptoms or manifestations of a disease, disorder, or condition. In certain aspects, the compositions of the present invention may also be administered prophylactically, *i.e.,* before development of any symptom or manifestation of the condition, where such prophylaxis is warranted. Typically, in such cases, the subject will be one that has been diagnosed for being "at risk" of developing such a disease or disorder, either as a result of familial history, medical record, or the completion of one or more diagnostic or prognostic tests indicative of a propensity for subsequently developing such a disease or disorder.

The tern "vector," as used herein, refers to a nucleic acid molecule (typically comprised of DNA) capable of replication in a host cell and/or to which another nucleic acid segment can be operatively linked so as to bring about replication of the attached segment. A plasmid, cosmid, or a virus is an exemplary vector.

In certain embodiments, it will be advantageous to employ one or more nucleic acid segments of the present invention in combination with an appropriate detectable marker (*i.e.,* a "label,"), such as in the case of employing labeled polynucleotide probes in determining the presence of a given target sequence in a hybridization assay. A wide variety of appropriate indicator compounds and compositions are known in the art for labeling oligonucleotide probes, including, without limitation, fluorescent, radioactive, enzymatic or other ligands, such as avidin/biotin, *etc.,* which are capable of being detected in a suitable assay. In particular embodiments, one may also employ one or more fluorescent labels or an enzyme tag such as urease, alkaline phosphatase or peroxidase, instead of radioactive or other environmentally less-desirable reagents. In the case of enzyme tags, colorimetric, chromogenic, or fluorogenic indicator substrates are known that can be employed to provide a method for detecting the sample that is visible to the human eye, or by analytical methods such as scintigraphy, fluorimetry, spectrophotometry, and the like, to identify specific hybridization with samples containing one or more complementary or substantially complementary nucleic acid sequences. In the case of so-called "multiplexing" assays, where two or more labeled probes are detected either simultaneously or sequentially, it may be desirable to label a first oligonucleotide probe with a first label having a first detection property or parameter (for example, an emission and/or excitation spectral maximum), which also labeled a second oligonucleotide probe with a second label having a second detection property or parameter that is different (*i.e.,* discreet or discernible from the first label. The use of multiplexing assays, particularly in the context of genetic amplification/detection protocols are well-known to those of ordinary skill in the molecular genetic arts.

### BIOLOGICAL FUNCTIONAL EQUIVALENTS

Modification and changes may be made in the structure of the nucleic acids, or to the vectors comprising them, as well as to mRNAs, polypeptides, or therapeutic agents encoded by them and still obtain functional systems that contain one or more therapeutic agents with desirable characteristics. As mentioned above, it is often desirable to introduce one or more mutations into a specific polynucleotide sequence. In certain circumstances, the resulting encoded polypeptide sequence is altered by this mutation, or in other cases, the sequence of the polypeptide is unchanged by one or more mutations in the encoding polynucleotide.

When it is desirable to alter the amino acid sequence of a polypeptide to create an equivalent, or even an improved, second-generation molecule, the amino acid changes may be achieved by changing one or more of the codons of the encoding DNA sequence, according to Table 1.

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated by the inventors that various changes may be made in the peptide sequences of the disclosed compositions or corresponding DNA sequences which encode said peptides without appreciable loss of their biological utility or activity.

**TABLE 1**

| | AMINO ACIDS | CODONS | | | | | |
|---|---|---|---|---|---|---|---|
| Alanine | Ala | GCA | GCC | GCG | GCU | | |
| Cysteine | Cys | UGC | UGU | | | | |
| Aspartic acid | Asp | GAC | GAU | | | | |
| Glutamic acid | Glu | GAA | GAG | | | | |
| Phenylalanine | Phe | UUC | UUU | | | | |
| Glycine | Gly | GGA | GGC | GGG | GGU | | |
| Histidine | His | CAC | CAU | | | | |
| Isoleucine | Ile | AUA | AUC | AUU | | | |
| Lysine | Lys | AAA | AAG | | | | |
| Leucine | Leu | UUA | UUG | CUA | CUC | CUG | CUU |
| Methionine | Met | AUG | | | | | |
| Asparagine | Asn | AAC | AAU | | | | |
| Proline | Pro | CCA | CCC | CCG | CCU | | |
| Glutamine | Gln | CAA | CAG | | | | |
| Arginine | Arg | AGA | AGG | CGA | CGC | CGG | CGU |
| Serine | Ser | AGC | AGU | UCA | UCC | UCG | UCU |
| Threonine | Thr | ACA | ACC | ACG | ACU | | |
| Valine | Val | GUA | GUC | GUG | GUU | | |
| Tryptophan | Trp | UGG | | | | | |
| Tyrosine | Tyr | UAC | UAU | | | | |

In making such changes, the hydropathic index of amino acids may be considered. The importance of the hydropathic amino acid index in conferring interactive biologic function on a protein is generally understood in the art (Kyte and Doolittle, 1982, incorporate herein by reference). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index based on its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i.e.* still obtain a biological functionally equivalent protein. In making such changes, the substitution of amino acids whose hydropathic indices are within ±2 is preferred, those within ±1 are particularly preferred, and those within ±0.5 are even more particularly preferred. It is also understood in the art that the substitution of like amino acids can be made effectively based on hydrophilicity. U.S. Patent No. 4,554,101 (specifically incorporated herein in its entirety by express reference thereto), states that the greatest local average hydrophilicity of a protein, as governed by the hydrophilicity of its adjacent amino acids, correlates with a biological property of the protein.

As detailed in U.S. Patent No. 4,554,101, the following hydrophilicity values have been assigned to amino acid residues: arginine (+3.0); lysine (+3.0); aspartate (+3.0 ±1); glutamate (+3.0 ±1); serine (+0.3); asparagine (+0.2); glutamine (+0.2); glycine (0); threonine (-0.4); proline (-0.5 ±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ± 2 is preferred, those within ± 1 are particularly preferred, and those within ± 0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take one or more of the foregoing characteristics into consideration are well known to those of ordinary skill in the art, and include arginine and lysine; glutamate and aspartate; serine and threonine; glutamine and asparagine; and valine, leucine and isoleucine.

The section headings used throughout are for organizational purposes only and are not to be construed as limiting the subject matter described. All documents, or portions of documents, cited in this application (including, but not limited to, patents, patent applications, articles, books, and treatises) are expressly incorporated herein in their entirety by express reference thereto. In the event that one or more of the incorporated literature and similar materials defines a term in a manner that contradicts the definition of that term in this application, this application controls.

### EXAMPLES

The following examples are included to demonstrate preferred embodiments of the invention. It should be appreciated by those of skill in the art that the techniques disclosed in the examples that follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1 - PREPARATION OF TRIZONAL COLLAGEN-BASED SCAFFOLDS

The present example demonstrates preparation of the trizonal membranes in accordance with one aspect of the present invention.

### MATERIALS AND METHODS

***Preparation of the Zone I Membrane:*** Type I collagen (1% collagen gel in acetic acid) from bovine tendon was purchased from Nitta Gelatin NA, Inc. (Morrisville, NC, USA). Collagen films were produced by a solvent-casting method, as previously described (Taraballi *et al.,* 2013). Briefly, the 1% gel was diluted 1:6 wt./vol. in ultrapure water. The suspension was homogenized at 4°C with a mixer for 2 min. After removal of the aggregates by vacuum filtration, 80 mL of collagen solution was poured into an 8.5 × 12.5 cm container, and the solvent evaporated in a fume hood for two days until complete dryness of the samples was obtained.

The membrane is then hydrated with phosphate buffer solution, allowed to swell and placed in the bottom of a silicon mold, which is used for final molding.

***Preparation of the Zone II and Zone III membranes:*** Bovine Type 1 collagen was purchased from Nitta Gelatin. A 1.0 wt% collagen suspension in acetic acid was prepared (pH 3.5). Soluble elastin from bovine was added to the acetic collagen slurry to a final concentration of 10 wt% of elastin. The mixture was then homogenized for 5 min at room temperature, and the blended slurry was precipitated by the addition of NaOH (0.1 M) to a final pH of 5.5. The precipitate was washed three times with deionized water, and then homogenously dispersed in an aqueous solution of the cross-linking agent 1,4-butanediol diglycidyl ether (BDDGE), setting a BDDGE/collagen ratio of 2 wt%, followed by incubation at 20°Cfor 48 hrs. Finally, the material was washed three times with DI, and then placed on top of the thin collagen film, in a silicon mold for casting.

A biohybrid composite membrane composed of collagen and magnesium-doped hydroxyapatite (MgHAp) is synthesized as previously published (Tampieri *et al.,* 2008). The collagen is dissolved at a concentration of 10 mg/mL in an aqueous acetic buffer solution at pH 3.5. The synthesis consisted of the following steps: 244 mL of H₃P0₄ (0.040 M solution was added to 70 g of 1 wt% collagen gel, and dropped in a basic suspension containing 1.203 g of Ca(OH)₂ and 25 mg MgCl₂ (Mg/Ca molar ratio of 5%) in 200 mL of distilled water to obtain an MgHA/Col composite having a ratio of 70:30 (wt%). The acidic suspension is dropped into the basic one under stirring to assure a slow decrease of pH, to neutrality.

The resulting material is cross-linked with 1 wt% BDDGE to stabilize the collagen matrix, and to control porosity and tortuosity (Minardi *et al.,* 2015). Finally, the material is washed three times with deionized water, and then placed on top of the collagen-elastin porous layer, at the slurry state, to apply a knitting procedure to ensure perfect integration of the overall three-layered construct.

Finally, the three-layered construct is cast by an optimized freeze-drying process with a ramp from +20°C to -20°C in 3 hr, and then heated from -20°C to +20°C in 3 hr, under vacuum (0.2 mbar).

The resulting material is able to mimic multifunctional regions (periosteum). The control of the material's structure at the nano-, micro- and macro-scale is of paramount importance to ensure the optimal osteointegration, and to avoid delamination.

### EXAMPLE 2 - IMPLANTING A TRIZONAL COLLAGEN-BASED SCAFFOLD

During a surgical procedure, the proximal periosteum was peeled back, and an osteotomy was performed. Then, the periosteum was sutured in place, forming a sleeve around the PEU shell. However the native periosteum was not wide enough to cover the defect completely (see FIG. 18A, FIG. 18B, FIG. 18C, FIG. 18D, FIG. 18E, FIG. 18F, FIG. 18G, FIG. 18H, FIG. 18I, and FIG. 18J). Under certain conditions such as extended trauma, the periosteum could be damaged or lacking completely. The use of an artificial periosteum capable of supporting regeneration triggered by stem cells is needed for long bone defects. Basic collagen scaffolds have been used to support MSC from periosteum. Thus, the practicality of a biomimetic periosteal surrogate is inferred.

### EXAMPLE 3 - LARGE-SCALE ANIMAL STUDY OF TRIZONAL COLLAGEN-BASED SCAFFOLD IMPLANTS IN SHEEP

The wealth of literature and existing collagen-based products currently in medical use support the collagen shell's utility and biocompatibility, and strongly argue against a possible deleterious effect. Additional results strongly underscore the collagen shell's ability to promote and bridge cell growth in ventral hernia repair and osteoconductivity in spinal fusion applications.

In the standard of care for control sheep, defect repair is performed by internal fixation of the bony tibial ends using permanent implantable Dynamic Compression or Limited Contact Dynamic Compression Plates (DCP or LC-DCP respectively) made of surgical steel or titanium. After assuring that the proximal and distal bony ends are in proper axial alignment and the created defect maintained using careful distal limb traction, plate(s) may be applied to the lateral and/or medial surfaces of the native tibia using a green drill guide for neutral surgical screw placement. Two or more neutral (to avoid unwanted compression and shortening of the critical defect) surgical screws may be used at the proximal and distal bony ends to attach the DCP/LC-DCP. As a correlate to the small amount of polymethyl methacrylate (PMMA) bone cement used as a final anchor for the PEU/collagen shell device in test animals, a similar smear of PMMA may also be used at both the proximal and distal attachment sites of implanted plates.

The skin is approximated with a running subcuticular 4-0 Monocryl absorbable suture and dermabond applied on top of the incision. A modified Schroeder-Thomas heavy support wrap cast and splint is applied intraoperatively prior to recovering the animals. The animals are monitored continuously for a minimum of 24 hours post-operation, and longer if deemed necessary by the veterinary staff. The endpoint for the standard of care controls is determined by the health and welfare of the animals after surgery. The naive control animals do not undergo surgery, and thus serve as baseline for all tests performed.

***Non-GLP Study:*** In an exemplary study, six animals may be analyzed with PEU implants and six animals with PEU plus collagen shell implants. Two control animals (no implants) and four animals with PEU plus collagen shell implants may also be evaluated separately. The samples from these animals may be used for method development in preparation for GLP studies and may include: 16 bones for 6 decalcified sections for histology staining and 2 non-decalcified sections for Stevenel's staining per specimen for the purpose of semi-quantitative evaluation of biocompatibility and bone healing.

***GLP Study:*** On days 31, 91 and 181, four animals per sex/per test article are evaluated (*i.e.* PEU and collagen shells) using both non-decalcified and decalcified samples. On day 91, 3 animals per sex for the standard of care animals is evaluated similarly. On day 366, 8 animals per sex for the test article and one animal per sex for the naive controls are analyzed. For the biocompatibility and bone healing evaluation, three transverse slabs are evaluated from each sample of the tibial defect sites. For studies of undecalcified samples, four whole, transverse sections are produced for analysis. On Day 366, biomechanical testing is performed on eight animals per sex.

To facilitate the analysis of the 72 animals included in this study, a surgical schedule has been developed that staggers surgical procedures and post-operative testing. Animals arrive in groups of eight (4 males ♂, 4 females ♀). Surgical procedures are performed on two animals (1♂, 1♀) each day of surgery. Four surgical procedures are scheduled in a set, performed on back-to-back weeks; two on the same day one week, followed by two on the same day the following week. The time between sets of surgical procedures varies based on the timing of subsequent procedures (*e.g.,* imaging). This surgical schedule takes into consideration all of the activities that occur subsequent to surgery, animal housing availability and the need for adequate staff to perform the necessary intensive post-surgical care.

Appropriate protocols are in place for both pre- and post-operative procedures and care. Immediately after closure of the surgical site and while the animal is still under general anesthesia, the surgeon placees the affected limb in a modified Schroeder-Thomas splint cast, as mentioned above. After immobilization of the limb, anesthesia is discontinued and the animal is transported to a specialized animal holding room for recovery. During recovery from anesthesia continuous monitoring of vitals (HR, RR, SpO₂, temperature, and NIBP) is performed and documented at least every 15 min. Supplemental heat support is provided until the animal is normothermic. The animal is extubated once chewing is exhibited. After extubation, the animal may be placed into sternal recumbency to allow normal eructation. Administration of analgesics begins immediately preoperatively, and continues through the intensive care period, with continuation to be made based on the clinical condition of the animal. For the first 24-72 hrs post-surgically, the animal is maintained under intensive care conditions with 24 hrs/day observation. Interventions and/or further diagnostic procedures needed to address any complication are performed and documented in the medical records.

Depending on the length of stay in intensive care, the animal may undergo general anesthesia for imaging procedures that assess the surgical site. The splint cast may be removed at this point to confirm no infection at the incision site. The splint cast is then replaced during the anesthesia. If the animal has been released from intensive care prior to the imaging event, then it is returned to the room designated for recovery from anesthesia and monitoring.

When intensive care is discontinued, the animals are returned to regular housing. They are housed singly during the period in which they have the splint cast. The splint cast is assessed daily for integrity and to confirm the absence of moisture. The animal is also assessed for signs of ulceration where the splint cast comes into friction with skin. The splint cast is maintained, with a minimum of one full cast replacement, for at least 30 days. The animal is observed regularly for function and weight bearing during this time. At the end of the 30 days, the splint cast is removed following imaging.

After splint cast removal and imaging, the animal is assessed for weight bearing and lameness. Once the animal has returned to an appropriate level of function, it is returned to pair or group housing to provide appropriate social interaction. The animal then continues to be monitored daily for physical and emotional behavior, food/water intake, urine/feces output, and for continued return-to-function of the affected limb. Abnormal findings are reported immediately to the veterinary staff for assessment. If intervention is necessary, the decision is made by the clinical veterinarian in consultation with the attending veterinarian and the principal investigator of the study.

### DISCUSSION

The inventors have extensive experience with complex, fine-tuned collagen-based scaffolds that are applied to a variety of tissue engineering applications, including but not limited to: osteochondral regeneration, spinal fusion, and soft tissues' critical-sized defect for general surgery (the ventral abdominal hernia). Previous data indicate that these engineered scaffolds provide the perfect environment to promote cell population, transmigration, and ultimately, tissue growth across a defect that far supersedes the weak scar that would otherwise form in its absence. With an ability to closely mimic with the collagen shell the three zones of native periosteum, it is believed that the problem of delayed or absent bone regrowth over exposed areas of the PEU implant will be overcome. In the event that the collagen shell does not give additive effect, the remainder of the study with PEU shell alone provides further data on its ability to provide functional recovery, and additional GMP/GLP studies can be similarly amended to include the PEU shell alone and/or the collagen shell further modified accordingly.

Different biomaterials have been proposed as bone substitutes with conflicting results. Among these, hydroxyapatite (HA) and other calcium phosphate ceramics have shown the most promising results due to their osteoconductive properties and absence of immune response. Tampieri and co-workers developed biomimetic multisubstituted apatites (*e.g.,* magnesium and carbonate substituted hydroxyapatite) with enhanced osteoinductivity in a sheep model and led to successful pilot clinical studies in Europe for repairing long-bone loss. The introduction of specific doping ions in the apatite lattice allowed for the enhancement of the bioactivity of the ceramic phase of these hybrid materials, such that the addition of biologics was not required. The efficacy of these materials has been also proven in other models for bone repair, such as for the regeneration of subchondral bone in a sheep model and in humans.

In this example, a new formulation and design of the collagen shell that confers a higher degree of mimicry for spongy bone has been developed and characterized at both the morphological, and the compositional levels. The data indicated that the new trizonal collagen shell could induce a faster and more efficient differentiation of MSC without the use of any biologies.

### EXAMPLE 4 - SPINAL FUSION USING TRIZONAL COLLAGEN-BASED SCAFFOLDS

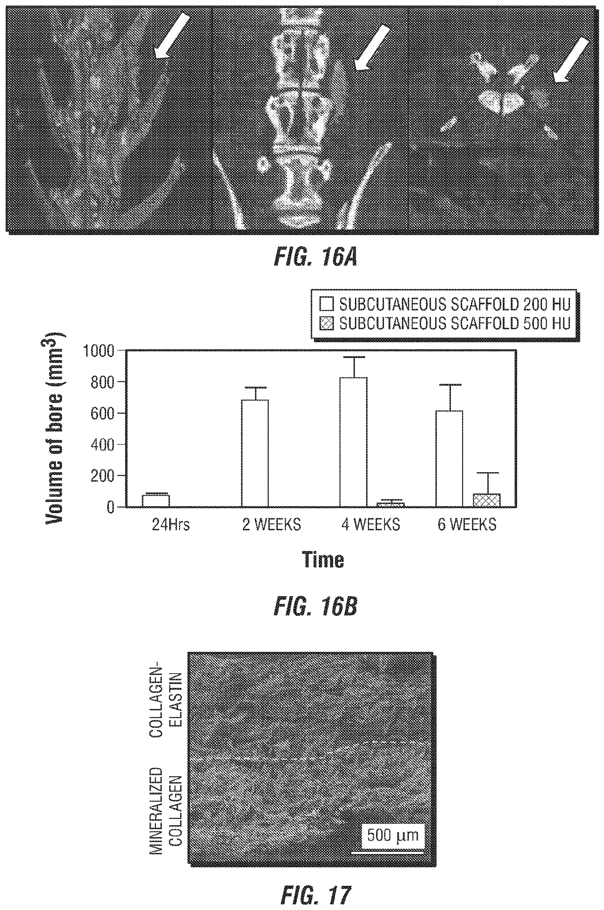

This example describes the use of the trizonal collagen-based scaffolds in reconstructive surgeries, including, for example, spinal fusion (FIG. 16A and FIG. 16B).

Studies show that 70-80% of the general population will suffer from back pain at some point in their lives. From a surgical standpoint, spinal fusion is the selective procedure to improve spine stability and alleviate back pain. The US Agency for Healthcare Research and Quality revealed that from 2003-2012 the number of patients discharged from hospitals after spinal fusion increase of almost 50%, reaching 90,000 cases per year.

BMP-2 is recognized to be the most vital factor in osteogenesis. The current standard of care for spinal fusion requires the use of collagen scaffolds infused with BMP-2 (INFUSE®, Medtronic). Recently, the massive doses of BMP-2 utilized were associated with severe side effects to the tissues surrounding the implant. These doses are about 5000 times higher than that required for bone formation *in vitro* and can cause side effects. For this reason, an approach that is growth factors-free is both beneficial and innovative.

A magnesium-doped hydroxyapatite/type I collagen scaffold (MHA/Coll) has been developed that has proved efficient in promoting new bone formation in an ectopic bone model in rabbit (FIG. 17). *In vitro,* MHA/Coll was tested with human bone marrow-derived mesenchymal stem cells and even higher than the physiologic doses found in the osteogenic niche, where the presence of other osteogenic stimuli (chemical, physical and structural) also contribute to the induction of bone formation.

### EXAMPLE 5 - SKULL REPAIR USING TRIZONAL COLLAGEN-BASED SCAFFOLDS

Bone repair occurs through well described steps in healthy patients. Some systemic conditions such as types 1 and 2 diabetes mellitus (DM), largely affect bone healing (Krpata and Novitsky, 2016) and are associated with impaired or delayed healing process, poor vascularization, and higher risk of infections (Feliciano, 2014).

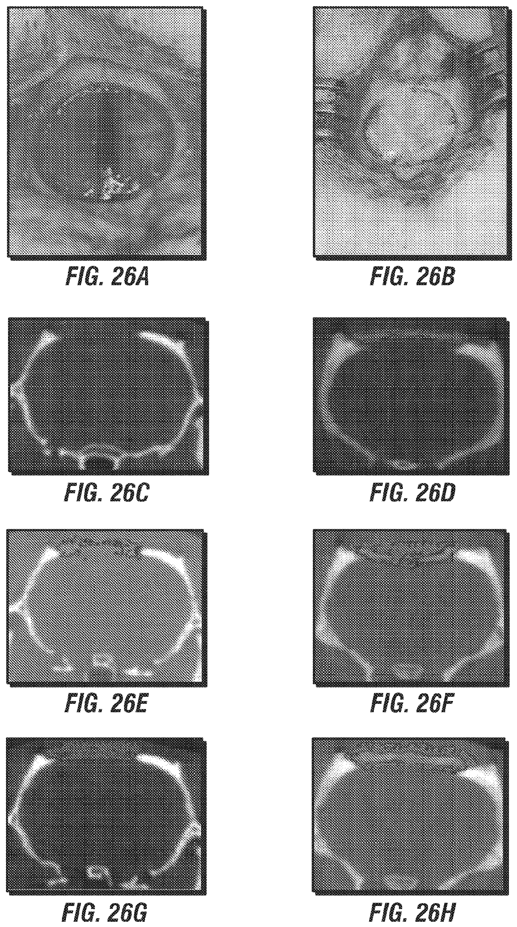

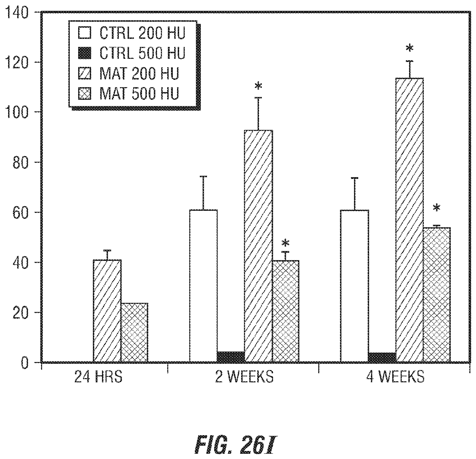

In 2012, 29.1 million Americans (9.3% of the population) were diagnosed as diabetic (Hall *et al.,* 2010). Unfortunately, for these patients, simple fractures can have disastrous outcomes. The effects of diabetes have been studied in relation to oral and maxillofacial surgery for many years (Mirmehdi and Ramshaw, 2016; Poulose *et al.,* 2012). The current reconstruction of bony craniomaxillofacial (CMF) defects involves autologous transplant with vascularized flaps (Bilsel and Abci, 2012). These procedures require extended hospitalization and a secondary donor site with associated morbidity and complications (Sadava *et al.,* 2014). Moreover, these procedures are not viable options for patients with vascular impairment. Tissue engineering (TE) and regenerative medicine strive to develop alternatives to current surgical procedures by utilizing cells, scaffolds, and growth factors to regenerate skeletal defects (Rouwkema *et al.,* 2008). Leveraging these results, the regenerative potential of these 3 layers integrated in a single implant for the repair of a critical size calvarial defect was investigated (**FIG. 26A****-****FIG. 26I**). The PMM was fabricated as a monolithic membrane maintaining its spatial multilayered organization. The defect (8 mm in diameter and 2 mm deep) was created on the midline sagittal suture of calvaria, forming a critical size defect unable to spontaneously heal. The calvarial defect was covered by PMM shaped and sized to fit the defect. Bone formation was evaluated at at 24 hrs, 2, and 4 weeks by dynaCT, and evaluated tissue regeneration using histological analysis 4 weeks after surgery on decalcified specimens (**FIG. 27A, FIG. 27B, and FIG. 27C**).

### EXAMPLE 6 - BIOMIMETIC COLLAGEN/ELASTIN MESHES FOR VENTRAL HERNIA REPAIR IN A RAT MODEL

Ventral hernia repair remains a major clinical need. Herein, a type I collagen/elastin crosslinked blend (CollE) was fabricated into a biomimetic mesh for ventral hernia repair. To evaluate the effect of architecture on the performance of the implants, CollE was formulated both as flat sheets (CollE sheets) and porous scaffolds (CollE scaffolds). The morphology, hydrophylicity and *in vitro* degradation were assessed by SEM, water contact angle and differential scanning calorimetry, respectively. The stiffness of the meshes was determined using a constant stretch rate uniaxial tensile test, and compared to that of native tissue. CollE sheets and scaffolds were tested in vitro with human bone marrow-derived mesenchymal stem cells (h-BM-MSC), and finally implanted in a rat ventral hernia model. Neovascularization and tissue regeneration within the implants was evaluated at 6 weeks, by histology, immunofluorescence, and q-PCR. It was found that CollE Sheets and Scaffolds were not only biomechanically sturdy enough to provide immediate repair of the hernia defect, but also promoted tissue restoration in only 6 weeks. In fact, the presence of elastin enhanced the neovascularization in both sheets and scaffolds. Overall, CollE scaffolds displayed mechanical properties more closely resembling those of native tissue, and induced higher gene expression of the entire marker genes tested, associated with *de novo* matrix deposition, angiogenesis, adipogenesis and skeletal muscles, compared to CollE sheets. Altogether, these data suggest that the improved mechanical properties and bioactivity of CollE sheets and scaffolds make them valuable candidates for applications of ventral hernia repair.

A ventral hernia is a bulge through an opening in the abdominal muscles (Krpata and Novitsky, 2016). Ventral hernias are currently repaired through the surgical implantation of a multiplicity of grafts used to patch the defect in the abdominal wall (Feliciano, 2014). Grafts for ventral hernia repair remain a major clinical need in the United States, with over 350,000 cases annually, for an approximately $1 Billion market (Hall *et al.,* 2010; Mirmehdi and Ramshaw, 2016). As 2-20% of hernias become chronic, these surgeries are one of the most expensive of the United States (Poulose *et al.,* 2012). Currently, two categories of meshes are used in clinical practice: permanent and resorbable. Since their introduction, non-resorbable synthetic meshes became the standard of care for abdominal wall repair (*e.g*., Marlex®, Goretex®, Prolene®) (Bilsel and Abci, 2012). However, their poor biodegradability is associated with several complications, including chronic inflammation, infection, adhesion, extrusion, chronic pain and recurrence (Mirmehdi and Ramshaw, 2016; Sadava *et al.,* 2014; Rouwkema *et al.,* 2008; Bellows *et al.,* 2011), making surgeons reluctant to implant non-resorbable prostheses in complex hernias. More recently, the use of decellularized matrices (*e.g.,* AlloDerm®, Permacol®, Veritas®) represented an effort towards the development of biomimetic grafts, characterized by higher biocompatibility and improved *in vivo* performances (Bilsel and Abci, 2012; Gefen, 2009). To some extent, biologically-derived grafts demonstrated advantageous, by triggering a lower inflammatory response and fewer infections, than synthetic meshes (Sandvall *et al.,* 2016; Huerta *et al.,* 2016). Recent evidence showed that their major drawback remains the low bioactivity, which causes poor vascularization and uneven cellularization of the implant itself, ultimately resulting in a lack of integration to the surrounding tissues and tissue regeneration (Bellows *et al.,* 2013; Liu *et al.,* 2011). Thus, these prosthetics have proved useful in proving immediate repair of abdominal wall defects, but are hindered by poor long-term mechanical strength, due to their uncontrolled biological activity and biodegradation mechanisms (Smart *et al.,* 2012).

Following implantation, a graft undergoes one of the following fates: (i) neovascularization and new ECM deposition; (ii) fibrotic encapsulation; (iii) resorption/degradation (Badylak and Gilbert, 2008). Thus, the ideal graft should be conducive for early neo-vascularization, fully biocompatible and biomimetic to promote a regenerative immunological response, rather than inflammation that could cause fibrotic encapsulation (Novitsky, 2013; Taraballi *et al.,* 2016). Furthermore, it should allow for the recruitment of stem cells and adult tissue-specific cells to ultimately recover tissue function (Gurtner *et al.,* 2008). Towards this end, biologically inspired approaches to the design of biomimetic meshes for abdominal wall reconstruction are currently been investigated (Badylak *et al.,* 2016; Ayala *et al.,* 2015).

Based on this evidence, this clinical need was addressed using the overarching principles of biomimicry to create materials that better resemble the composition, architecture, and mechanical properties of the target tissue. Collagen and elastin are the most abundant components of the extracellular matrix (ECM) of almost every tissue in the body, which provides essential cues for cell attachment, migration and organization (Minardi *et al.,* 2016). Collagen is essential in maintaining tissue architecture, while elastin provides resilience and deformability to tissues. The properties of elastin are critical to specific tissue functions (*e.g.,* dermis, vessels, and muscles) (Quaglino *et al.,* 2009). Furthermore, several evidences suggest a role of soluble elastin in favoring tissue regeneration by promoting angiogenesis (Daamen *et al.,* 2008; Waterhouse *et al.,* 2011). Collagen and elastin are also the two main components of the ECM of the abdominal wall (Franz, 2006), and changes in their ratio or metabolism have been associated with hernia occurrence (Bellon *et al.,* 1997; Franz, 2008). It is established that the pathological changes in the collagen of the abdominal wall set the stage for the development of hernias (Bendavid, 2004). Thus, a type I collagen/elastin crosslinked blend (CollE) has been developed for the fabrication of meshes with enhanced mechanical properties and bioactivity, to improve hernia repair. The porosity of biomaterials also plays a crucial role in their interaction with cells and newly formed tissues (Zhang *et al.,* 2015). For this reason, the CollE was molded in two different architectures: as flat sheets (CollE Sheets) and porous scaffolds (CollE Scaffolds), to evaluate the effect of porosity on the performance of CollE, in a rat ventral hernia model. Collagen type I sheets and scaffolds were used as controls (Coll sheet and Coll scaffold).

### MATERIALS AND METHODS

***Fabrication of Collagen and Collagen*/*Elastin Sheets and Scaffolds.*** For the fabrication of the collagen sheets (Coll Sheets) and the porous collagen scaffolds (Coll Scaffolds) 1 g of type I collagen (Nitta Casings, Inc.) was dissolved in an acetate buffer (pH 3.5) to reach the desired concentration (20 mg/mL). The collagen suspension was precipitated by the addition of sodium hydroxide (0.1 M) solution at pH 5.5. The collagen was washed three times with DI water. The resulting collagen slurry was cross-linked through incubation in a 1,4-butanediol diglycidyl ether (BDDGE) (Sigma-Aldrich) aqueous solution (2.5 mM) for 48 hrs, setting up the BDDGE/collagen ratio to 1 wt%, as previously optimized (Minardi *et al.,* 2014). The cross-linked collagen was washed 3 times in DI water. To fabricate Coll Sheet, the slurry was molded in metal racks, at a thickness of 5 mm, and air dried under a fume hood for 5 days (final thickness: 0.3 mm). On the contrary, to fabricate the Coll Scaffolds, the slurry was molded into metal racks, at a thickness of 5-mm, and freeze dried. For the fabrication of the collagen/elastin sheets (CollE Sheets) and scaffolds (CollE Scaffolds) the same procedure to prepare the collagen slurry was followed. Then, the elastin was added (10 wt%) to the collagen slurry, and blended. The resulting collagen/elastin slurry was cross-linked as previously described. After the washings, the collagen/elastin slurry was cast as previously described, to fabricated either the CollE sheets or CollE scaffolds.

***Scanning Electron Microscopy.*** The morphology of sheets and scaffolds was evaluated by scanning electron microscopy (SEM) (Quanta 600 FEG, FEI Company, Hillsboro, OR, USA). Freeze dried samples were sputter coated with 10 nm of Pt/Pd and imaged at a voltage of 10 kV. Also, scaffolds seeded with h-BM-MSC were images after 1 and 3 weeks in culture. Rat ventral fascia was also imaged. Prior to SEM imaging samples were dehydrated according to an established protocol for the preparation of fresh tissues for SEM imaging, as reported elsewhere (Minardi *et al.,* 2015).

***Contact Angle.*** The contact angle measurements were conducted using a Ramé-Hart 200-F1 goniometer. The contact angles were measured by dropping a 2 µL water drop onto the surface of Coll Sheets, Coll Scaffolds, CollE Sheets and CollE Scaffolds, using a micro syringe (Gilmont). At least six measurements were performed on each surface (n=5). The errors in contact angle were ±1°.

***Pore Size, Porosity and Swelling.*** The volumes of the scaffolds (cylinders of 0.5 × 0.1 cm) were (Vs) were calculated from their geometry. The mean size of the pores was measured from the SEM images of the scaffolds with the software ImageJ (NIH). 20 measurements were acquired per image. The overall porosity of the scaffolds was calculated as described elsewhere, through an ethanol infiltration method [31]. Values are expressed as means ± standard deviation (n = 3).

***Swelling and* In Vitro *Degradation.*** The swelling properties (PBS uptake) of the scaffolds were determined. Briefly, scaffolds were weighted after lyophilization (dry), and after incubation in PBS at 37°C, at different time points. The uptake ratio was defined as % of swelling. Values are expressed as means ± standard deviation (n = 3).

The *in vitro* degradation of sheets and scaffolds was evaluated. Sheets (squares of 0.55 × 0.55 × 0.03 cm) and scaffolds (cylinders of 0.55 × 0.1 cm) were allowed to hydrate in PBS at 37°C for 1 hr prior to start the experiment, and then weighted. Following complete re-hydration sheets and scaffolds were placed in 0.5 mL of 0, 0.05, 0.1 and 0.2 mg/mL of Collagenase Type I (≥125 units per mg dry weight) in PBS (CLS 1, Worthington Biochemical Corporation) at 37°C, under mild agitation. At selected time points, the sheets and scaffolds were collected and weighted. Values are expressed as means ± SD (n = 5).

***Differential Scanning Calorimetry.*** Differential scanning calorimetry (DSC) was performed though a TGA/DSC analyzer (METTLER TOLEDO), by placing the samples in alumina pans, and undergoing a heating ramp from 25°C to 500°C, at 10°C/min.

***Mechanical Testing.*** The modulus (specifically the stiffness dσ/dε or tangent modulus measured at the terminal, linear portion of a stress strain curve), strength, and engineering strain at max stress of the materials was assessed using a constant stretch rate uniaxial tensile test performed in wet conditions at 37°C and compared to that of native tissue. The stiffness of the materials was assessed using a constant stretch rate uniaxial tensile test performed in wet conditions at 37°C and compared to that of native tissue. Five samples each of Coll and CollE Sheets and Scaffolds, as well as 7 abdominal walls taken from male Sprague Dawley rats (obtained from the tissue share program at Texas A&M) and 7 abdominal walls taken from Lewis rats (Obtained from Houston Methodist Research Hospital) were tested. Samples were prepared with approximately 0.5 inch widths and gage lengths of precisely 1 inch (approximately 0.5 inches on each side of the gage length were clamped). The samples were hydrated in PBS at room temperature for at least 30 min, and then loaded into a dual column load frame (Test Resources, R Controller) with an environmental chamber filled with PBS at 37°C. The temperature was allowed to equilibrate for 10 min prior to preconditioning. Samples were preconditioned at 1 Hz for 50 cycles with an amplitude of 5% stretch and a mean of 5% stretch, then the samples were loaded at 5% per minute stretch rate until failure. Loads were normalized by the initial cross-sectional area of the samples (the scaffolds were lightly compressed to measure their thickness) giving the first component of the first Piola-Kirchhoff stress, and the stretch ratio was determined by dividing the current length by the initial gage length. The stiffness was determined by fitting a line to the linear portion of the stress stretch plot. Finally, the "modulus" was determined by fitting a line to the linear portion of the stress stretch plot, and the maximum stress (herein called "strength"), as well as the max elongation, were determined.

***3D* In Vitro *Culture of h-BM-MSC.*** All of the h-BM-MSC used in this study were bone marrow-derived from two women (age 28 and 35 respectively), and were isolated at the Texas A&M Institute for Regenerative Medicine (Temple, TX, USA). Cells were seeded at a density of 10⁴ cells/cm² and incubated at 37°C in a humidified atmosphere (90%) with 5% CO₂ and 5% O₂. The number of viable cells was counted by the trypan blue dye exclusion method using a Burker chamber. The media utilized was composed of α-MEM, 10% FBS, 2% glutamine, 1% β-FGF and 1% streptomycin/amphotericin B (Gibco). Cells were serially passaged using TrypLE Express (Invitrogen) when a confluency of 80% was reached. After 4 passages, h-BM-MSC were seeded on Coll and CollE Sheets of 0.5 × 0.5 cm (80000 cells) and Coll and CollE Scaffolds of 0.8 cm in diameter, 1 mm thick, (150000 cells). Media change was performed every three days. At 1 week and 3 week time points, the viability of the h-BM-MSC was assessed by LIVE/DEAD™ assays (ThermoFisher Scientific) according to the manufacturer's instructions, and images captured through an ECLIPSE Ti-E inverted fluorescence microscope (Nikon). Images were analyzed through the NIS Elements software (Nikon). Cell proliferation on the sheet or scaffolds was evaluated by Alamar Blue assay (Invitrogen), according to manufacturer's protocol, up to 3 weeks. Absorbance was measured at a wavelength of 570 and 600 nm. Gene expression analysis was performed as described below.

***Surgical Procedure.*** Twenty-four male Lewis rats of average weight of 360g (Charles River Labs, Houston, TX) were divided in four groups: Coll Sheet, CollE Sheet, Coll Scaffold and CollE scaffold (n=6 each). The study was performed in conformity with the guidelines established by American Association for Laboratory Animal Science. All procedures were approved by the Houston Methodist Institutional Animal Care and Use Committee (IACUC) (Protocol: AUP-0115-0002). After acclimation, under the effects of anesthesia (2.5-3.0% Isoflurane/O₂ gas mixture through a non-rebreather mask) and in supine position, all animals were shaved and the operative site will was prepped with a 2% chlorhexidine acetate solution; they all had chronic ventral hernias created by incising the skin, subcutaneous tissues, and full thickness of the abdominal wall at the linea alba for a length of 3 cm, and the overlying skin closed with surgical clips. The defect was allowed to mature for at least 28 days before the secondary surgery was performed for ventral hernia repair. Reparative ventral hernia repair was performed identically regardless of mesh material. After carefully excising the hernia sac using sharp dissection to define hernia borders, a mesh (3 × 3 cm) was fit in the resultant defect with approximately 5 mm of circumferential overlap. The mesh was placed intraperitoneally and secured to the abdominal wall across the defect in a bridging fashion using eight interrupted 5-0 Prolene® (Ethicon, Somerville, NJ, USA) sutures. The overlying skin was closed with skin staples, which were removed 14 days postoperatively. To minimize the contamination of the surgery site, aseptic technique and dapping was used during surgery. All meshes were sterilized using an ethylene oxide chamber (Andersen). After 6 weeks, all rats were euthanized by inhalation of carbon dioxide at 70%.

***Histology.*** Following euthanasia, the site of implantation was harvested and embedded in 10% formalin buffer, for 48 hrs, and then embedded in paraffin, according to established standard protocols (n=3) (Rouwkema *et al.,* 2008). 10-µm-thick sections were deparaffinized twice in fresh xylene for 8-10 min and rehydrated sequentially with decreasing ethanol concentrations (100%, 95%, 90%, 80%, and 70%) and distilled water (8-10 min for each step). Tissue slices were stained using a kit for Masson's trichrome staining (Abcam; ab150686), according to manufacturer's protocol. For H/E and elastic staining, slides were thawed, hydrated, washed and stained with hematoxylin and eosin according to manufacturer's protocol (Sigma-Aldrich®). Stained slices were mounted with Cytoseal XYL (Thermo Scientific) mounting medium and then imaged with an ECLIPSE Ci-E histological microscope (Nikon). Images were processed and analyzed through the NIS Elements software (Nikon).

***Immunohistochemistry.*** The tissue slides were stained for alpha-smooth muscle Actin (a-SMA) (Abcam; ab5694), CD31 (LSBio, Seattle, WA) and DAPI (Sigma-Aldrich) according to standard protocols and manufacturer's protocols. Samples were imaged with a confocal laser microscope (A1 Nikon Confocal Microscope) and the images were analyzed through the NIS-Elements software (Nikon).

**In Vivo *Gene Expression Analysis.*** Part of the specimens harvested from the rats at 6 weeks were also designated to qPCR analysis (n=3). The tissue specimens were embedded in 1 mL of Trizol reagent (Life Technologies), homogenized and the RNA extracted according to the manufacturer's protocol. RNA concentration and purity were measured using a NanoDrop ND1000 spectrophotometer (NanoDrop Technologies). The cDNA was synthesized from 800 ng total RNA, using the iScript retrotranscription kit (Bio-Rad Laboratories). Transcribed products were analyzed using Taqman fast advanced master mix (ThermoFisher Scientific) and the appropriate target probes (ThermoFisher Scientific) on a StepOne Plus real-time PCR system (Applied Biosystems).

***Statistical Analysis.*** Statistical analysis was performed with the software GraphPad Prism, using a one-way ANOVA, and a post-hoc unpaired Student's t-test. All experiments were performed at least in triplicate. Data is presented as mean ± standard deviation. A value of p < 0.05 was considered statistically significant: *p < 0.05; **p < 0.01; ***p < 0.001; ****p < 0.0001.

### RESULTS

***Characterization of Sheets and Scaffolds.*** Coll Sheets, Coll Scaffolds, CollE Sheets and CollE Scaffolds were fully characterized. In FIG. 28A-FIG. 28L the images of CollE Sheet (dry and re-hydrated) and the CollE Scaffold (dry and rehydrated) are reported. The micro-architecture and fine morphology of the meshes were evaluated through SEM imaging. Coll and CollE Sheet presented a similar architecture, however it was observed that, compared to Coll Sheet, CollE Sheet displayed thicker fibers and a rougher surface. Coll and CollE Scaffolds appeared both highly porous, and their pore size was measured by ImageJ (Coll Scaffold 31±7.0; CollE Scaffold 35±4.8). Inside the pores of the Coll Scaffold, its walls looked smooth and delimited by collagen fibers. On the contrary, the walls of the pores of CollE Scaffold presented protrusions of the material, creating a more complex architecture. Their porosity was found to be 80% (±2.0) for Coll Scaffold and 79 % (±0.6) CollE Scaffold.

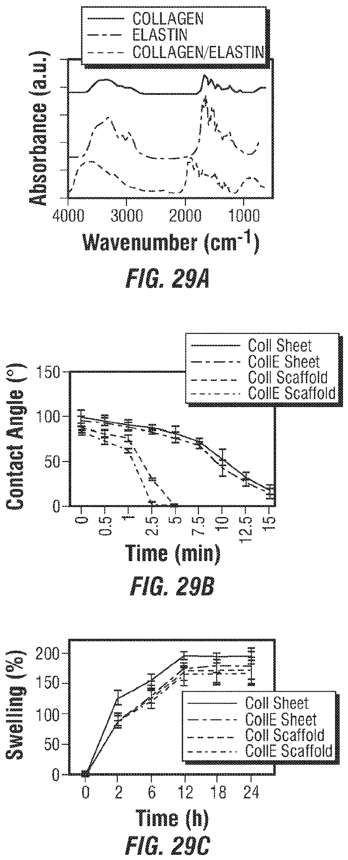

The water contact angle (WCA) of the four groups of meshes was measured to determine and compare their surface hydrophilicity (FIG. 29A-FIG. 29C). Initially, both WCA of CollE Sheets (97°± 3.4°) and CollE Scaffolds (85°± 5.0°) were found slightly lower than those of Coll Sheet (99°±8.8°) and Scaffold (88°± 5.0°). WCA of Coll and CollE Sheets remained higher for a longer time, respect to that of Coll and CollE Scaffolds. The water drop was fully adsorbed at 2.5 and 5 min for the CollE and Coll Scaffold, respectively. At 15 min, the drop on the CollE Sheets was still not fully adsorbed. The meshes were then embedded in PBS until complete swelling. The meshes were all fully re-hydrated within 2 hrs, but if let swell for 12 hrs, their volume augmented of approximately 1.5 times.

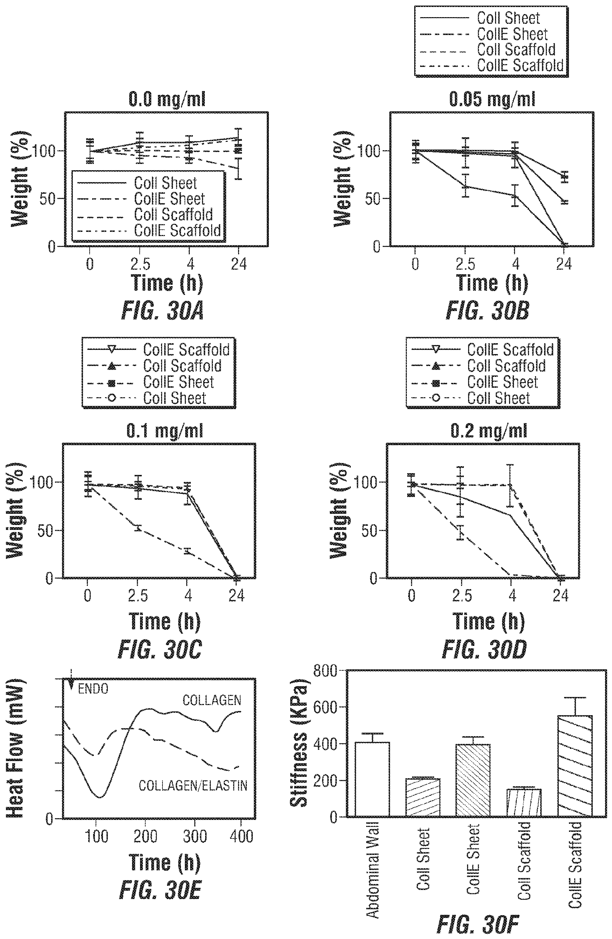

The enzymatic degradation (FIG. 30A-FIG. 30F) of the Coll and CollE Sheets and Coll and CollE Scaffolds was investigated *in vitro,* in physiological-like conditions (PBS, 37°C, under mild agitation), at increasing concentrations of Collagenase I. At a concentration of 0.0 mg/mL of Collagenase I there was no significant change in weight loss. Over 24 hrs, all of the meshes swelled, except Coll Scaffold, which faced an approximately 18% weight loss. At 0.05 mg/mL of Collagenase I, all of the meshes faced weight loss within 24 hrs, but only Coll Scaffold and CollE Scaffold were fully degraded (Coll Sheet 73 %±4.5; CollE Sheet 46% ±0.3; Coll Scaffold 1% ±0.2; CollE Scaffold 0% ±0.0).

At 0.1 mg/mL of Collagenase I all the meshes faced a faster weight loss within 24 hrs, and all of them were completely degraded within 24 hrs. Finally, at 0.2 mg/mL of Collagenase I all of the meshes faced weight loss within 24 hrs. Coll Scaffold presented the faster degradation rate at all of the concentrations of Collagenase 1. On the contrary, Coll and CollE Sheets presented the slowest degradation rate, *in vitro.*

To assess the interaction between collagen and elastin, the thermal degradation of the collagen/elastin blend was performed by Differential Scanning calorimetry (DSC). The Tm of Type I collagen was found at 105°C (± 0.4), while that of the collagen/elastin blend was lower, at 93°C (± 0.1). A second endothermic reaction was recorded at 345°C (± 0.5) for type I collagen alone, and at 353°C (± 0.2).

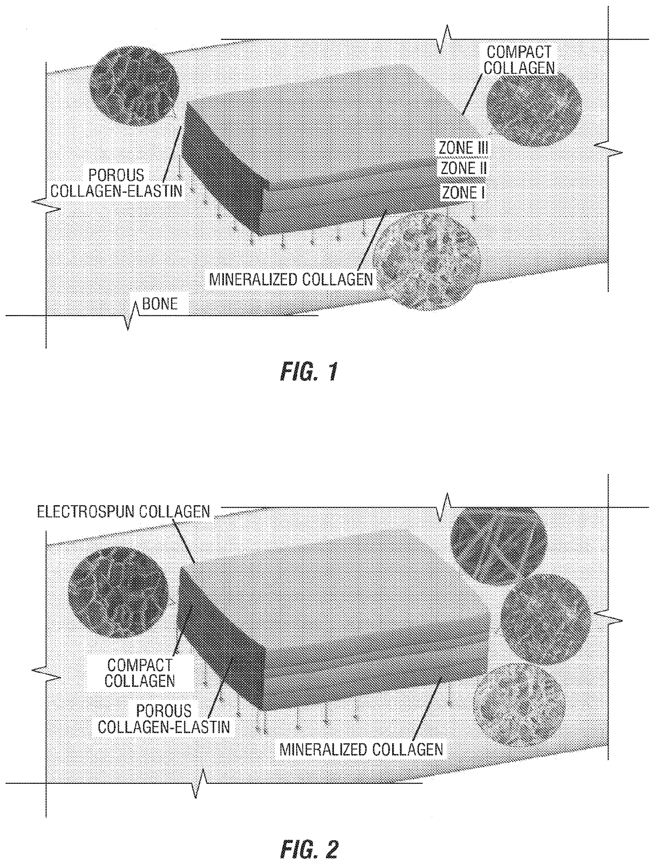

Finally, the mechanical properties of the meshes were determined by uniaxial tensile testing and are reported in Fig. 2C. The Coll and CollE Sheets had relatively high stiffnesses moduli (3.55 ± 1.01 MPa and 2.91±.59 MPa respectively) compared to the native abdominal wall tissue (392 ± 102; 429±103 kPa). The Coll and CollE Scaffolds (690 ± 188 kPa and 535 ± 159 kPa respectively) had values more similar to those of native tissue but were still somewhat stiffer, with the CollE Scaffold being the closest match with the native tissue at only about 36% stiffer. All meshes had statistically different values of stiffness (p < 0.0001 for Coll Sheet and CollE Sheet; p < 0.01 for Coll Scaffold), compared to the rat abdominal wall, but CollE Scaffold. The strength (max engineering stress) of the meshes showed that the Coll and CollE sheets were much stronger (1784±431 kPa and 959±165 kPa respectively) than the Coll and CollE meshes (196±91 kPa and 128±42 kPa respectively) and native tissue (211±51 kPa). The elongation at maximum stress of the meshes showed that the Coll and CollE sheets stretched more (54±5% and 38±4% respectively) than the Coll and CollE meshes (30±4% and 33±3% respectively) but much less than the native tissue (82±4%).

***Meshes* in vitro *testing with h-BM-MSC.*** The meshes were tested with h-BM-MSC and 3D cultures were established *in vitro.* At 1 and 3 weeks the organization and viability of the cells was evaluated by LIVE/DEAD™ assays (FIG. 31A-FIG. 31R). Live cells were stained in green, while dead cells were stained in red. Over 1 week, h-BM-MSC already appeared homogenously distributed on the surface of the Coll and CollE Sheets. The samples were also imaged by SEM microscopy, to further evaluate cell morphology. On the Coll and CollE Sheets, h-BM-MSC organized as a mono-layer. On the 3D scaffolds, the cells were found homogenously distributed also in the depth of the pores of the scaffolds. Here, the cells aligned along the walls of the pores. Similarly, at 3 weeks, the cells appeared at a higher confluency, but still mostly alive, as confirmed by imaging. At SEM evaluation, the cells appeared more packed, either when grown on the Coll and CollE Sheets or on the porous scaffolds. In particular, cells appeared aligned on the Coll and CollE Sheets, while the pores of Coll and CollE Scaffolds were hardly visible at 3 weeks. These SEM images were also compared to those of rat ventral fascia tissue that presented the same packed and dense organization.

The viability of the cells was quantified at 1 and 3 weeks, from the LIVE/DEAD™ assays: (i) Coll Sheet, 74% (±8.5) at 1 week and 99% (±2.8) at 3 weeks; (ii) CollE Sheet, 79% (±5.6) at 1 week and 99% (±3.8) at 3 weeks; (iii) Coll Scaffold, 90% (±7.0) at 1 week and 97% (±4.0) at 3 weeks; (iv) CollE Scaffold, 84% (±7.6) at 1 week and 99% (±2.0) at 3 weeks.

The organization and morphology of cells cultured on the meshes were also investigated and compared to that of cells culture in 2D. Cell growth was monitored over 3 weeks by Alamar Blue assay. After 1 weeks, cells in 2D culture (2D CTRL) reached the confluency, and started detaching. On the contrary, cells seeded on all of the Sheets and Scaffolds, maintained approximately the same number of cells, which only slightly increased over time, but significantly differed from the growth of 2D CTRL (p < 0.05; **p<0.01, ****p<0.0001).

Finally, it was assessed the ability of the meshes to maintain the stemness of h-BM-MSC up to 3 weeks by gene expression analysis. All data were normalized to that of cells cultured in 2D. No significant difference (p < 0.05) in the expression of any of the marker genes of choice was found, either at 1 week or at 3 weeks. The values of gene expression for the genes CD44, CD90, CD73 and CD105 were also determined.

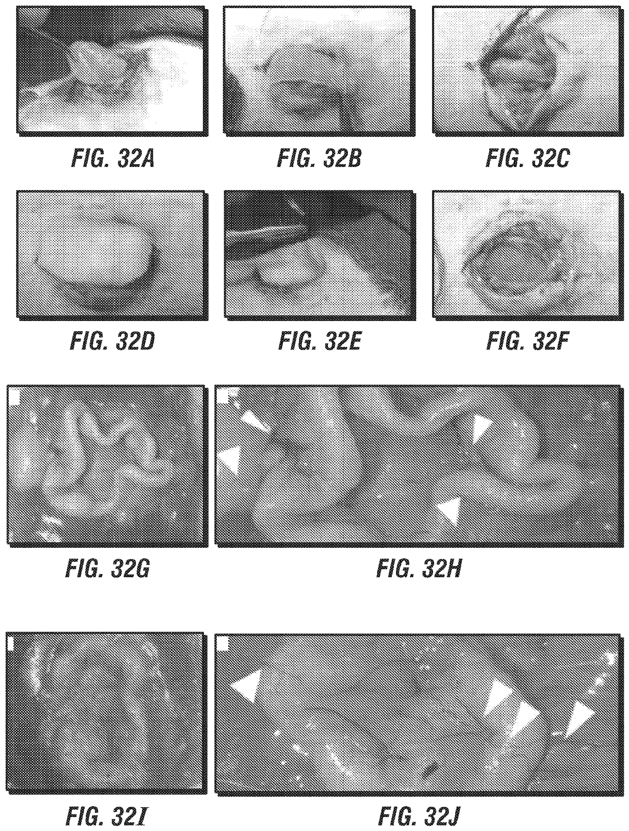

***Ventral Hernia Repair in Rat.*** CollE Sheet and CollE Scaffold were ultimately tested in a rat ventral hernia repair model (FIG. 32A-FIG. 32J). Images of the implantation of the meshes were obtained, and Coll Sheet and Coll Scaffold were also tested, as controls.

A few animals developed postoperative seroma - 1 Coll Scaffold, 1 CollE Scaffold, and 3 CollE-elastin blend - but all were mild in nature and resolved without intervention or infectious complication. At 6 weeks, all animals receiving Coll Sheet meshes (n=6) suffered gross recurrence of their hernias, secondary to mechanical failure of the meshes. This was evidenced by bulging viscera on exam and confirmed at necropsy. Breakage of the meshes occurred invariably at its center, where there existed no overlap of implant with host abdominal wall musculature. Two animals receiving Coll Scaffold suffered recurrence (33% recurrence), also at the mesh center. This was manifested clinically first by eventration at the repair site followed by gross hernia recurrence at 4 weeks postoperatively. Significantly, no animals receiving CollE Sheets (p < 0.0001) or Scaffolds (p < 0.05) suffered recurrence.

CollE Sheets appeared fully integrated with the surrounding tissue (FIG. 25G) and highly vascularized, at higher magnification (FIG. 32H). CollE Scaffold was also found integrated and highly vascularized throughout (FIG. 32I), as indicated by the white arrows in the magnification in FIG. 32J.

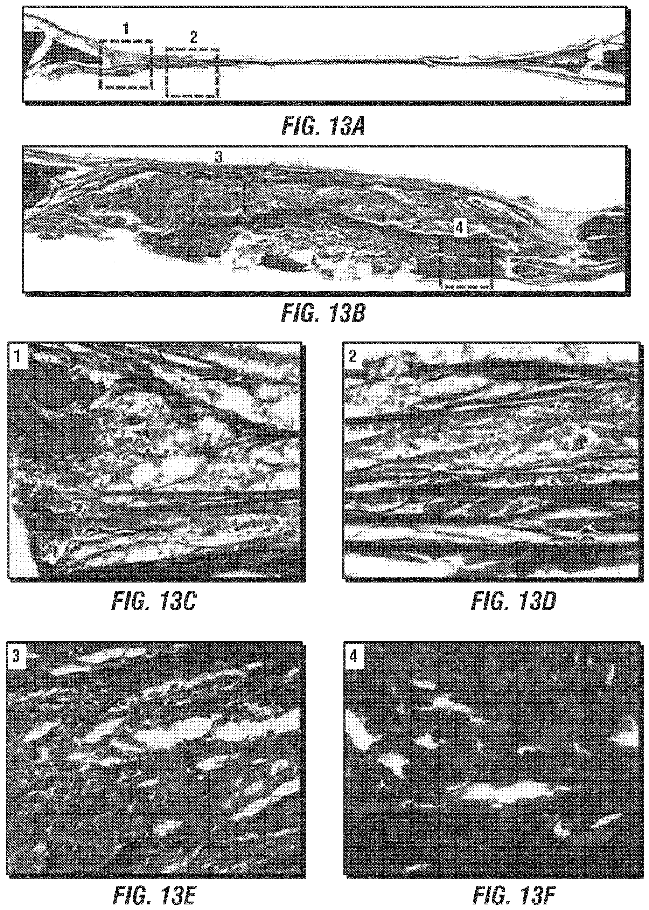

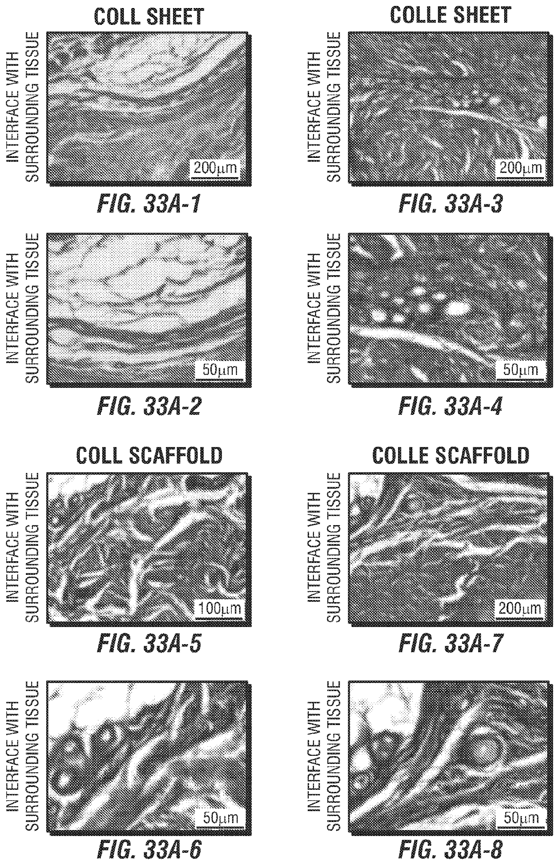

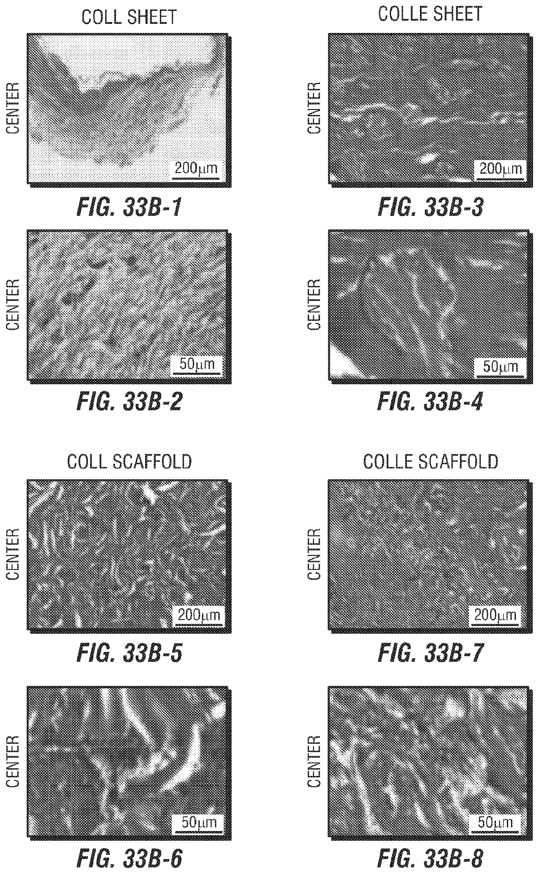

After sacrificing the animals, Masson's trichrome staining was used to evaluate tissue remodeling within the grafts, after 6 weeks post-implantation (FIG. 33A-1 to FIG. 33B-8). Collagen type I stained in blue, muscles in bright red and cell cytoplasm in pink. Both the graft-tissue interface and center of the implants were imaged for all groups. Poor cell infiltration was observed in what was left of the Coll Sheet, and minimal evidence of vascularization was found, at 6 weeks. On the contrary CollE Sheet was found highly vascularized at the tissue-graft interface, as well as Coll Scaffold and CollE Scaffold, which presented the vessels with bigger diameter. No sign of fibrotic encapsulation of the implants was found. Regarding the center of the implants, very poor cell infiltration was found in the Coll Sheet, while in CollE Sheet several newly formed muscle fascia were found, as well as in CollE Scaffolds, in even higher amount. Some extent of muscle tissue was also found in the Coll Scaffold group, as well as infiltrating cells. The overall area occupied by the newly formed muscles was: (i) CollE Sheet, 4% (±1.14); (ii) CollE Scaffold, 9% (±2.6).

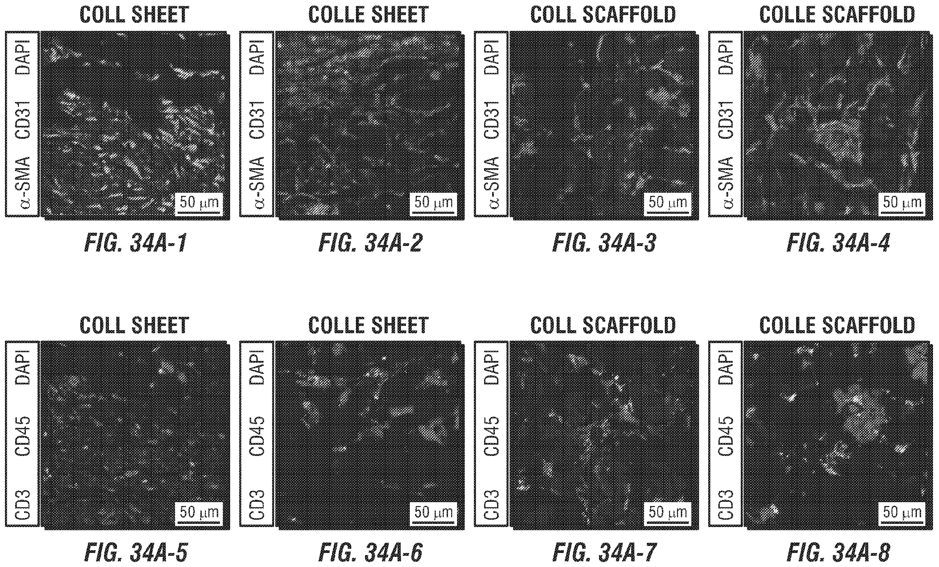

Finally, to assess the formation of functional vessels, immunofluorescence was used to verify the expression of key markers for new-vessel formation (a-SMA, CD31), lymphocytes and leukocytes (CD3, CD45), and basal membrane proteins (Laminin, Collagen IV) (FIG. 34A-1 to FIG. 34D). It was found that all markers were significantly more expressed in CollE Sheet and CollE Scaffold, compared to their respective controls, expect for Collagen IV that was expressed at the same extent in both Coll and CollE Scaffold.

Moreover, tissue regeneration was also evaluated by quantifying the expression of tissue-specific marker genes (Col1a1, Vim, S100a4 for de novo matrix deposition; Vegfa, Vwf, Col3a1 for vascular tissue; Adipoq, Pparg, Lpl for adipose tissue; and Acta1, Actn3, Myh1 for muscle tissue).

Through radar charts, the relative expression of each gene of each tissue-specific markers (ECM, vessels, fat, muscle) is displayed; the size of the shadow area represents the normalized overall expression of all three markers belonging to a specific set of tissue-associated genes (FIG. 35A and FIG. 35B). CollE Scaffold showed the highest level of expression for all of the four sets of tissue-specific marker genes, while Coll Sheet was the group that displayed the least level of expression for all genes tested. CollE Sheet and Coll Scaffold displayed comparable expression of the gene associated with the de novo deposition of ECM. However, while CollE Sheet induced a higher expression of Col3α1, Coll Scaffold induced the over-expression of Vwf and Vegfa. It was also found that CollE Sheet induced a higher expression of the adipogenesis-associated genes, compared to Coll Scaffold. Finally, the expression of muscle-associated marker genes was comparable in CollE Sheet and Coll Scaffold, but Coll Scaffold appeared to preferably induce the expression of Acta1 and Myh1, over Actn3.

The interactive functional network among the marker genes selected for this study (highlighted and divided in clusters), and their associated genes, according to their co-expression, co-localization, genetic interaction and physical interaction, pathways, and shared protein domains were determined and described herein.

### DISCUSSION

It's been found that the number of ventral hernias recurrence drops from 43% to 4%, when repaired with a mesh (Feliciano, 2014). For these reasons, there is a strong clinical need for bioactive meshes able to prevent recurrence, while favoring tissue repair. The present example describes the development of biomimetic meshes to improve ventral hernia repair. The collagen-based meshes investigated in this work were functionalized with soluble elastin, for its known role in promoting vascularization and tissue repair (Daamen *et al.,* 2008; Waterhouse *et al.,* 2011). During material fabrication a pH driven self-assembly of type I collagen was followed, while elastin was added, to favor the blending of the two proteins, before being crosslinked.

Elastin is an important component of virtually all connective tissues, and one of the most abundant proteins of the ECM of elastic tissues such as arterial vessels, skin, and muscle (Sandberg, *et al.,* 1981; Uitto, 1979). In skin and dermis, elastin represents approximately the 10% dry weight of the whole tissue (Uitto, 1979). There, its role consists of maintaining tissue elasticity and resilience (He *et al.,* 2013). Currently, the only commercially available biomimetic meshes used in clinical practice for hernia repair consist of decellularized dermal matrix (Smart *et al.,* 2012; Melman *et al.,* 2011; Deeken *et al.,* 2011). Therefore, by introducing a 10 wt% elastin in the collagenous meshes (CollE Sheets, CollE Scaffolds) an attempt was made to to mimic the conditions found in native dermis. Thus, the working hypothesis was that biologically inspired meshes functionalized with elastin could provide immediate repair of a ventral wall defect in rat, and enhance neovascularization, resulting in improved tissue regeneration. The ultimate goal was to develop new, off-the-shelf, bioactive solutions for hernia repair.

Towards this end, firstly, the key features for their final in vivo application of CollE Sheets and Scaffolds (structure, wettability, degradation, composition and mechanical properties) were assessed. Their morphology and micro-structure were evaluated by SEM. Although not macroscopically different, at the microscale CollE Sheets and CollE Scaffolds appeared significantly dissimilar from their Coll counterparts. Elastin seemed to make the surface of the CollE Sheets and of the pores of CollE Scaffolds rougher. It is known that elastin naturally organizes in smaller fibrils than collagen, which results in increased roughness (Pepe *et al.,* 2007).

The *in vivo* cellularization and vascularization of biomaterials are enhanced by high porosity, resulting in enhanced de novo tissue formation (Zhang *et al.,* 2015; Annabi *et al.,* 2014). Thus, to assess if the use of porous over flat meshes (like the commercially available ones) could further favor tissue repair, CollE Scaffolds were also included in this study. Compared to decellularized tissues, the controlled synthesis and freeze-drying process employed permitted control of the porosity and pore size of the final grafts, which represented a significant advantage.

To date, several methods to control pores and the overall porosity of scaffolds have been proposed (*e.g.,* porogen leaching, foaming, freeze-drying) (Zhang, 2014; Kim *et al.,* 2008; White *et al.,* 2012). Although both porogen leaching and foaming are easy techniques to leave replica pores and control the porosity of scaffolds, they lack the ability to create interconnected and oriented porosity, which is crucial for cells infiltration and neo-vascularization (Zhang, 2014). As a result, the pores unavailable to cells and vessels were found to result as defects, impairing tissue regeneration (Lee *et al.,* 2011; Zhang *et al.,* 2016). Crosslinking the porogens before addition to the scaffolding material revealed only partially advantageous, requiring the use of organic solvents which may result toxic to cells. Towards this end, a freeze-drying method was optimized to precisely control the pore structure and porosity of our CollE Scaffold, by simply directing the nucleation of the ice crystals in the collagen/elastin water-based slurry. The slow freezing ramp allowed for the formation of larger crystals (resulting in larger interconnected pores), oriented on the minor axis of the scaffolds, perpendicularly to the freezing shelf of the lyophilizer. The formation of oriented interconnected pores was paramount to support the throughout cellularization and neovascularization of the porous meshes.

The porous scaffolds resulted more hydrophilic than the respective Sheets. This was hypothesized to impact the *in vivo* cellularization of the meshes, however, the PBS uptake was comparable for all meshes, and all meshes swelled between 170 % and 200%, over 24 hrs. The in vitro degradation study of the meshes showed that Coll Scaffold was the mesh more prone to degradation. On the contrary, Coll and CollE Sheets were the most resistant to enzymatic degradation, probably due to the smaller surface area. The thermal degradation showed that the Tm of type I collagen was higher than that of the CollE blend, and thus that more energy was necessary to denature it. By comparison with the DSC profile of soluble elastin (Vasconcelos *et al.,* 2012), this is possibly due to the interaction between type I collagen and elastin, that modified the highly hierarchically organized structure of type I collagen (Hu *et al.,* 2012), further demonstrating collagen blending with elastin.

Also, it has been demonstrated that a controlled and interconnected porosity, with pore size of 30 to 40 µm is needed to enable the exchange of metabolic components and to facilitate endothelial cell entrance and new-vessel formation (Madden *et al.,* 2010; Loh and Choong, 2013; Artel *et al.,* 2011). The synthesis was optimized to obtain a mean pore size of 30-35 µm, which appeared to have supported vascularization of the scaffolds throughout as expected.

Mechanically, there are two ways of looking at the stiffness performance of these grafts. The first, used commonly in the literature, is to determine a planar stiffness, which neglects differences in material thickness (Amoroso, 2012). The second, which was chosen, is to compute the full three dimensional stiffness (Mason *et al.,* 2013). This stiffness accurately describes the fundamental mechanical performance of the material itself, and is not affected by geometry of the graft (*i.e.,* a thicker graft will bear a larger load, but the stiffness would not change). This mechanical analysis showed that, at the stretch rate that was tested, the stiffness of the Coll and CollE Sheets were an order of magnitude higher than that of the native tissue. These higher stiffnesses cause stiffness/compliance mismatch at the interface between the graft and the tissue. This phenomenon is recognized as problematic in tissues that have substantial mechanical functions like vasculature (Fung, 2013) and muscle (Bilston and Tan, 2015), as it leads to non-physiological stress patterns. In this case the mismatch may result in regions at the interface that have much higher stress than the surrounding tissue and graft, and are consequently more likely to fail. Conversely, the Coll and CollE Scaffolds more closely matched the stiffness of the native tissue, with the CollE Scaffold matching slightly better (though not statistically significant), reducing stiffness mismatch in either material. Overall, this analysis is promising for the performance of CollE Scaffolds, demonstrating how the porous structure of the scaffolds, closely mimics the mechanical properties of the target tissue.

From a mechanical perspective, there are two ways of looking at the performance of these grafts. The first, which is not uncommon in the hernia repair literature, is to determine planar mechanical properties that neglect differences in material thickness (Deeken *et al.,* 2011; Cordero *et al.,* 2016). The second, a more standard approach found in general materials testing, is to compute the full three dimensional mechanical properties. Using a 3D analysis, we described the mechanical performance of the material itself in a way not affected by geometry of the graft (*i.e.,* a thicker graft will of course bear a larger load, but the stiffness or 'modulus' would not change). This simple mechanical analysis demonstrated that, at the stretch rate that was tested, the moduli of the Coll and CollE Sheets were an order of magnitude higher than that of the native tissue. These higher moduli may cause stiffness/compliance mismatch at the interface between the graft and the tissue. This phenomenon is recognized as problematic in tissues that have substantial mechanical functions like vasculature (Fung, 2013), and muscle (Bilston and Tan, 2015), as it leads to non-physiological stress patterns in tissues. In this application the mismatch may result in regions at the interface that have much higher stress than the surrounding tissue and graft, and are consequently more likely to fail. Note that these materials are highly viscoelastic and very sensitive to the stretch rate, and the mechanical performance of these constructs may vary significantly at different stretch rates. The sensitivity of each material was not quantified and thus, the effect of change in stretch rate may impact each material differently. The Scaffolds (Coll and CollE) more closely matched the modulus of the native tissue than the Sheets (Coll and CollE). The trends observed suggest that the CollE Scaffold provided the best match, reducing stiffness mismatch between the material and native tissue. Though the CollE Scaffolds did not exhibit the same strength as the native tissue in the mechanical tests, but since the material resulted successful in the hernia model, it is believed that the strength is sufficient for the application. This evidence suggests they possess sufficient strength for this application despite to matching the native tissue. One feature of the mechanical behavior of native abdominal wall, that none of the synthetic materials matched, is the elongation at maximum load, where the native tissue elongated 30% -50% more than the meshes. Furthermore, although the stiffness measures have been reported as "moduli" in order to maintain consistency with existing literature in the field, the term modulus can be misleading. By definition, the values of modulus herein reported are not a Young's modulus, as the linear region we measured does not occur at small strains. Young's Modulus is defined to describe the "stiffness" of linearly elastic homogeneous isotropic materials subjected to very small deformations. This is not consistent with the behavior of soft tissue, or collagenous materials, as they are non-linear viscoelastic heterogeneous materials typically subjected to large deformations (Humphrey and Delange, 2004). Thus, the modulus reported here is more appropriate. For this reason, it has been recognized that a full mechanical characterization of soft tissues (even in a one-dimensional manner, while neglecting viscoelasticity) requires more information than just a single 'modulus.' Amensag and McFetridge determined some empirical measures that more fully describe the behavior of these tissues (Amensag and McFetridge, 2014). More recently, Freed and Rajagopal created a mathematical model for collagen and elastin containing tissues that use similar parameters (Freed and Rajagopal, 2016). Specifically, these models measure an initial stiffness (modulus) that is comparable to a Young's Modulus, a terminal stiffness that is comparable to the measure we use herein, and a dimensionless parameter that describes the inverse of the strain at which we move from the initial stiffness to the terminal stiffness. Altogether, these findings are very promising for the performance of the CollE Scaffolds, and a more detailed analysis is to be performed in future studies.

The meshes were also tested in vitro with h-BM-MSC, and all of the above mentioned peculiar features of CollE Sheets and Scaffold appeared to have a relevant role in cell organization in 3D. The presence of elastin (in both CollE Sheet and Scaffold) seemed to support the organization and alignment of h-BM-MSC over 3 weeks, while cells appeared less organized on Coll Sheet and Scaffolds. It appears that there is a correlation between the presence of elastin in tissues and their level of organization. For instance, both tendons and arteries, in which elastin is the main component of their ECM, are longitudinally organized, along their main axis [58, 59]. Elastin is also well known to be essential in the morphogenesis of arteries (Li *et al.,* 1998).

In addition, both Coll and CollE-based meshes appeared to ensure the preservation of h-BM-MSC stemness (as assessed by qPCR), as well as their viability and growth. This seemed to suggest that functionalizing meshes with elastin would enable cells to migrate, proliferate and reorganize within the grafts that are the main initial steps of tissue repair.

Considering the promising results obtained in vitro, the CollE-based meshes (Sheets and Scaffolds) were utilized in the repair of a ventral hernia in rat. An early time point of 6 weeks was chosen as the terminal time point. Coll Sheets and Scaffolds were also implanted as controls, because similar to the biological grafts currently used in clinical practice (*i.e.,* Strattice™ or Alloderm®) (Fortelny 2016). Anecdotally, CollE Sheets and CollE Scaffolds were found more capable of handling the stresses of suture fixation with more adequate tactile feedback, and were easily personalized with intraoperative trimming, compared to their Coll counterparts. More importantly, they were not only biomechanically sturdy enough to provide immediate repair of the hernia defect, but also more bioactive than the biological meshes currently used in the clinical practice, promoting tissue restoration in only 6 weeks. In fact, at necropsy, it was noted that the appearance of the CollE-based meshes presented more vascularization than what reported for commercially available meshes such as Strattice™ (Life Cell) (Cavallo *et al.,* 2015), or Permacol® (Life Cell) (Abdelfatah *et al.,* 2015), which are commonly used for human surgical applications.

It is established that neovascularization plays a pivotal role in biomaterial-mediated tissue regeneration (Auger *et al.,* 2013). The inability to provide a sufficient blood supply during the initial phase of tissue repair after biological meshes implantation is one of the main limitations in tissue engineering (Rouwkema *et al.,* 2008). The insufficient vascularization of an implant can cause limited cell infiltration and eventually even cell death (Rouwkema *et al.,* 2008).

Thus, it was hypothesized that the higher performance of CollE Sheets and Scaffolds in vivo was associated with their extensive vascularization throughout, which connected the whole implants to the main blood stream. This was probably conducive to the formation of new muscle tissue, as shown through histology. The newly formed vessels were proven to be fully organized vessels, by immunofluorescence, by staining endothelial and smooth muscle cells, and two of the main component of the tunica (collagen IV and laminin). In addition, lymphocytes, leukocytes and red blood cells were found in correspondence of the lumen of the vessels.

The gene expression analysis further corroborated such hypothesis: it was found that all of the selected marker genes specific for ECM, vessels, adipose tissue, and muscle tissue were significantly overexpressed in CollE-based meshes, compared to Coll-based meshes. Furthermore, it was observed that Scaffolds appeared to favor the deposition of more tissue compared to Sheet. These data correlated with the mechanical testing and histological evaluation, and further proved that the architecture is crucial to allow the proper cell infiltration and new tissue formation within the graft; but also that its composition is fundamental, as the presence of elastin promoted the formation of a significantly higher amount of vessels, which supported a faster and more efficient mesh remodeling and functional tissue formation. In particular, elastin preferably induced the expression of Col3a1, Adipoq, and Actn3. The selectively upregulation of Adipoq may result especially advantageous to hernia repair: recent evidence proved that besides its function in metabolism, Adipoq is also involved in the pathway for hematopoiesis, vasculogenesis, as well as muscle regeneration, were adiponectin acts similarly to a stem factor (Chiarugi and Fiaschi, 2010).

Altogether this data showed that by adding elastin to collagen-based meshes for hernia repair; it was possible to enhance their early vascularization, for improved hernia repair, avoiding recurrence. In addition, designing macroporous CollE Scaffolds, it was possible to more closely match the architecture and mechanical properties of native abdominal wall, further enhancing new vessel ingrowth and tissue regeneration. Considering the advantages of both meshes (ease of surgical handling of the CollE Sheets, and the improved bioactivity of CollE Scaffolds), multilayered meshes may be engineered that combine all of their individual features, providing for an improved mesh for ventral hernia repair.

### SUMMARY

This example provides data validating the performance of CollE-based meshes (CollE Sheets and Scaffolds) in an orthotopic model of rat bioprosthetics hernia repair. At 6 weeks, both CollE Sheets and Scaffolds were highly vascularized and integrated within the surrounding tissues. No recurrence was observed, compared to their Coll-based counterparts. Altogether these data demonstrate that CollE Sheets and Scaffolds hold promise for a future clinical application. In fact, they not only showed promising mechanical performance, but also allowed for an efficient neovascularization, enhancing cell infiltration and resulting in new adipose and muscle tissue formation within the implant, in only 6 weeks. In addition, the disclosed meshes allowed for the use of the same surgical procedure utilized in clinical practice, with the commercially-available grafts. This study represents a significant step in the design of bioactive acellular off-the-shelf biomimetic meshes for ventral hernia repair.

### REFERENCES

The following references, to the extent that they provide exemplary procedural or other details supplementary to those set forth herein, are specifically incorporated herein by reference:
ABDELFATAH, M et al., "Long-term outcomes (> 5-year follow-up) with porcine acellular dermal matrix (Permacol™) in incisional hernias at risk for infection," Hernia, 19:135-140 (2015).
ALTSCHUL, SF et al., "Gapped BLAST and PSI-BLAST: a new generation of protein database search programs," Nucl. Acids Res., 25(17):3389-3402 (1997).
AMENSAG, S and MCFETRIDGE, PS, "Tuning scaffold mechanics by laminating native extracellular matrix membranes and effects on early cellular remodeling," J. Biomed. Mat. Res. Part A, 102:1325-1333 (2014).
AMOROSO, NJ et al., "Microstructural manipulation of electrospun scaffolds for specific bending stiffness for heart valve tissue engineering," Acta Biomaterialia, 8:4268-4277 (2012).
ANNABI, N et al., "25th anniversary article: rational design and applications of hydrogels in regenerative medicine," Adv. Mat., 26:85-124 (2014).
ARTEL, A et al., "An agent-based model for the investigation of neovascularization within porous scaffolds," Tissue Eng. Part A, 17:2133-2141 (2011).
AUGER, FA et al., "The pivotal role of vascularization in tissue engineering," Annu. Rev. Biomed. Eng., 15:177-200 (2013).
AYALA, P et al., "Engineered composite fascia for stem cell therapy in tissue repair applications," Acta Biomaterialia, 26:1-12 (2015).
BADYLAK, SF and GILBERT, TW, "Immune response to biologic scaffold materials," Semin. Immunol., Elsevier; pp. 109-116 (2008).
BADYLAK, SF et al., "Marrow-derived cells populate scaffolds composed of xenogeneic extracellular matrix," Exp. Hematol., 29(11):1310-1318 (Nov. 2001).
BADYLAK, SF et al., "Mechanisms by which acellular biologic scaffolds promote functional skeletal muscle restoration," Biomaterials, (2016).
BEATTIE, AJ et al., "Chemoattraction of progenitor cells by remodeling extracellular matrix scaffolds," Tissue Eng. Part A, 15(5):1119-1125 (May 2009).
BELLON, J et al., "Study of biochemical substrate and role of metalloproteinases in fascia transversalis from hernial processes," Eur. J. Clin. Invest., 27:510-516 (1997).
BELLOWS, CF et al., "Repair of incisional hernias with biological prosthesis: a systematic review of current evidence, Am. J. Surg., 205:85-101 (2013).
BELLOWS, CF et al., "The effect of bacterial infection on the biomechanical properties of biological mesh in a rat model," PLoS One, 6:e21228 (2011).
BENDAVID, R, "The unified theory of hernia formation," Hernia, 8:171-176 (2004).
BILSEL, Y and ABCI, I, "The search for ideal hernia repair; mesh materials and types," Intl. J. Surg., 10:317-321 (2012).
BILSTON, LE and TAN, K "Measurement of passive skeletal muscle mechanical properties in vivo: recent progress, clinical applications, and remaining challenges," Ann. Biomed. Eng., 43:261-73 (2015).
CAVALLO, J et al., "Remodeling characteristics and biomechanical properties of a crosslinked versus a non-crosslinked porcine dermis scaffolds in a porcine model of ventral hernia repair," Hernia, 19:207-218 (2015).
CHIARUGI, P and FIASCHI, T, "Adiponectin in health and diseases: from metabolic syndrome to tissue regeneration," Exp. Opin. Therapeut. Targets, 14:193-206 (2010).
CORDERO, A et al., "Biaxial mechanical evaluation of absorbable and nonabsorbable synthetic surgical meshes used for hernia repair: physiological loads modify anisotropy response," Ann. Biomed. Eng., 44:2181-2188 (2016).
DAAMEN, W et al., "Preparation and evaluation of molecularly-defined collagen-elastin-glycosaminoglycan scaffolds for tissue engineering, Biomaterials, 24:4001-4009 (2003).
DAAMEN, WF et al., "A biomaterial composed of collagen and solubilized elastin enhances angiogenesis and elastic fiber formation without calcification," Tissue Eng. Part A, 14:349-360 (2008).
DEEKEN, CR et al., "Histologic and biomechanical evaluation of crosslinked and non-crosslinked biologic meshes in a porcine model of ventral incisional hernia repair," J. Amer. Coll. Surg., 212:880-888 (2011).
DEVILLE, S et al., "Freeze casting of hydroxyapatite scaffolds for bone tissue engineering," Biomaterials, 27(32):5480-5489 (Nov. 2006).
EYRE-BROOK, AL "The periosteum: its function reassessed," Clin. Orthop. Relat. Res., 189:300-307 (Oct. 1984).
FELICIANO, D, "Ventral hernia repair," Amer. Coll. Surg. (2014).
FILARDO, G et al., "New bio-ceramization processes applied to vegetable hierarchical structures for bone regeneration: an experimental model in sheep. Tissue Engineering Part A, 20(3-4):763-73 (2013).
FORTELNY, RH et al., "Open and laparo-endoscopic repair of incarcerated abdominal wall hernias by the use of biological and biosynthetic meshes," Frontiers Surg., 2016:3 (2016).
FRANZ, MG, "The biology of hernia formation," Surg. Clin. N. Amer., 88:1-15 (2008).
FRANZ, MG, "The biology of hernias and the abdominal wall," Hernia, 10:462-471 (2006).
FREED, AD and RAJAGOPAL, K, "A promising approach for modeling biological fibers," Acta Mechanica, 2016:1-11 (2016).
FREYTES, DO et al., "Uniaxial and biaxial properties of terminally sterilized porcine urinary bladder matrix scaffolds," J. Biomed. Mater. Res. B Appl. Biomater., 84(2):408-414 (Feb. 2008).
FUNG, Y-C, "Biomechanics: mechanical properties of living tissues," Springer Science & Business Media (2013).
GEFEN, A, "Bioengineering research of chronic wounds: a multidisciplinary study approach," Springer Science & Business Media (2009).
GILBERT, TW et al., "Degradation and remodeling of small intestinal submucosa in canine Achilles tendon repair," J. Bone Joint Surg. Am., 89(3):621-630 (Mar. 2007).
GRIBSKOV, M, and BURGESS, RR, "Sigma factors from E. coli, B. subtilis, phage SP01, and phage T4 are homologous proteins," Nucleic Acids Res., 14(16):6745-6763 (Aug. 1986).
GUGALA, Z et al., (Eds.) New Approaches in the Treatment of Critical-Size Segmental Defects in Long Bones. Macromolecular Symposia; Wiley Online Library (2007).
GURTNER, GC et al., "Wound repair and regeneration," Nature, 453:314-321 (2008).
HALE, WG, and MARGHAM, JP, "HARPER COLLINS DICTIONARY OF BIOLOGY," HarperPerennial, New York (1991).
HALL, MJ et al., "National hospital discharge survey: 2007 summary," Natl Health Stat Report, 29:1-20 (2010).
HARDMAN, JG, and LIMBIRD, LE, (Eds.), "GOODMAN AND GILMAN'S THE PHARMACOLOGICAL BASIS OF THERAPEUTICS" 10th Edition, McGraw-Hill, New York (2001).
HE, B et al., "Elastin fibers display a versatile microfibril network in articular cartilage depending on the mechanical microenvironments," J. Orthopaedic Res., 31:1345-1353 (2013).
HILL, MA et al., "Small artery elastin distribution and architecture - focus on three dimensional organization," Microcirculation (2016).
HODDE, J et al., "Effects of sterilization on an extracellular matrix scaffold: part II. Bioactivity and matrix interaction," J. Mater. Sci. Mater. Med., 18(4):545-550 (Apr. 2007).
HOFFMAN, MD and BENOIT, DS, "Emerging ideas: engineering the periosteum: revitalizing allografts by mimicking autograft healing," Clin. Orthopaed. Rel. Res., 471(3):721-726 (2013).
HU, X et al., "Protein-based composite materials," Mat. Today, 15:208-215 (2012).
HUERTA, S et al., "Biological mesh implants for abdominal hernia repair: US Food and Drug Administration approval process and systematic review of its efficacy," JAMA Surg., 151:374-381 (2016).
HUMPHREY, JD and DELANGE, SL, "An introduction to biomechanics. Solids and Fluids, Analysis and Design Springer, Heidelberg (2004).
KANG, Y et al., "Engineering vascularized bone grafts by integrating a biomimetic periosteum and β-TCP scaffold," ACS Appl. Mat. Interface, 6(12):9622-9633 (2014).
KIM, TG et al., "Macroporous and nanofibrous hyaluronic acid/collagen hybrid scaffold fabricated by concurrent electrospinning and deposition/leaching of salt particles," Acta Biomaterialia, 4:1611-1619 (2008).
KING, KF "Periosteal pedicle grafting in dogs," J. Bone Joint Surg. Br., 58(1):117-121 (Feb. 1976).
KON, E et al., "A novel nano-composite multi-layered biomaterial for treatment of osteochondral lesions: technique note and an early stability pilot clinical trial," Injury, 41(7):693-701 (Jul. 2010).
KON, E et al., "Novel nano-composite multi-layered biomaterial for the treatment of multifocal degenerative cartilage lesions," Knee Surg. Sports Traumatol. Arthroscop., 17(11):1312-1315 (Nov. 2009).
KON, E et al., "Novel nano-composite multilayered biomaterial for osteochondral regeneration a pilot clinical trial," Am. J. Sports Med., 39(6):1180-1190 (Jun. 2011).
KON, E et al., "Novel nanostructured scaffold for osteochondral regeneration: pilot study in horses," J. Tissue Eng. Regen. Med., 4(4):300-308 (Jun. 2010).
KON, E et al., "Orderly osteochondral regeneration in a sheep model using a novel nano-composite multilayered biomaterial," J. Orlhop. Res., 28(1):116-124 (Jan. 2010).
KON, E et al., "Platelet autologous growth factors decrease the osteochondral regeneration capability of a collagen-hydroxyapatite scaffold in a sheep model," BMC Musculoskelet. Disord., 11:220 (Sept. 2010).
KOSTOPOULOS, L and KARRING, T, "Role of periosteum in the formation of jaw bone: An experiment in the rat," J. Clin. Periodontal., 22(3):247-254 (Mar. 1995).
KRPATA, DM, and NOVITSKY, YW, "Laparoscopic ventral hernia repair," Hernia Surgery: Springer; p. 223-230 (2016).
KYTE, J, and DOOLITTLE, RF, "A simple method for displaying the hydropathic character of a protein," J. Mol. Biol, 157(1):105-132 (1982).
LEE, G-S et al., "Direct deposited porous scaffolds of calcium phosphate cement with alginate for drug delivery and bone tissue engineering," Acta Biomaterialia, 7:3178-3186 (2011).
LI, DY et al., "Elastin is an essential determinant of arterial morphogenesis, Nature, 393:276-280 (1998).
LIU, Z et al., "Comparison of two porcine-derived materials for repairing abdominal wall defects in rats," PLoS One, 6:e20520 (2011).
LOH, QL and CHOONG, C, "Three-dimensional scaffolds for tissue engineering applications: role of porosity and pore size," Tissue Eng. Part B: Rev., 19:485-502 (2013).
LONG, T et al., "The effect of mesenchymal stem cell sheets on structural allograft healing of critical sized femoral defects in mice," Biomaterials, 35(9):2752-2759 (2014).
MADDEN, LR et al., "Proangiogenic scaffolds as functional templates for cardiac tissue engineering," Proc. Natl. Acad. Sci. USA, 107:15211-15216 (2010).
MARCACCI, M et al., "Stem cells associated with macroporous bioceramics for long bone repair: 6- to 7-year outcome of a pilot clinical study," Tissue Eng., 13(5):947-955 (May 2007).
MASON, BN et al., "Tuning three-dimensional collagen matrix stiffness independently of collagen concentration modulates endothelial cell behavior," Acta Biomaterialia, 9:4635-4644 (2013).
MELMAN, L et al., "Early biocompatibility of crosslinked and non-crosslinked biologic meshes in a porcine model of ventral hernia repair," Hernia, 15:157-164 (2011).
MINARDI, S et al., "Biomimetic concealing of PLGA microspheres in a 3D scaffold to prevent macrophage uptake," Small, (2016).
MINARDI, S et al., "Evaluation of the osteoinductive potential of a bio-inspired scaffold mimicking the osteogenic niche for bone augmentation," Biomaterials, 62:128-137 (Sept. 2015).
MINARDI, S et al., "Multiscale patterning of a biomimetic scaffold integrated with composite microspheres," Small, 10(19):3943-3953 (Oct. 2014).
MIRMEHDI, I and RAMSHAW, B, "Synthetic mesh: making educated choices," Hernia Surgery, Springer, p. 53-60 (2016).
MURPHY, MB et al., "Engineering a better way to heal broken bones," Chem. Eng. Prog., 106(11):37-43 (2010).
MURPHY, MB et al., "Multi-composite bioactive osteogenic sponges featuring mesenchymal stem cells, platelet-rich plasma, nanoporous silicon enclosures, and peptide amphiphiles for rapid bone regeneration," J. Funct. Biomat., 2(2):39-66 (2011).
MURPHY, MB et al., "Adult and umbilical cord blood-derived platelet-rich plasma for mesenchymal stem cell proliferation, chemotaxis, and cryo-preservation," Biomaterials, 33(21):5308-5316 (2012).
NEEDLEMAN, SB and WUNSCH, CD, "A general method applicable to the search for similarities in the amino acid sequence of two proteins," J. Mol. Biol., 48(3):443-453 (1970).
NIEPONICE, A et al., "Reinforcement of esophageal anastomoses with an extracellular matrix scaffold in a canine model," Ann. Thorac. Surg., 82(6):2050-2058 (Dec. 2006).
NOAH, EM et al., "Impact of sterilization on the porous design and cell behavior in collagen sponges prepared for tissue engineering," Biomaterials, 23(14):2855-2861 (Jul. 2002).
NOVITSKY, YW, "Biology of biological meshes used in hernia repair," Surg. Clin. N. Amer., 93:1211-1215 (2013).
O'BRIEN, FJ, "Biomaterials & scaffolds for tissue engineering," Mar Today, 14(3):88-95 (2011).
OLDE DAMINK, LH et al., "Influence of ethylene oxide gas treatment on the in vitro degradation behavior of dermal sheep collagen,"J. Biomed. Mater. Res., 29(2):149-155 (Feb. 1995).
PEPE, A et al., "Supramolecular organization of elastin and elastin-related nanostructured biopolymers," Nanomedicine, 2:203-218 (2007).
POULOSE, B et al., "Epidemiology and cost of ventral hernia repair: making the case for hernia research," Hernia, 16:179-183 (2012).
QUAGLINO, D et al., "Elastin and elastin-based polymers. Nano and Biocomposites, Taylor & Francis 2009:249-274 (2009).
REING, JE et al., "Degradation products of extracellular matrix affect cell migration and proliferation," Tissue Eng. Part A, 15(3):605-614 (Mar. 2009).
ROUWKEMA, J et al., "Vascularization in tissue engineering," Trends Biotechnol., 26:434-441 (2008).
ROVERI, N et al., "Biologically inspired growth of hydroxyapatite nanocrystals inside self-assembled collagen fibers," Mat. Sci. Eng. C, 23(3):441-446 (Mar. 2003).
RUSSO, L et al., "Carbonate hydroxyapatite functionalization: a comparative study towards (bio)molecules fixation," Interface Focus, 4(1):20130040 (Feb. 2014).
SADAVA, EE et al., "Wound healing process and mediators: Implications for modulations for hernia repair and mesh integration," J. Biomed. Mat. Res. Part A, 102:295-302 (2014).
SANDBERG, LB et al., "Elastin structure, biosynthesis, and relation to disease states," New Engl. J. Med., 304:566-579 (1981).
SANDVALL, BK et al., "Comparison of synthetic and biologic mesh in ventral hernia repair using components separation technique," Ann. Plastic Surg., 76:674-679 (2016).
SCHRODINGER 2013. Schrodinger, LLC: New York, NY (2013).
SINGLETON, P and SAINSBURY, D, "DICTIONARY OF MICROBIOLOGY AND MOLECULAR BIOLOGY," 2nd Ed., John Wiley and Sons, New York (1987).
SMART, et al., "Biological meshes: a review of their use in abdominal wall hernia repairs," The Surgeon, 10:159-171 (2012).
TAMPIERI, A et al., "Biologically inspired synthesis of bone-like composite: self-assembled collagen fibers/hydroxyapatite nanocrystals," J. Biomed. Mater. Res. A, 67(2):618-625 (Nov. 2003).
TAMPIERI, A et al., "Design of graded biomimetic osteochondral composite scaffolds," Biomaterials, 29(26):3539-3546 (Sept. 2008).
TAMPIERI, A et al., "From biomimetic apatites to biologically inspired composites," Anal. Bioanal. Chem., 381(3):568-576 (Feb. 2005).
TAMPIERI, A et al., "Mimicking natural bio-mineralization processes: a new tool for osteochondral scaffold development," Trends Biotechnol., 29(10):526-535 (Oct. 2011).
TARABALLI, F et al., "Amino and carboxyl plasma functionalization of collagen films for tissue engineering applications," J. Colloid Interface Sci., 394:590-597 (2013).
TARABALLI, F et al., "Biomimetic collagenous scaffold to tune inflammation by targeting macrophages," J. Tissue Eng., 7:2041731415624667 (2016).
TATE, MK et al., "Surgical membranes as directional delivery devices to generate tissue: testing in an ovine critical sized defect model," PloS One, 6(12):e28702 (2011).
THITISET, T et al., "Development of collagen/demineralized bone powder scaffolds and periosteum-derived cells for bone tissue engineering application," Int. J. Mol. Sci., 14(1):2056-2071 (Jan. 2013).
UENO, T et al., "Small intestinal submucosa (SIS) in the repair of a cecal wound in unprepared bowel in rats," J. Gastrointest. Surg., 11(7):918-922 (Jul. 2007).
UITTO, J, "Biochemistry of the elastic fibers in normal connective tissues and its alterations in diseases," J. Invest. Dermatol., 72:1-10 (1979).
VALENTIN, JE et al., "Extracellular matrix bioscaffolds for orthopaedic applications. A comparative histologic study," J. Bone Joint Surg. Am., 88(12):2673-2686 (Dec. 2006).
VASCONCELOS, A et al., "Novel silk fibroin/elastin wound dressings," Acta Biomaterialia, 8:3049-3060 (2012).
WAGENSEIL, JE and MECHAM, RP, "Elastin in large artery stiffness and hypertension," J. Cardiovasc. Translat. Res., 5:264-273 (2012).
WATERHOUSE, A et al., "Elastin as a nonthrombogenic biomaterial," Tissue Eng. Part B Rev., 17:93-99 (2011).
WHITE, LJ et al., "The effect of processing variables on morphological and mechanical properties of supercritical CO2 foamed scaffolds for tissue engineering," Acta Biomaterialia, 8:61-71 (2012).
ZANTOP, T et al., "Extracellular matrix scaffolds are repopulated by bone marrow-derived cells in a mouse model of achilles tendon reconstruction," J. Orthop. Res., 24(6):1299-1309 (Jun. 2006).
ZHANG, C et al., "A study on a tissue-engineered bone using rhBMP-2 induced periosteal cells with a porous nano-hydroxyapatite/collagen/poly (L-lactic acid) scaffold," Biomed. Mat., 1(2):56 (2006).
ZHANG, J et al., "A simple and effective approach to prepare injectable macroporous calcium phosphate cement for bone repair: Syringe-foaming using a viscous hydrophilic polymeric solution," Acta Biomaterialia, 31:326-338 (2016).
ZHANG, Q et al., "Pore size effect of collagen scaffolds on cartilage regeneration, Acta Biomaterialia, 10:2005-2013 (2014).
ZHANG, X et al., "A perspective: engineering periosteum for structural bone graft healing," Clin. Orthopaed. Rel. Res., 466(8):1777-1787 (2008).
ZHANG, YN et al., "A highly elastic and rapidly crosslinkable elastin-like polypeptide-based hydrogel for biomedical applications," Adv. Funct. Mat., 25:4814-4826 (2015).

It should be understood that the examples and embodiments described herein are for illustrative purposes only and that various modifications or changes in light thereof will be suggested to persons skilled in the art and are to be included within the spirit and purview of this application and the scope of the appended claims.

All references, including publications, patent applications and patents, cited herein are hereby incorporated by reference to the same extent as if each reference was individually and specifically indicated to be incorporated by reference and was set forth in its entirety herein.

Recitation of ranges of values herein are merely intended to serve as a shorthand method of referring individually to each separate value falling within the range, unless otherwise indicated herein, and each separate value is incorporated into the specification as if it were individually recited herein.

The description herein of any aspect or embodiment of the invention using terms such as "comprising," "having," "including," or "containing," with reference to an element or elements is intended to provide support for a similar aspect or embodiment of the invention that "consists of," "consists essentially of," or "substantially comprises," that particular element or elements, unless otherwise stated or clearly contradicted by context (*e.g.,* a composition described herein as comprising a particular element should be understood as also describing a composition that contains and/or that includes that particular element, unless otherwise explicated stated, or clearly contradicted by context).

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the compositions and methods and in the steps or in the sequence of steps of the method described herein without departing from the concept, spirit and scope of the invention. More specifically, it will be apparent that certain agents that are chemically- and/or physiologically-related may be substituted for the agents described herein while the same or similar results would be achieved. All such similar substitutes and modifications apparent to those skilled in the art are deemed to be within the spirit, scope, and concept of the invention as defined by the appended claims.

The present invention will now be described by way of reference to the following clauses:
1. A multilayer collagen membrane comprising a first upper layer that comprises compact collagen, a second middle layer that comprises collagen and elastin, and a third lower layer that comprises mineralized collagen.
2. The multilayer collagen membrane of clause 1, wherein the first upper layer comprises Type I collagen.
3. The multilayer collagen membrane of clause 1 or clause 2, wherein the Type I collagen is obtained from bovine tendon.
4. The multilayer collagen membrane of any preceding clause, wherein the first upper layer is non-porous.
5. The multilayer collagen membrane of any preceding clause, wherein the second middle layer comprises bovine elastin.
6. The multilayer collagen membrane of any preceding clause, wherein the second middle layer permits vascularization and recapitulates one or more elastic features of periosteum.
7. The multilayer collagen membrane of any preceding clause, in which the third lower layer comprises hydroxyapatite.
8. The multilayer collagen membrane of any preceding clause, wherein the third lower layer comprises magnesium-doped hydroxyapatite.
9. The multilayer collagen membrane of any preceding clause, wherein one or more layers further comprise nanoparticles.
10. The multilayer collagen membrane of any preceding clause, wherein the nanoparticles comprise one or more bioactive molecules.
11. The multilayer collagen membrane of any preceding clause, wherein one or more layers further comprise bone marrow mesenchymal stem cells.
12. The multilayer collagen membrane of any preceding clause, adapted and configured to mimic human periosteum.
13. The multilayer collagen membrane of any preceding clause, further comprising a fourth, non-porous layer superimposed on the first upper layer.
14. The multilayer collagen membrane of any preceding clause, wherein the fourth layer comprises an electrospun collagen.
15. The multilayer collagen membrane of any preceding clause, wherein the interface between each layer is seamless.
16. The multilayer collagen membrane of any preceding clause, wherein the pore architecture of at least two of the layers is typically different.
17. The multilayer collagen membrane of any preceding clause, in which the pore architecture characteristic is selected from pore size, pore size homogeneity and pore size distribution.
18. The multilayer collagen membrane of any preceding clause, wherein there is continuous physical integration at the interface of each adjacent layer.
19. The multilayer collagen membrane of any preceding clause, wherein the scaffold has a porosity of at least 50%.
20. The multilayer collagen membrane of any preceding clause, wherein the scaffold has an average pore diameter of at least 1 micron in the first layer, at least 5 microns in the second layer, and at least 10 microns in the third layer.
21. The multilayer collagen membrane of any preceding clause, wherein one or more layers is seeded with at least one of osteoblasts, osteoblast-like cells, fibroblasts, fibroblast-like cells, chondrocyte-like cell, and stem cells.
22. The multilayer collagen membrane of any preceding clause, wherein one or more layers is imparted with a medicament selected from the group consisting of anti-infectives, antibiotics, bisphosphonate, hormones, analgesics, anti-inflammatory agents, growth factors, angiogenic factors, chemotherapeutic agents, anti-rejection agents, and RGD peptides.
23. The multilayer collagen membrane of any preceding clause, wherein more layers is imparted with one or more bone-derived growth factors.
24. A method for producing an integrated three-layer collagen membrane, the method comprising:
   (a) preparing a first homogenous suspension of collagen and solvent evaporating the suspension to provide a first layer;
   (b) rehydrating the formed first layer;
   (c) preparing a second homogenous suspension of collagen and elastin, crosslinked with 1,4-butanediol diglycidyl ether;
   (d) pouring the second homogenous crosslinked suspension onto the re-hydrated first layer to form a two-layered composite,
   (e) preparing a third homogenous suspension of magnesium- doped hydroxyapatite and collagen crosslinked with 1,4-butanediol diglycidyl ether;
   (f) pouring the third homogenous crosslinked suspension onto the two-layer composite to form a three-layer composite, and
   (g) lyophilizing the three-layer composite to form an integrated three-layer collagen membrane.
25. The method of clause 24, wherein the crosslinking of one or more layers controls the porosity or the tortuosity of the membrane.
26. The method of clause 24 or clause 25, further comprising incorporating a plurality of nanoparticles into one or more layers of the membrane.
27. The method of any one of clauses 24 to 26, further comprising incorporating one or more bioactive molecules into the plurality of nanoparticles.

## Claims

**1.** A multilayer collagen membrane comprising: (a) a first, non-porous, upper layer that comprises compact collagen; (b) a second, non-mineralized, middle layer comprising elastin; and (c) a third, lower layer that comprises a mineralized component, preferably a mineralized collagen.

**2.** The multilayer collagen membrane of claim 1, further comprising: (d) a fourth, non-porous layer superimposed on the first upper layer, wherein preferably the fourth, non-porous layer comprises an electrospun collagen.

**3.** The multilayer collagen membrane of claim 1 or claim 2, wherein one or more layers comprise Type I collagen, preferably obtained from bovine tendon.

**4.** The multilayer collagen membrane of any preceding claim, wherein the second, middle layer permits vascularization and recapitulates one or more elastic features of mammalian periosteum.

**5.** The multilayer collagen membrane of any preceding claim, wherein the third, lower layer further comprises hydroxyapatite, preferably magnesium- and/or carbamate-doped hydroxyapatite.

**6.** The multilayer collagen membrane of any preceding claim, wherein one or more layers further comprise a population of microspheres, microparticles, nanospheres, or nanoparticles, and/or preferably one or more bioactive molecules.

**7.** The multilayer collagen membrane of any preceding claim, wherein one or more layers comprise chemically-crosslinked collagen, preferably 1,4-butanediol diglycidyl ether-crosslinked collagen.

**9.** The multilayer collagen membrane of any preceding claim, adapted and configured to mimic human periosteum.

**10.** The multilayer collagen membrane of any preceding claim, wherein the interface between each layer is seamless.

**11.** The multilayer collagen membrane of any preceding claim, wherein the pore architecture of at least two of the layers is typically different and/or in which the pore architecture characteristic is selected from pore size, pore size homogeneity, and pore size distribution.

**12.** The multilayer collagen membrane of any preceding claim, wherein there is continuous physical integration at the interface of each adjacent layer.

**13.** The multilayer collagen membrane of any preceding claim, wherein (i) one or more layers is seeded with at least one of osteoblasts, osteoblast-like cells, fibroblasts, fibroblast-like cells, chondrocyte-like cell, and stem cells; and/or (ii) one or more layers is imparted with a medicament selected from the group consisting of anti-infectives, antibiotics, bisphosphonate, hormones, analgesics, anti-inflammatory agents, growth factors, angiogenic factors, chemotherapeutic agents, anti-rejection agents, and RGD peptides; and/or (iii) one or more layers is imparted with one or more bone-derived growth factors or bone-marrow-derived mesenchymal stem cells.

**14.** The multilayer collagen membrane of any one of the preceding claims for use in therapy.
